(19) Europäisches Patentamt
European Patent Office
Office européen des brevets

(11) EP 4 763 871 A1

(12) EUROPEAN PATENT APPLICATION

(43) Date of publication:
**24.06.2026 Bulletin 2026/26**

(21) Application number: **24307277.4**

(22) Date of filing: **23.12.2024**

(51) International Patent Classification (IPC):
***C08F 2/50*** *(2006.01)* ***C07C 323/22*** *(2006.01)*
***C07C 323/62*** *(2006.01)* ***C08F 222/10*** *(2006.01)*

(52) Cooperative Patent Classification (CPC):
**C08F 2/50; C08F 222/102;** C07C 323/22;
C07C 323/62

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **ARKEMA FRANCE**
**92800 Puteaux (FR)**

(72) Inventors:
- **SEHNAL, Petr**
  **WETHERBY, LS22 7NS (GB)**
- **SQUIRES, Kelly**
  **WETHERBY, LS22 7NS (GB)**
- **PLENDERLEITH, Richard**
  **WETHERBY, LS22 7NS (GB)**

(74) Representative: **Arkema Patent**
**Arkema France**
**DRD-DPI**
**51, Esplanade du Général de Gaulle**
**CS 10478**
**92907 Paris La Défense Cedex (FR)**

(54) **BENZOPHENONE PHOTOINITIATORS**

(57) The present invention relates to a redshifted benzophenone photoinitiator, to a process for preparing said photoinitiator. The present invention further relates to a photoinitiator composition, to a curable composition, to a process for the preparation of a cured product, to a process of inkjet printing, to a process of 3D printing and to a process for coating a nail. The invention also relates to the use of the photoinitiator or the photoinitiator composition.

EP 4 763 871 A1

## Description

### TECHNICAL FIELD

The present invention relates to a redshifted benzophenone photoinitiator, to a process for preparing said photoinitiator. The present invention further relates to a photoinitiator composition, to a curable composition, to a process for the preparation of a cured product, to a process of inkjet printing, to a process of 3D printing and to a process for coating a nail. The invention also relates to the use of the photoinitiator or the photoinitiator composition.

### TECHNICAL BACKGROUND

Radiation curable compositions containing ethylenically unsaturated compounds can be polymerized by exposure to radiation, such as ultraviolet (UV) light. For rapid and effective curing, a photoinitiator is often used. The photoinitiator forms radical species upon irradiation with photons and initiates free-radical polymerization of unsaturated groups, leading to hardening (curing) of the material.

Free radical photoinitiators can adopt two different modes of action and are classified by mode of action as Norrish Type I and Norrish Type II Photoinitiators. Norrish Type I photoinitiators cleave upon exposure to radiation, producing radical species which are capable of initiating the polymerization of unsaturated compounds. Norrish Type II photoinitiators are compounds which do not fragment upon exposure to radiation and so will not typically initiate radical-chain polymerization unless a co-initiator is present. Upon exposure to radiation, interaction between the Type II photoinitiator and the co-initiator leads to the generation of radical species which can initiate the polymerization of UV-curable resins.

The most common Type-II photoinitiators are diaryl ketones such as benzophenones (e.g. SpeedCure® BP available from Arkema) or thioxanthones such as 2-isopropylthioxanthone (SpeedCure® 2-ITX available from Arkema) or diethylthioxanthone (SpeedCure® DETX available from Arkema).

One well-recognized problem with the use of UV curable systems for coating and adhesive applications is the fate of the photo-by products created by the curing process. In the case of typical $\alpha$-cleavage type photoinitiators, the production of benzaldehyde (and often related compounds) is often a significant concern from both a toxicity and product odor standpoint. Such concerns become especially important when the use of radiation curable materials is considered for applications that involve skin or food contact. Various effective approaches have been taken to reduce the odor and extractable by-product content of UV curable materials. One approach has been the use of co-polymerizable or polymeric photoinitiators which are chemically incorporated into the cured polymeric matrix as opposed to remaining in the irradiated material as a small molecule (see (a) Fouassier, J. P.; Rabek, J. F., Eds. Radiation Curing in Science and Technology, 1993, Elsevier Appl. Sci., vol. 2, 283-321. (b) Fouassier, J. P. Photoinitiation, Photopolymerization and Photocuring, Fundamentals and Applications, 1995, Hanser Publishers, 71-73.). Unfortunately, when utilizing $\alpha$-cleavage photoinitiators at least one of the cleavage by-products still remains as a small molecule even if the other fragment is incorporated into a polymeric component of the system. Thus, although extractable and odorous by-products can be reduced through the use of polymeric or polymerizable Type I photoinitiators, they are not eliminated entirely.

The use of polymerizable or polymeric H-abstraction type photoinitiators, in principle, presents the possibility of creating a system with zero extractable components related to the photoinitiation system. Various groups have presented systems based upon poly(vinyl benzophenone) and its copolymers or polymers derived from acrylated benzophenone derivatives (see (a) David, C.; Demarteu, W.; Geuskens, G. Polymer, 1969, 10, 21-27. (b) Carlini, C.; Ciardelli, F.; Donati, D.; Gurzoni, F. Polymer, 1983, 24, 599-606.). The direct use of acrylated benzophenones has also been disclosed (U.S. Pat. No. 3,429,852). Unfortunately, these Type II systems often suffer from issues related to photoefficiency relative to analogous small molecule photoinitiation systems.

WO2023161049A1 discloses redshifted polymeric benzophenone photoinitiators bearing a carboxylic group in the ortho-position relative to the carbonyl of the chromophore. Unfortunately, these photoinitiators exhibit low reactivity under LED lamps. Further, the synthesis of compounds disclosed in WO2023161049A1 can lead to formation of an undesirable pseudo-ester by-product with no photoinitiation activity as shown in the scheme below:

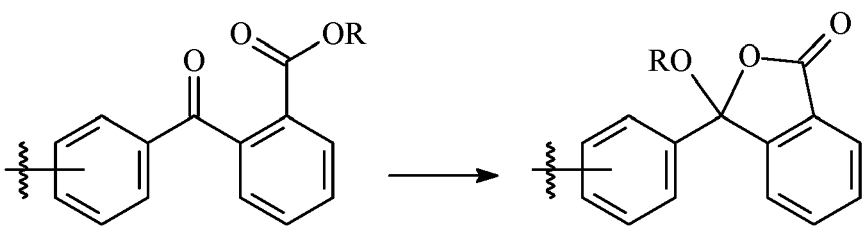

Accordingly, there continues to be a need in the art for improved H-abstraction photoinitiators useful in the manufacture of radiation curable adhesives and coating formulations that do not suffer from the above-identified drawbacks and still produce low odor products with fewer (or no) inherent extractable photochemical by-products. The present development fulfils this need.

Novel redshifted benzophenones are of particular interest since they are curable by UV radiation from light emitting diode (LED) light sources due to their absorption band in the range of 365-420 nm. UV-LED curing is advantageous since LEDs are more compact, less expensive and more environmentally-friendly than broad spectra mercury lamps. However, the use of LEDs is challenging due to increased oxygen inhibition which may limit surface curing and hence the performances of the resulting cured product. The novel benzophenone photoinitiators should therefore advantageously exhibit good photochemical activity (good surface cure and deep cure), low oxygen sensitivity.

The benzophenone photoinitiators of the invention may also present one or more of the following advantages:

- good absorption in the range of 350-420 nm enabling cure with LED lamps,
- low yellowing,
- good solubility with other components of a curable formulation (e.g. with (meth)acrylate-functionalized monomers and/or oligomers),
- they do not significantly act as plasticizers,
- halogen-free.

Further, the benzophenone photoinitiators of the invention may be prepared from inexpensive and readily-available materials, which makes it possible to obtain such photoinitiators in a cost-effective manner.

The polymerizable compositions comprising the photoinitiators of the invention may present one or more of the following advantages:

- usable in sensitive applications (food and beverage packaging, food and beverage processing, pharmaceutical packaging, nail polish, dental products, textiles in contact with a human body, medical devices, water pipes) due to their low migration properties,
- low viscosity,
- high UV reactivity,
- low yellowing,
- weak odor or no odor,
- good mechanical properties after curing (e.g. hardness, scratch resistance, solvent resistance).

The invention is intended to overcome or ameliorate at least some aspects of this problem.

## SUMMARY OF THE INVENTION

The invention relates to a photoinitiator according to formula (1):

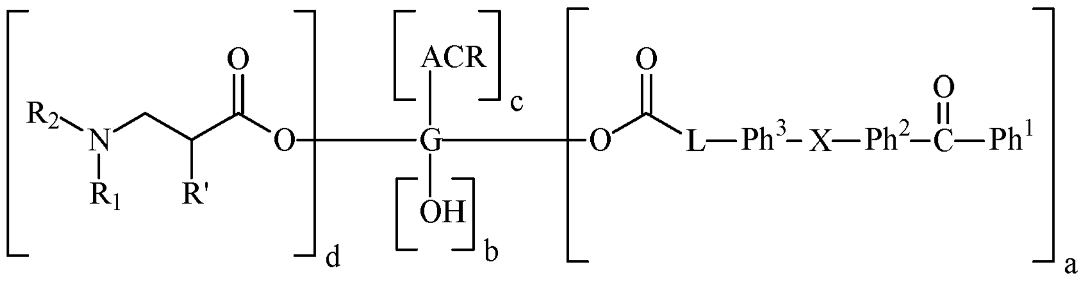

(1)

wherein G, L, ACR, $Ph^1$, $Ph^2$, $Ph^3$, R', $R_1$, $R_2$, X, a, b, c and d are as defined herein.

The invention also relates to a process for the preparation of a photoinitiator of formula (1) according to the invention, wherein the process comprises:

- reacting at least one precursor of formula (3) with at least one compound of formula (4) to provide a compound of formula (1a) and optionally reacting the compound of formula (1a) with at least one amine of formula (8); or
- reacting at least one precursor of formula (3) with at least one compound of formula (5a) to provide a compound of formula (1b); or
- reacting at least one precursor of formula (3) with at least one compound of formula (5) and at least one compound of

formula (6) to provide a compound of formula (1a) and optionally reacting the compound of formula (1a) with at least one amine of formula (8); or

- reacting at least one precursor of formula (3) with at least one compound of formula (7) to provide a compound of formula (1c); or
- reacting at least one precursor of formula (3) with at least one compound of formula (7) and at least one compound of formula (6) to provide a compound of formula (1d) and optionally reacting the compound of formula (1d) with at least one amine of formula (8) to provide a compound of formula (1e);

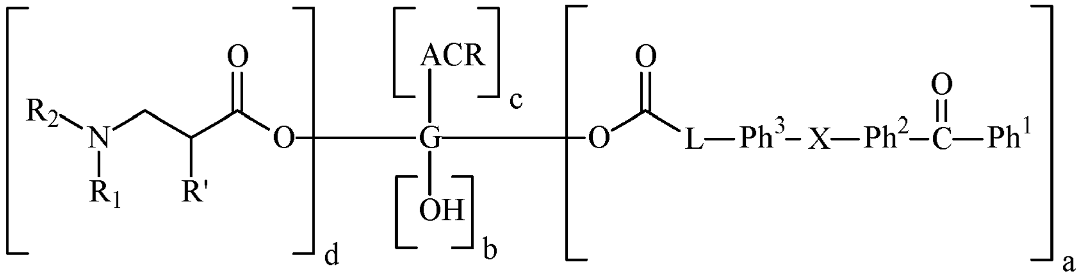
(1)

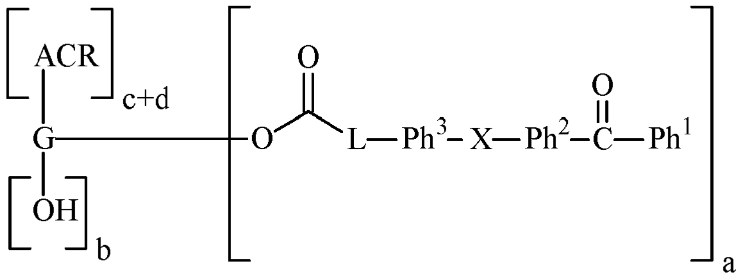
(1a)

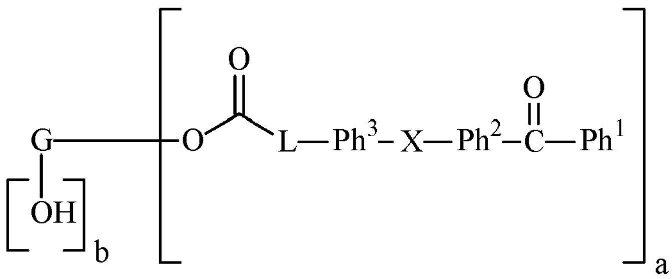
(1b)

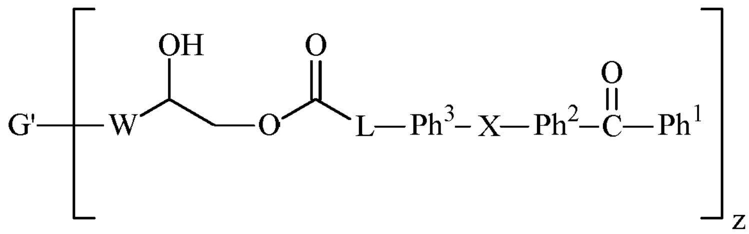
(1c)

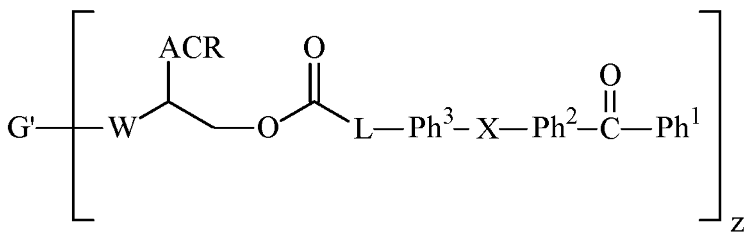
(1d)

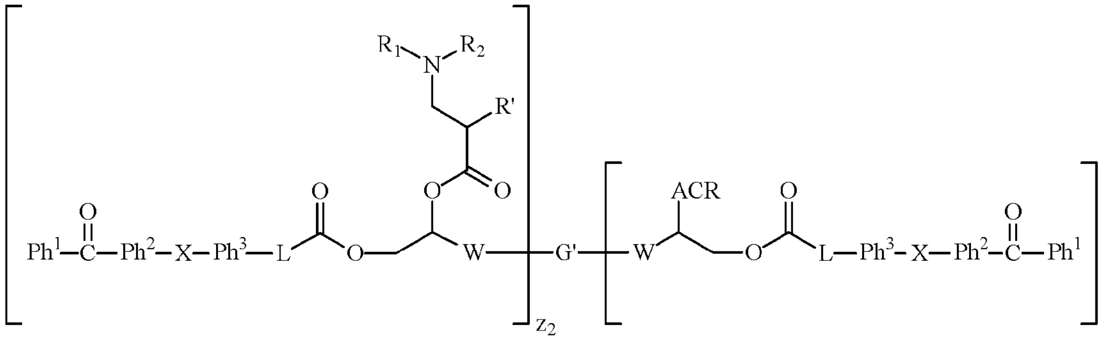 (1e)

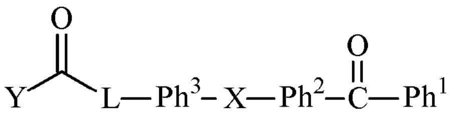 (3)

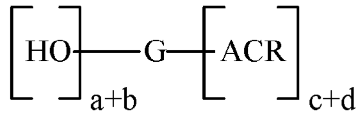 (4)

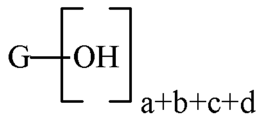 (5)

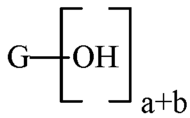 (5a)

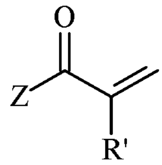 (6)

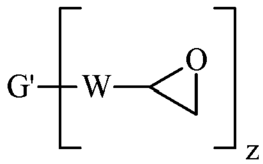 (7)

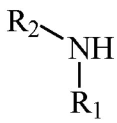 (8)

wherein G, G', L, ACR, $Ph^1$, $Ph^2$, $Ph^3$, R', $R_1$, $R_2$, W, X, Y, Z, a, b, c, d and z are as defined herein;

The invention also relates to a precursor according to formula (3):

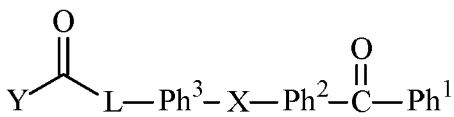 (3)

wherein:

L, $Ph^1$, $Ph^2$, $Ph^3$, X and Y are as defined herein.

The invention also relates to a photoinitiator composition comprising a mixture of at least two photoinitiators of formula (1) according to the invention.

The invention also relates to a photoinitiator composition comprising a photoinitiator of formula (1) according to the invention and a photoinitiator other than a photoinitiator of formula (1).

The invention further relates to a process for photopolymerizing one or more ethylenically unsaturated compounds, wherein the process comprises contacting one or more ethylenically unsaturated compounds with a photoinitiator of formula (1) according to the invention or a photoinitiator composition according to the invention, and irradiating the mixture, in particular with UV, near-UV, visible, infrared and/or near-infrared radiation.

The invention further relates to a curable composition comprising:

a) a photoinitiator of formula (1) according to the invention or a photoinitiator composition according to the invention; and

b) an ethylenically unsaturated component.

The invention further relates to a process for the preparation of a cured product, comprising curing the curable

composition according to the invention, preferably by exposing the curable composition to radiation such as UV, near-UV, visible, infrared and/or near-infrared radiation, more preferably by exposing the curable composition to a LED light source.

The invention further relates to a process of 3D printing comprising printing a 3D article with the curable composition according to the invention, in particular layer by layer or continuously.

The invention further relates to a process of inkjet printing comprising jetting the curable composition according to the invention onto a substrate.

The invention further relates to a process of coating a nail, wherein the process comprises applying the curable composition according to the invention on a nail and curing the composition on the nail.

The invention further relates to a use of a photoinitiator of formula (1) according to the invention or a photoinitiator composition according to the invention, as a photoinitiator or a photoinitiating system in a radiation curable composition, preferably in a UV or LED-curable composition.

The invention further relates to a use of a photoinitiator of formula (1) according to the invention or a photoinitiator composition according to the invention, to cure one or more ethylenically unsaturated compounds.

The invention further relates to a use of a photoinitiator of formula (1) according to the invention or a photoinitiator composition according to the invention, to obtain a cured product having a reduced amount of extractables.

## DETAILED DESCRIPTION

The invention will now be described in more detail without limitation in the following description.

### Definitions

The term *"alkyl"* means a monovalent saturated acyclic hydrocarbon group of formula $-C_nH_{2n+1}$ wherein n is 1 to 20. An alkyl may be linear or branched. Examples of alkyl groups include methyl, ethyl, propyl, isopropyl, butyl, isobutyl, tert-butyl, pentyl, hexyl, 2-methylbutyl, 2,2-dimethylpropyl, n-hexyl, 2-methylpentyl, 2,2-dimethylbutyl, n-heptyl, 2-ethylhexyl, and the like.

The term *"aryl"* means an optionally substituted monovalent polyunsaturated aromatic group. The aryl may contain a single ring (i.e. phenyl) or more than one ring wherein at least one ring is aromatic. When the aryl comprises more than one more ring, the rings may be fused, linked via a covalent bond (for example biphenyl). The aromatic ring may optionally comprise one to two additional fused rings (i.e. cycloalkyl, heterocycloalkyl or heteroaryl). The term *"aryl"* also encompasses partially hydrogenated derivatives of the carbocyclic system described above. Examples include phenyl, naphthyl, biphenylyl, phenanthrenyl and naphthacenyl.

The term *"arylene"* means a substituent that derives from an aromatic hydrocarbon (arene) and is bivalent.

The term *"halogen"* means an atom selected from Cl, Br, F and I.

The term *"cycloalkyl"* means a monovalent saturated alicyclic hydrocarbon group comprising a cycle. Examples of cycloalkyl groups include cyclopentyl, cyclohexyl and isobornyl.

The term *"heterocycloalkyl"* means a cycloalkyl having at least one ring atom that is a heteroatom selected from O, N or S.

The term *"alkoxy"* means a group of formula -O-alkyl, wherein the alkyl is as defined above.

The term *"aryloxy"* means a group of formula -O-aryl, wherein the aryl is as defined above.

The term *"thioalkyl"* means a group of formula -S-alkyl, wherein the alkyl is as defined above.

The term *"thioaryl"* means a group of formula -S-aryl, wherein the aryl is as defined above.

The term *"linker"* means a plurivalent group. A linker may connect at least two moieties of a compound together, in particular 2 to 16 moieties of a compound together. For example, a linker that connects two moieties of a compound together is referred to as a divalent linker, a linker that connects three moieties of a compound together is referred to as a trivalent linker, etc....

The term *"hydrocarbon linker"* means a linker having a carbon backbone chain which may optionally be interrupted by one or more heteroatoms selected from N, O, S, Si and mixtures thereof. A hydrocarbon linker may be aliphatic, cycloaliphatic or aromatic. A hydrocarbon linker may be saturated or unsaturated. A hydrocarbon linker may be optionally substituted.

The term *"aliphatic"* means a non-aromatic acyclic compound. It may be linear or branched, saturated or unsaturated. It may be substituted by one or more groups, for example selected from alkyl, hydroxyl, halogen (Br, Cl, I, F), isocyanate, carbonyl, amine, carboxylic acid, -C(=O)-OR', -C(=O)-O-C(=O)-R', each R' being independently a $C_1$-$C_6$ alkyl. It may comprise one or more bonds selected from ether, ester, amide, urethane, urea and mixtures thereof.

The term *"acyclic"* means a compound that does not comprise any rings.

The term *"cycloaliphatic"* means a non-aromatic cyclic compound. It may be substituted by one or more groups as defined for the term *"aliphatic"*. It may comprise one or more bonds as defined for the term *"aliphatic"*.

The term *"aromatic"* means a compound comprising an aromatic ring, which means that it respects Hückel's

aromaticity rule, in particular a compound comprising a phenyl group. It may be substituted by one or more groups as defined for the term *"aliphatic".* It may comprise one or more bonds as defined for the term *"aliphatic".*

The term *"saturated"* means a compound that does not comprise any double or triple carbon-carbon bonds.

The term *"unsaturated"* means a compound that comprises a double or triple carbon-carbon bond, in particular a double carbon-carbon bond.

The term *"polyether polyol"* or *"polyether linker"* means a polyol (in other words a compound comprising at least two hydroxyl groups), respectively a linker, comprising at least two ether bonds.

The term *"polyester polyol"* or *"polyester linker"* means a polyol, respectively a linker, comprising at least two ester bonds.

The term *"polycarbonate polyol"* or *"polycarbonate linker"* means a polyol, respectively a linker, comprising at least two carbonate bonds.

The term *"polyurethane linker"* means a linker comprising at least two urethane bonds.

The term *"polyorganosiloxane polyol"* or *"polyorganosiloxane linker"* means a polyol, respectively a linker, comprising at least two organosiloxane bonds. The organosiloxane may, for example be a dimethylsiloxane bond.

The term *"polycaprolactone polyol"* or *"polycaprolactone linker"* means a polyol, respectively a linker, comprising at least two units derived from the ring-opening polymerization of ε-caprolactone, in particular at least two $-[(CH_2)_5-C(=O)O]-$ units.

The term *"polybutadiene polyol"* or *"polybutadiene linker"* means a polyol, respectively a linker, comprising at least two units derived from the polymerization of butadiene, in particular at least two units selected from $-CH_2-CH=CH-CH_2-$ and $CH_2-CH(CH=CH_2)-$.

The term *"isocyanate group"* means a -N=C=O group.

The term *"isocyanurate linker"* means a linker comprising an isocyanurate moiety, in particular a moiety of formula:

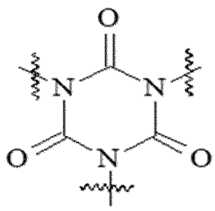

**Photoinitiator of formula (1)**

The present invention relates to a photoinitiator. The photoinitiator is according to the following formula (1):

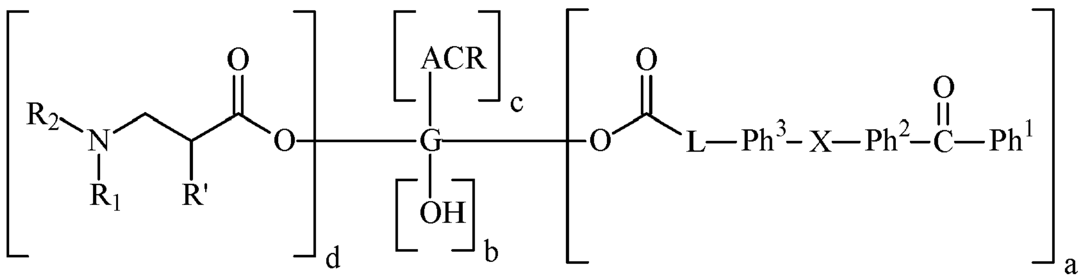
(1)

wherein G, L, ACR, $Ph^1$, $Ph^2$, $Ph^3$, R', $R_1$, $R_2$, X, a, b, c and d are as defined herein.

In formula (1) each ACR is independently a (meth)acrylate group. As used herein, a (meth)acrylate group indifferently refers to an acrylate group $(-O-C(=O)-CH=CH_2)$ or to a methacrylate group $(-O-C(=O)-C(CH_3)=CH_2)$. In one embodiment, each ACR is an acrylate group. In another embodiment, each ACR is a methacrylate group. In yet another embodiment, part of the ACR groups are acrylate groups and the other part of the ACR are methacrylate groups.

In formula (1), each R' is independently H or methyl. Preferably, each R' is H.

In formula (1), each $R_1$ and $R_2$ is independently H or an optionally substituted group selected from alkyl, cycloalkyl and aryl; or $R_1$ and $R_2$ may, with the nitrogen atom to which they are attached, form a 5-7-membered ring; or one of $R_1$ and $R_2$ is an optionally substituted group selected from alkyl, cycloalkyl and aryl and the other of $R_1$ and $R_2$ is a moiety bearing a $-O-C(=O)-L-Ph^3-X-Ph^2-C(=O)-Ph^1$ group.

In a preferred embodiment, each $R_1$ and $R_2$ is independently an optionally substituted group selected from alkyl, cycloalkyl, and aryl. In particular, each $R_1$ and $R_2$ is independently methyl, ethyl, propyl, isopropyl, butyl, sec-butyl, pentyl,

hexyl, octyl, 2-ethylhexyl, 2-hydroxyethyl, 2-hydroxypropyl, 2-methoxyethyl, 3-(dimethylamino)propyl, 3-(bis-(dimethylaminopropyl))propyl, cyclohexyl, phenyl, benzyl, hexahydrobenzyl or a phenyl substituted by one or more groups selected from methyl, ethyl, methoxy, hydroxy -COOMe, -COOEt and mixtures thereof. More particularly, each $R_1$ and $R_2$ is independently ethyl, propyl, isopropyl, butyl, sec-butyl, pentyl, hexyl, octyl, 2-ethylhexyl or 2-hydroxyethyl.

In another preferred embodiment $R_1$ and $R_2$ may, with the nitrogen atom to which they are attached, form a 5-7-membered ring. In particular, $R_1$ and $R_2$ may, with the nitrogen atom to which they are attached, form a ring selected from morpholine, piperidine, 2-, 3- or 4-methylpiperidine, 2-, 3- or 4-ethylpiperidine, N-methylpiperazine, N-ethylpiperazine, N-[3-(dimethylamino)propyl]piperazine, and 1,2,3,4-tetrahydroisoquinoline. More particularly, $R_1$ and $R_2$ may, with the nitrogen atom to which they are attached, form a ring selected from morpholine and piperidine.

In formula (1), each $Ph^1$ is an optionally substituted phenyl.

In particular, each $Ph^1$ may independently be according to formula (2a):

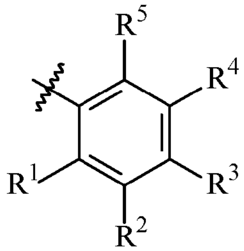

(2a)

wherein

each $R^1$, $R^2$, $R^3$, $R^4$ and $R^5$, is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, $-NO_2$, -CN, $-C(=O)OR_a$, $-OC(=O)R_b$, $-C(=O)R_c$, $-NR_dR_e$ and $-C(=O)NR_dR_e$; or any two of $R^1$ to $R^5$ may, with the carbon atoms to which they are attached, form a 5 to 6-membered ring;

each $R_a$, $R_b$ and $R_c$ is independently an optionally substituted group selected from alkyl and aryl;

each $R_d$ and $R_e$ is independently H or an optionally substituted group selected from alkyl and aryl.

In a preferred embodiment, $R^1$, $R^2$, $R^3$, $R^4$ and $R^5$ are all H.

In formula (1), each $Ph^2$ and $Ph^3$ is independently an optionally substituted phenylene.

Each $Ph^2$ and $Ph^3$ may independently be an optionally substituted ortho-, meta- or para-phenylene, preferably an optionally substituted ortho- or para-phenylene, more preferably an optionally substituted para-phenylene.

In particular, each $Ph^2$ may independently be selected from one of the following formulae (2b) and (2c):

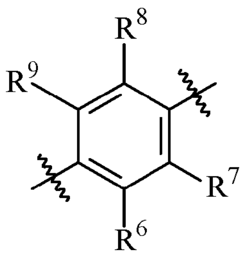

(2b)

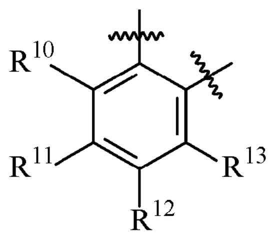

(2c)

wherein

each $R^6$, $R^7$, $R^8$, $R^9$, $R^{10}$, $R^{11}$, $R^{12}$ and $R^{13}$ is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, $-NO_2$, -CN,$-C(=O)OR_a$, $-OC(=O)R_b$, $-C(=O)R_c$, $-NR_dR_e$ and $-C(=O)NR_dR_e$; or any two of $R^6$ to $R^9$ and $R^{10}$ to $R^{13}$ may, with the carbon atoms to which they are attached, form a 5 to 6-membered ring; or one of $R^6$, $R^9$, $R^{10}$ and $R^{13}$ may form a 5 to 6-membered ring with $Ph^3$;

each $R_a$, $R_b$, $R_c$, $R_d$ and $R_e$ is as defined above for formula (2a).

In a preferred embodiment, $R^6$, $R^7$, $R^8$, $R^9$, $R^{10}$, $R^{11}$, $R^{12}$ and $R^{13}$ are all H.

In particular, each $Ph^3$ may independently be selected from one of the following formulae (2d) and (2e):

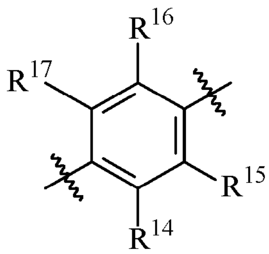
(2d)
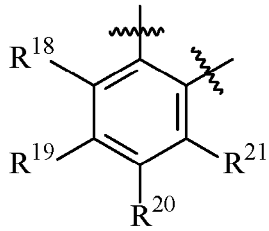
(2e)

wherein

each $R^{14}$, $R^{15}$, $R^{16}$, $R^{17}$, $R^{18}$, $R^{19}$, $R^{20}$ and $R^{21}$ is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, $-NO_2$, -CN,$-C(=O)OR_a$, $-OC(=O)R_b$, $-C(=O)R_c$, $-NR_dR_e$ and $-C(=O)NR_dR_e$; or any two of $R^{14}$ to $R^{17}$ and $R^{18}$ to $R^{21}$ may, with the carbon atoms to which they are attached, form a 5 to 6-membered ring; or one of $R^{15}$, $R^{16}$, $R^{18}$ and $R^{21}$ may form a 5 to 6-membered ring with $Ph^2$;

each $R_a$, $R_b$, $R_c$, $R_d$ and $R_e$ is as defined above for formula (2a).

In a preferred embodiment, $R^{14}$, $R^{15}$, $R^{16}$, $R^{17}$, $R^{18}$, $R^{19}$, $R^{20}$ and $R^{21}$ are all H.

In formula (1), each L is independently a direct bond or a divalent linker. The divalent linker may be a linker having from 1 to 20 carbon atoms and optionally one or more heteroatoms selected from O, N and S. The divalent linker may be aliphatic, aromatic, cycloaliphatic or a combination thereof.

In particular, each L may independently be a direct bond or $*-C(=O)-L_1-$, wherein $L_1$ is an optionally substituted group selected from alkylene, alkenylene and arylene, any of which may be optionally interrupted with one or more of -O-, -S-, and -C(=O)-; the symbol * representing the point of attachment to $-Ph^3-$.

In a preferred embodiment, $L_1$ is according to one of the following formulae:

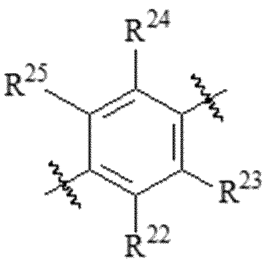
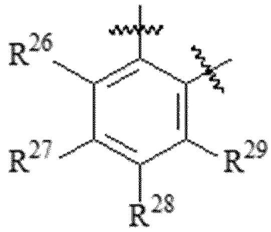
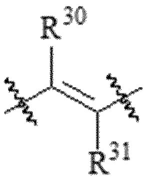
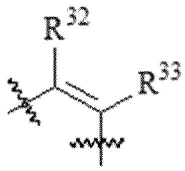

-ALK-

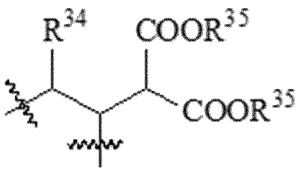
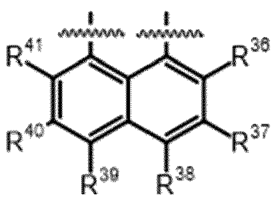
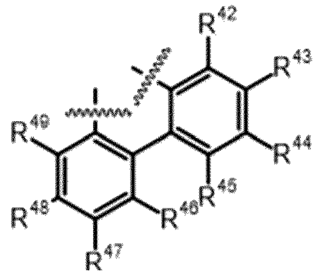

wherein

each $R^{22}$ , $R^{23}$ , $R^{24}$, $R^{25}$, $R^{26}$, $R^{27}$, $R^{28}$, $R^{29}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$ and $R^{49}$ is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, $-NO_2$, -CN, $-C(=O)OR_a$, $-OC(=O)R_b$, $-C(=O)R_c$, $-NR_dR_e$ and $-C(=O)NR_dR_e$; or any two of $R^{22}$ to $R^{25}$ and $R^{26}$ to $R^{29}$ and $R^{36}$ to $R^{41}$ and $R^{42}$ to $R^{49}$ may, with the carbon atoms to which they are attached, form a 5 to 6-membered ring;

each $R_a$, $R_b$, $R_c$, $R_d$ and $R_e$ is as defined above for formula (2a);
each $R^{30}$, $R^{31}$, $R^{32}$, $R^{33}$ and $R^{34}$ is independently H or an optionally substituted group selected from alkyl and aryl;
ALK is an optionally substituted alkylene;
each $R^{35}$ is independently an optionally substituted group selected from alkyl and aryl.

In formula (1), each X is independently -S-, -S(=O)-, -O- or -NR-; and each R is independently H or an optionally substituted group selected from alkyl and aryl.
In one embodiment, each X is -S-.
In another embodiment, each X is -S(=O)-.
In another embodiment, each X is -O-.
In formula (1), G is a (a+b+c+d)valent linker. In particular, G may be a divalent, trivalent, tetravalent, pentavalent, hexavalent, heptavalent, octavalent, nonavalent, decavalent, undecavalent or dodecavalent linker. More particularly, G may be a divalent, trivalent or tetravalent linker.
G may be selected from an aromatic linker, an aliphatic linker, a cycloaliphatic linker, a polyether linker, a polythioether linker, a polyalkylene imine linker, a polyester linker, a polycarbonate linker, a polyurethane linker, a polyorganosiloxane linker, a polybutadiene linker, and combinations thereof. Preferably, G is selected from an aromatic linker, an aliphatic linker, a cycloaliphatic linker, a polyether linker, a polythioether linker, a polyalkylene imine linker, a polyester linker, a polyorganosiloxane linker, and combinations thereof.
G may be selected from:

- a divalent moiety according to any one of formulae (9) to (18):

$$-(CR_4R'_4)_e- \qquad (9)$$

$$-CH_2-O-CH_2- \qquad (10)$$

$$-[(CR_5R'_5)_f-W]_g-(CR_5R'_5)_f- \qquad (11)$$

$$-[(CR_6R'_6)_h-W]_i-(CR_7R'_7)_j-[W-(CR_6R'_6)_h]_i- \qquad (12)$$

$$-[(CR_8R'_8)_k-C(=O)O]_l-(CR_9R'_9)_m- \qquad (13)$$

$$-(CR_9R'_9)_m-[OC(=O)-(CR_8R'_8)_k]_l- \qquad (14)$$

$$-[(CR_{10}R'_{10})_n-O-C(=O)-(CR_{11}R'_{11})_o-C(=O)-O]_p-(CR_{10}R'_{10})_n- \qquad (15)$$

$$-(CR_{12}R'_{12})_q-C(=O)-O-(CR_{13}R'_{13})_r-O-C(=O)-(CR_{12}R'_{12})_q- \qquad (16)$$

$$-(CR_{14}R'_{14})_s-Cy-[L_0-Cy]_t-(CR_{14}R'_{14})_s- \qquad (17)$$

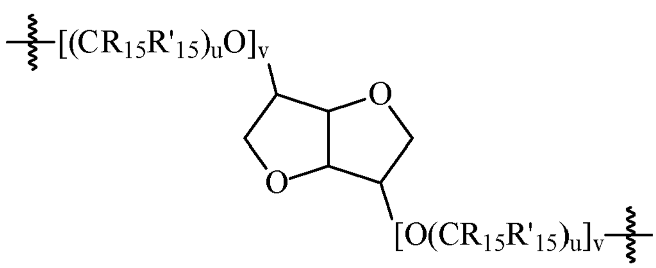

(18)

wherein:

- each $R_4$, $R'_4$, $R_7$, $R'_7$, $R_9$, $R'_9$, $R_{10}$, $R'_{10}$, $R_{11}$, $R'_{11}$, $R_{12}$, $R'_{12}$, $R_{13}$, $R'_{13}$, $R_{14}$ and $R'_{14}$ is independently H or alkyl;
- each $R_5$, $R'_5$, $R_6$, $R'_6$, $R_8$, $R'_8$, $R_{15}$ and $R'_{15}$ is independently H or methyl;
- each W is independently -O- or -S-;
- each Cy is independently an optionally substituted ring, in particular an optionally substituted cyclohexylene or

phenylene;

- each $L_0$ is independently a bond or a linker such as Alk, -C(=O)-,-C(=O)-O-Alk-O-C(=O)-, -SO-, $-SO_2-$, $-C(=CCl_2)-$ and -Alk-Ph-Alk-;
- Alk is an optionally substituted alkylene;
- Ph is an optionally substituted phenylene;
- each e, j, m, n, o and r is independently an integer from 2 to 20;
- each s is independently an integer from 0 to 20;
- each f, h and u is independently an integer from 2 to 4;
- t is an integer equal to 0 or 1;
- each g, l, p and q is independently an integer from 1 to 20;
- each v is independently an integer from 0 to 10, in particular from 1 to 6;
- each i is independently an integer from 0 to 20 with the proviso that at least one i is not 0;
- k is an integer from 3 to 12;

- a trivalent moiety according to any one of formulae (19) to (24):

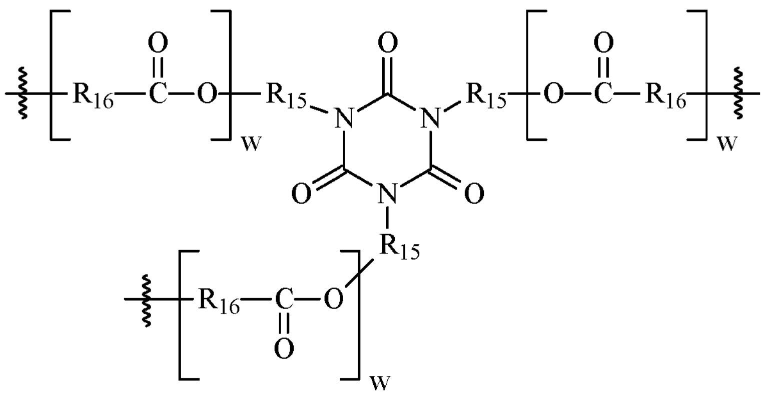

(19)

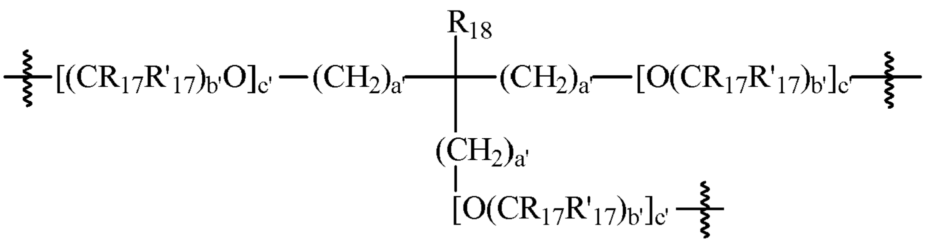

(20)

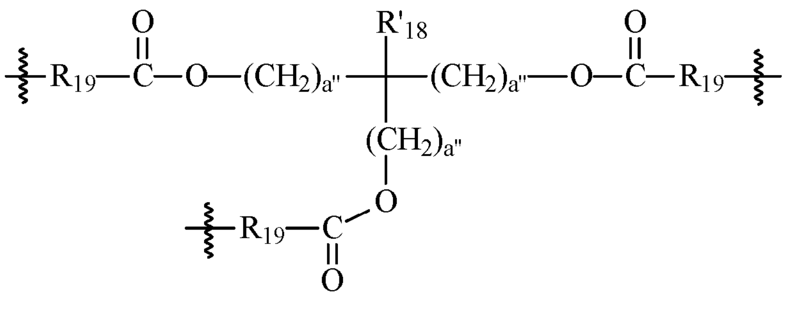

(21)

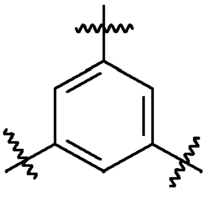

(22)

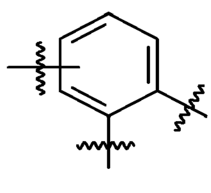

(23)

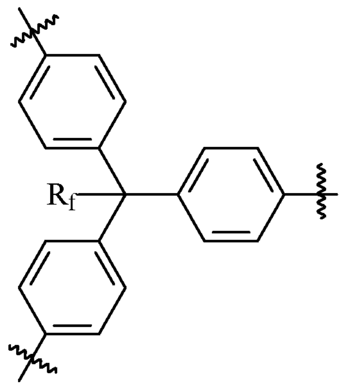

(24)

wherein:

- each $R_{15}$ and $R_{16}$ is independently a linear or branched alkylene;
- each $R_{17}$ and $R'_{17}$ is independently H or methyl;
- each $R_{18}$ and $R'_{18}$ is independently H, alkyl or alkoxy, preferably each $R_{18}$ and $R'_{18}$ is alkyl;
- each $R_{19}$ is independently a linear or branched alkylene;
- $R_f$ is H or methyl;
- w is an integer equal to 0 or 1;
- each a' is independently an integer from 0 to 2 with the proviso that not more than one a' is equal to 0, preferably each a' is equal to 1 or one a' is equal to 0 and the two other a' are equal to 1;
- each a" is independently an integer from 0 to 2 with the proviso that not more than one a" is equal to 0, preferably each a" is equal to 1 or one a" is equal to 0 and the two other a" are equal to 1;
- each b' is independently an integer from 2 to 4, in particular 2;
- each c' is independently an integer from 0 to 10, in particular from 1 to 6;

- a tetravalent moiety according to any one of formulae (25) to (28):

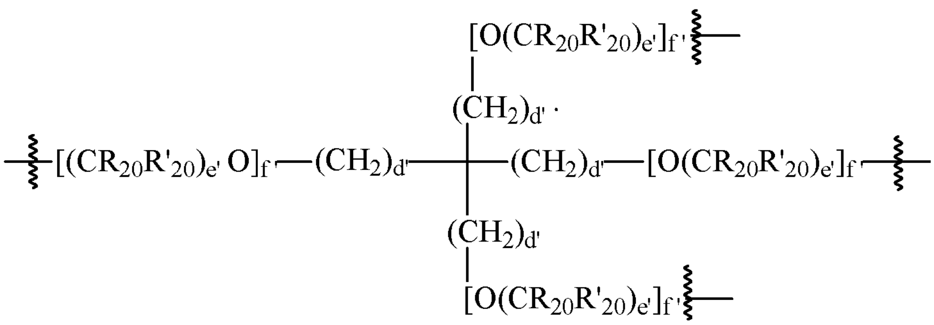

(25)

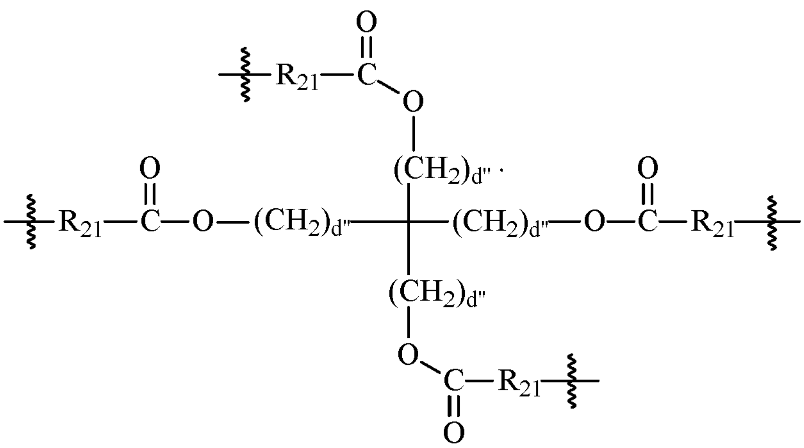

(26)

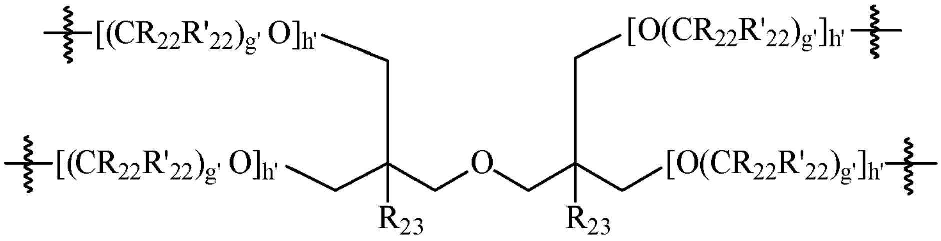

(27)

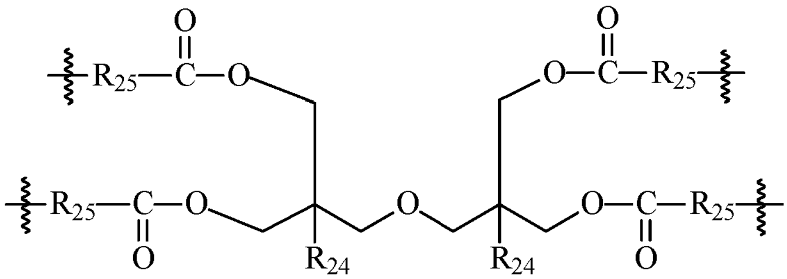

(28)

wherein:

- each $R_{20}$, $R'_{20}$, $R_{22}$ and $R'_{22}$ is independently H or methyl;
- each $R_{21}$ and $R_{25}$ is independently a linear or branched alkylene;
- each $R_{23}$ and $R_{24}$ is independently H, alkyl or alkoxy, preferably alkyl;
- each d' is independently an integer from 0 to 2 with the proviso that not more than one d' is equal to 0, preferably each d' is equal to 1;
- each d'' is independently an integer from 0 to 2 with the proviso that not more than one d'' is equal to 0, preferably each d'' is equal to 1;
- each e' and g' is independently an integer from 2 to 4, in particular 2;
- each f' and h' is independently an integer from 0 to 10, in particular from 1 to 6;

- a tetra-, penta- or hexavalent moiety according to formula (29):

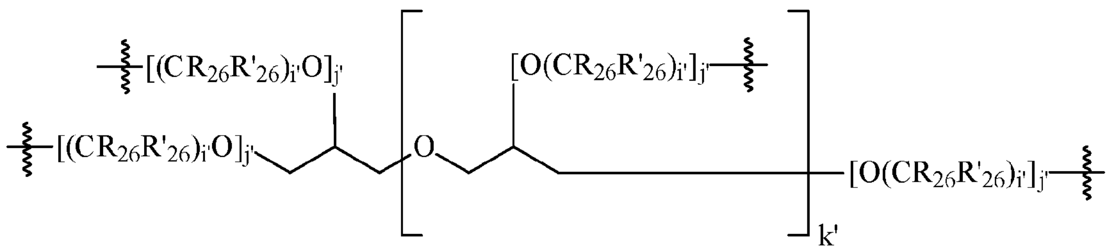

(29)

wherein:

- each $R_{26}$ and $R'_{26}$ is independently H or methyl;
- each i' is independently an integer from 2 to 4, in particular 2;
- each j' is independently an integer from 0 to 10, in particular from 1 to 6;
- k' is an integer from 1 to 3;

- a tri-, tetra-, penta- or hexavalent moiety according to formula (30):

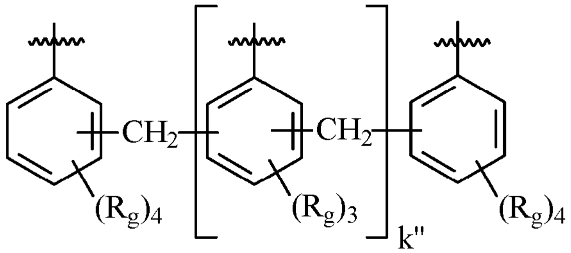

(30)

wherein

- each $R_g$ is independently selected from H, alkyl, cycloalkyl, aryl, alkaryl, aralkyl, alkoxy, -C(=O)O-Alkyl and a halogen atom;
- k" is an integer from 1 to 4.

- a hexavalent moiety according to any one of formulae (31) to (33):

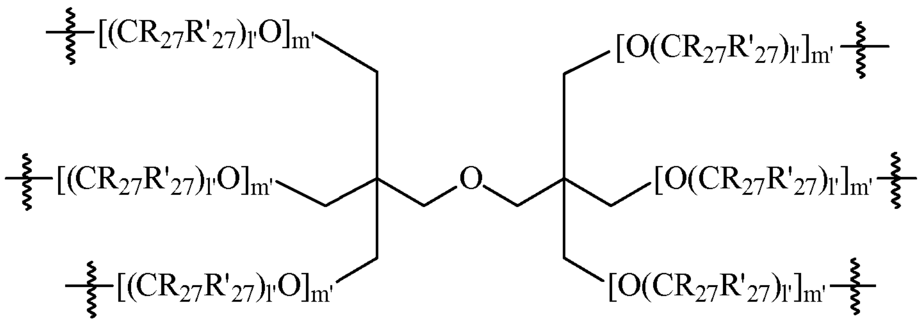

(31)

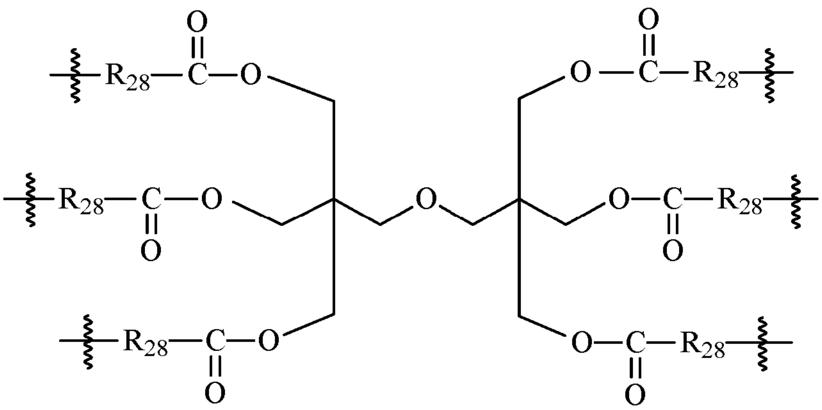

(32)

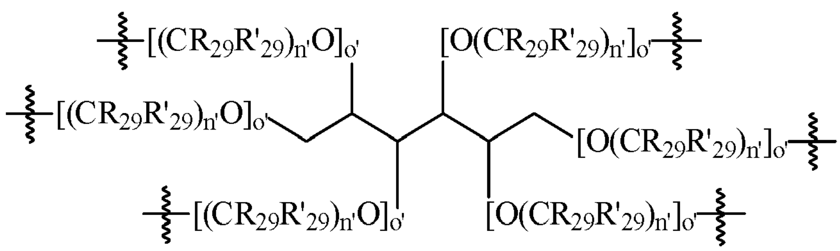

(33)

wherein

- each $R_{27}$, $R'_{27}$, $R_{29}$ and $R'_{29}$ is independently H or methyl;
- each $R_{28}$ is independently a linear or branched alkylene;

- each l' and n' is independently an integer from 2 to 4, in particular 2;
- each m' and o' is independently an integer from 0 to 10, in particular from 1 to 6.

According to some embodiments, G may be the residue of a polyol (i.e. the residue that is obtained by removing the OH groups of a polyol). Examples of suitable polyols, referred to herein as $P_{OH}$, include ethylene glycol, 1,2- or 1,3-propylene glycol, 1,2-, 1,3- or 1,4-butylene glycol, 1,5-pentanediol, 1,6-hexanediol, 1,7-heptanediol, 1,8-octanediol, 1,9-nonanediol, 1,10-decanediol, 1,12-dodecanediol, 2-methyl-1,3-propanediol, 2,2-diethyl-1,3-propanediol, 3-methyl-1,5-pentanediol, 3,3-dimethyl-1,5-pentanediol, neopentyl glycol, 2,4-diethyl-1,5-pentanediol, 3,3-butylethyl-1,5-pentane diol, cyclohexanediol, cyclohexane-1,4-dimethanol, norbornene dimethanol, norbornane dimethanol, tricyclodecanediol, tricyclodecane dimethanol, dicyclopentadiene diol, hydroquinone bis(2-hydroxyethyl) ether, pyrocatechol, resorcinol, cardol, phloroglucinol, pyrogallol, tris(hydroxyphenyl)methane, tris(hydroxyphenyl)ethane, bisphenol A, B, F or S, hydrogenated bisphenol A, B, F or S, trimethylolmethane, trimethylolethane, trimethylolpropane, di(trimethylolpropane), triethylolpropane, pentaerythritol, di(pentaerythritol), tri(pentaerythritol), glycerol, di-, tri- or tetraglycerol, a polyglycerol, di-, tri- or tetra(ethylene glycol), di-, tri- or tetra(1,2-propylene glycol), di-, tri- or tetra(1,3-propylene glycol), di-, tri- or tetra(1,4-butylene glycol), a poly(ethylene glycol), a polypropylene glycol), a poly(trimethylene glycol), a poly(tetramethylene glycol), a poly(ethylene glycol-co-propylene glycol), an alditol (i.e. erythritol, threitol, arabitol, xylitol, ribitol, mannitol, sorbitol, galactitol, fucitol or iditol), a dianhydrohexitol (i.e. isosorbide, isomannide, isoidide), tris(2-hydroxyethyl)isocyanurate, a hydroxylated vegetable oil, a $C_{36}$ dimer diol, a polybutadiene polyol, a polyester polyol, a polyether polyol, a polyorganosiloxane polyol (such as Silmer OH A0-UP, Silmer OH C50, Silmer OH J10, Silmer OH Di-10, Silmer OH Di-50, Silmer OHT A0, Silmer OHT Di-10, Silmer OHT Di-50, Silmer OHT Di-100, Silmer OHT Di-400, Silmer OHT E13 which are available from Siltech or SiSiB® OF0035, SiSiB® OF0042, SiSiB® OF0156A, SiSiB® OF0156B, SiSiB® OF1300-M1100, SiSiB® OF1300-M2000, SiSiB® OF1300-M4000, SiSiB® OF6053, SiSiB® OF6055, SiSiB® OF9020 which are available from Sinosil) , a polycarbonate polyol, as well as the alkoxylated (e.g., ethoxylated and/or propoxylated) derivatives thereof and the derivatives obtained by ring-opening polymerization of a lactone (such as ε-caprolactone) initiated with one of the aforementioned polyols, and combinations thereof.

In particular, G may be the residue of a polyol selected from an optionally alkoxylated trimethylolpropane, an optionally alkoxylated di(trimethylolpropane), an optionally alkoxylated pentaerythritol, an optionally alkoxylated di(pentaerythritol), an optionally alkoxylated glycerol, an optionally alkoxylated di-, tri or tetraglycerol, an optionally alkoxylated sorbitol, a poly(ethylene glycol), a polypropylene glycol), a poly(ethylene glycol-co-propylene glycol), a poly(trimethylene glycol), a poly(tetramethylene glycol), a polycaprolactone polyol, and combinations thereof.

Alternatively, G may be a plurivalent moiety corresponding to the following formula (34):

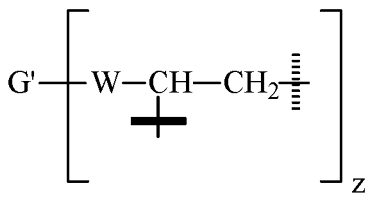

(34)

wherein

- G' is a z-valent linker;
- W is a bond, #-O-$CH_2$- or #-C(=O)-O-$CH_2$-;
- z is from 2 to 6;
- the symbol # represents a point of attachment to G';
- the bold line represents a point of attachment to a hydroxyl group or to a ACR group or to a group of formula -O-C(=O)-CH(R')-$CH_2$-$NR_1R_2$;
- each hashed line represents a point of attachment to a -O-C(=O)-L-$Ph^3$-X-$Ph^2$-C(=O)-$Ph^1$ group or to a ACR group or to a group of formula -O-C(=O)-CH(R')-$CH_2$-$NR_1R_2$, provided that at least one of the hashed lines represents a point of attachment to a -O-C(=O)-L-$Ph^3$-X-$Ph^2$-C(=O)-$Ph^1$ group.

In formula (34), G' may be selected from:

- a divalent moiety according to any one of formulae (9) to (18) as defined above for G;
- a trivalent moiety according to any one of formulae (19) to (24) as defined above for G;
- a tetravalent moiety according to any one of formulae (25) to (28) as defined above for G;
- a tetra-, penta- or hexavalent moiety according to formula (29) as defined above for G;

- a tri-, tetra-, penta- or hexavalent moiety according to formula (30) as defined above for G;
- a hexavalent moiety according to any one of formulae (31) to (33) as defined above for G.

In particular, G' may be the residue of a polyepoxide (i.e. the residue obtained by removing the epoxy groups of a polyepoxide). Examples of suitable polyepoxides, referred to herein as $P_{EPOX}$, diglycidyl ether, 1,2,3,4-diepoxybutane, 1,2,4,5-diepoxypentane, 1,2,5,6-diepoxyhexane, 1,2,7,8-diepoxyoctane, 1,2,9,10-diepoxydecane, ethylene glycol diglycidyl ether, 1,2- or 1,3-propylene glycol diglycidyl ether, 1,2-, 1,3- or 1,4-butanediol diglycidyl ether, 1,5-pentanediol diglycidyl ether, 1,6-hexanediol diglycidyl ether, 1,7-hexanediol diglycidyl ether, 1,8-octanediol diglycidyl ether, 1,9-nonanediol diglycidyl ether, 1,10-decanediol diglycidyl ether, 1,12-dodecanediol diglycidyl ether, 2-methyl-1,3-propanediol diglycidyl ether, neopentyl glycol diglycidyl ether, 2,2-diethyl-1,3-propane diol diglycidyl ether, 3-methyl-1,5-pentanediol diglycidyl ether, 3,3-dimethyl-1,5-pentanediol diglycidyl ether, 2,4-diethyl-1,5-pentanediol diglycidyl ether, 3,3-butylethyl-1 ,5-pentane diol diglycidyl ether, di-, tri- or tetra(ethylene glycol) diglycidyl ether, di-, tri- or tetra(1,2-propylene glycol) diglycidyl ether, di-, tri- or tetra(1,3-propylene glycol) diglycidyl ether, di-, tri- or tetra(1,4-butylene glycol) diglycidyl ether, a poly(ethylene glycol) diglycidyl ether, a polypropylene glycol) diglycidyl ether, a poly(trimethylene glycol) diglycidyl ether, a poly(tetramethylene glycol) diglycidyl ether, a poly(ethylene glycol-co-propylene glycol) diglycidyl ether, glycerol triglycidyl ether, a polyglycerol polyglycidyl ether, trimethylolmethane triglycidyl ether, trimethylolethane triglycidyl ether, trimethylolpropane triglycidyl ether, di(trimethylolpropane) tetraglycidyl ether, pentaerythritol tetraglycidyl ether, diglycidyl cyclohexanedicarboxylate, cyclohexane diglycidyl ether, cyclohexane-1,4-dimethanol diglycidyl ether, tricyclodecane dimethanol diglycidyl ether, isosorbide diglycidyl ether, pyrocatechol diglycidyl ether, resorcinol diglycidyl ether, cardol diglycidyl ether, phloroglucinol triglycidyl ether, pyrogallol triglycidyl ether, tris(hydroxyphenyl)methane triglycidyl ether, tris(hydroxyphenyl)ethane triglycidyl ether, bisphenol A, B, F or S diglycidyl ether, hydrogenated bisphenol A, B, F or S diglycidyl ether, diglycidyl phthalate, diglycidyl terephthalate, diglycidyl isophthalate; an epoxidized vegetable oil (such as epoxidized soybean oil and epoxidized linseed oil), epoxidized polybutadiene; triglycidyl isocyanurate, an epoxy-functionalized polyorganosiloxane, and combinations thereof.

In particular, each G may independently be the residue of a polyepoxide selected from 1,4-butanediol diglycidyl ether, di-, tri- or tetra(ethylene glycol) diglycidyl ether, di-, tri- or tetra(1,2-propylene glycol) diglycidyl ether, di-, tri- or tetra(1,3-propylene glycol) diglycidyl ether, di-, tri- or tetra(1,4-butylene glycol) diglycidyl ether, a poly(ethylene glycol) diglycidyl ether, a polypropylene glycol) diglycidyl ether, a poly(trimethylene glycol) diglycidyl ether, a poly(tetramethylene glycol) diglycidyl ether, a poly(ethylene glycol-co-propylene glycol) diglycidyl ether, glycerol triglycidyl ether, a polyglycerol polyglycidyl ether, and combinations thereof.

In formula (34), W is a bond, #-O-$CH_2$- or #-C(=O)-O-$CH_2$-. In particular, W may be #-O-$CH_2$- or #-C(=O)-O-$CH_2$-. More particularly W may be #-O-$CH_2$-.

In formula (34), z is from 2 to 6. In particular, z may be from 2 to 4. More particularly, z may be an integer equal to 2 or 3.

In formula (1), a is from 1 to 7; b is from 0 to 7; c is from 0 to 7; d is from 0 to 7; and the sum a+b+c+d is from 1 to 12.

In formula (1), a may be an integer equal to 1, 2, 3, 4, 5 or 6, in particular a may be an integer equal to 1, 2, 3 or 4.

In formula (1), b may be an integer equal to 0, 1 or 2, in particular b may be an integer equal to 0 or 1.

In formula (1), c may be an integer equal to 0, 1, 2, 3, 4 or 5, in particular, c may be an integer equal to 0, 1, 2 or 3.

In formula (1), d may be an integer equal to 0, 1, 2, 3, 4 or 5, in particular, d may be an integer equal to 0, 1, 2 or 3.

In formula (1), the sum a+b+c+d may be from 2 to 12, preferably from 2 to 8.

Non-limiting examples of photoinitiators of formula (1) are detailed below:

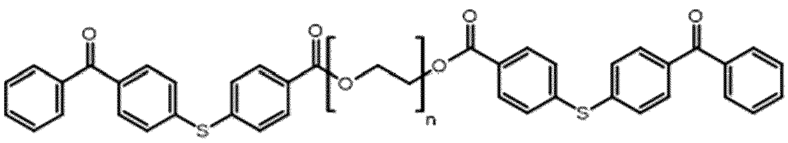

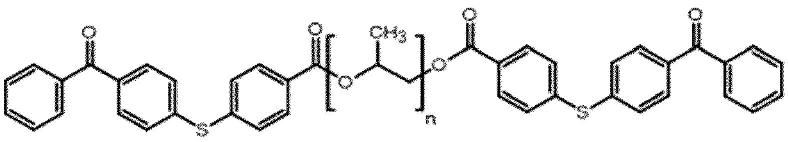

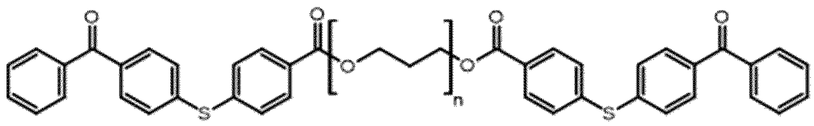

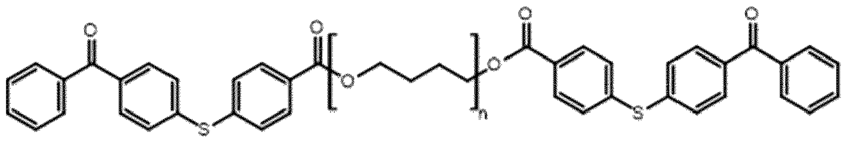

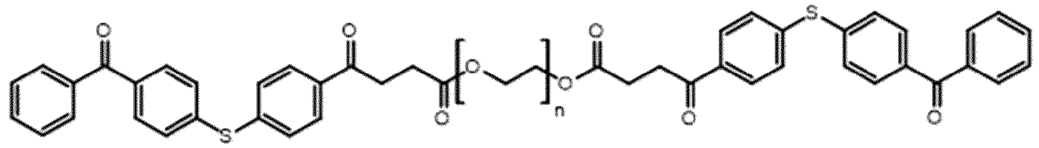

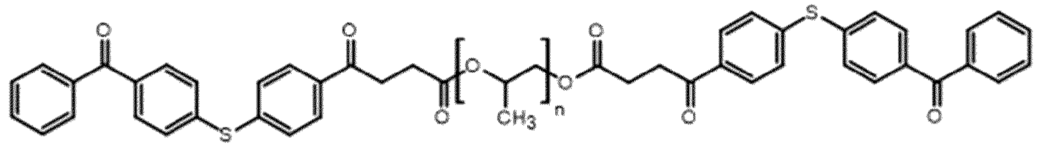

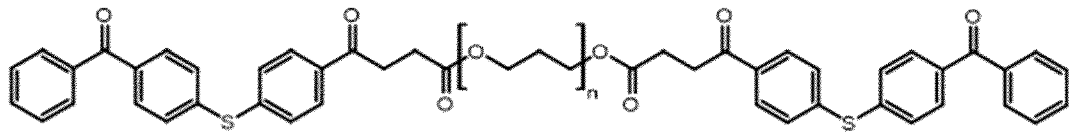

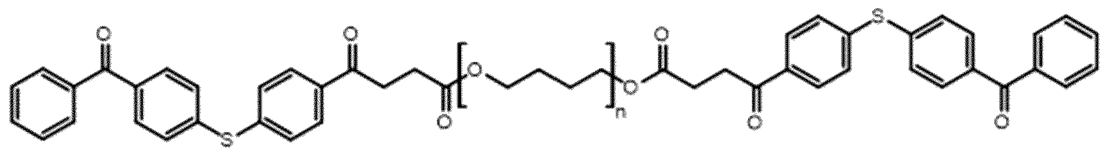

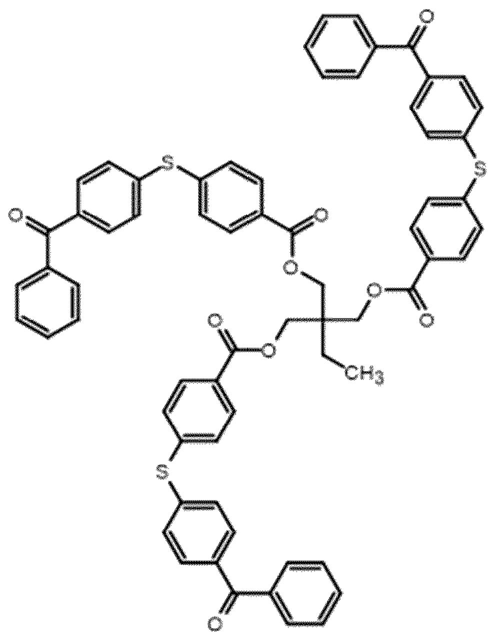

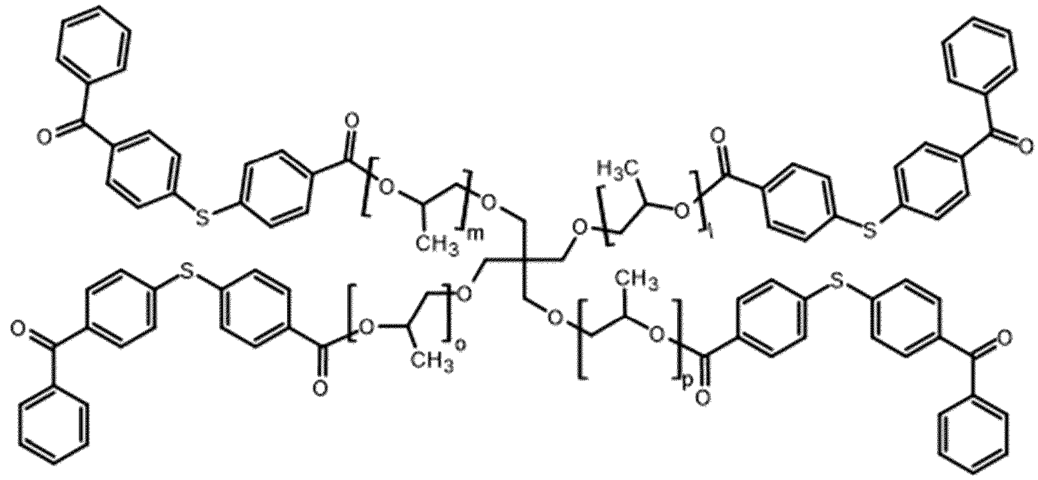

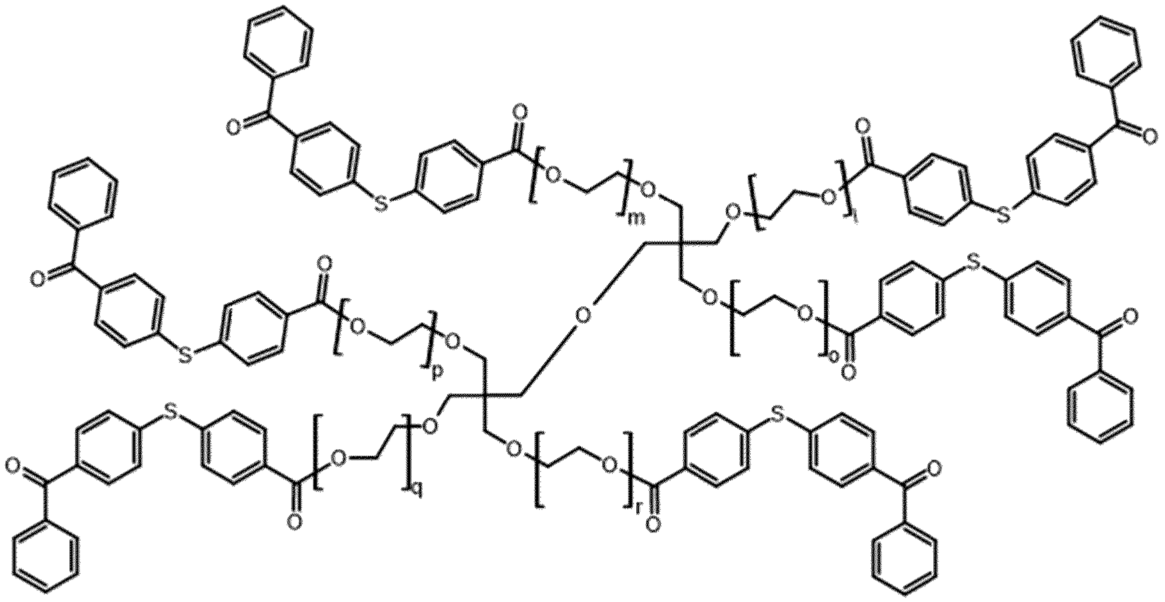

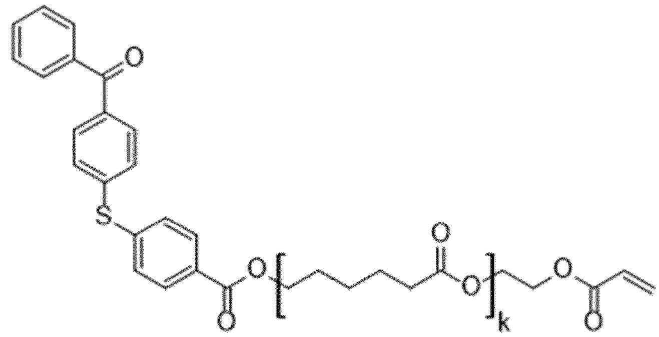

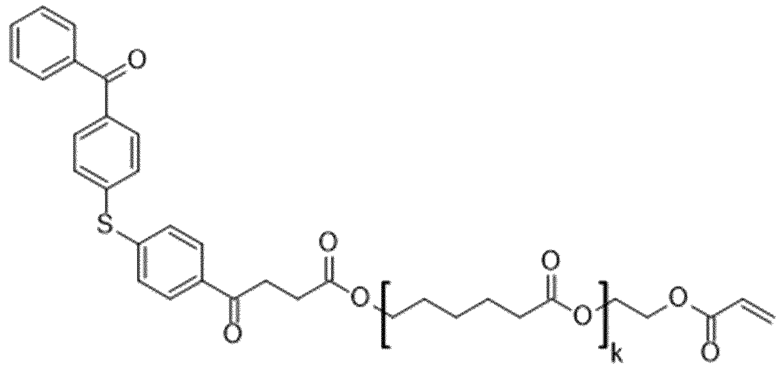

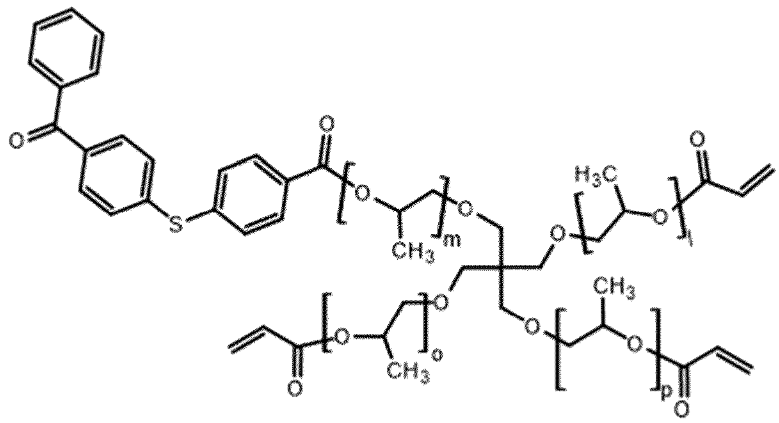

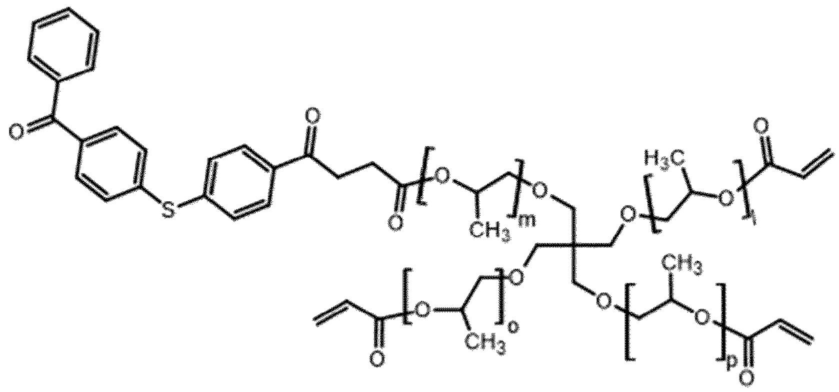

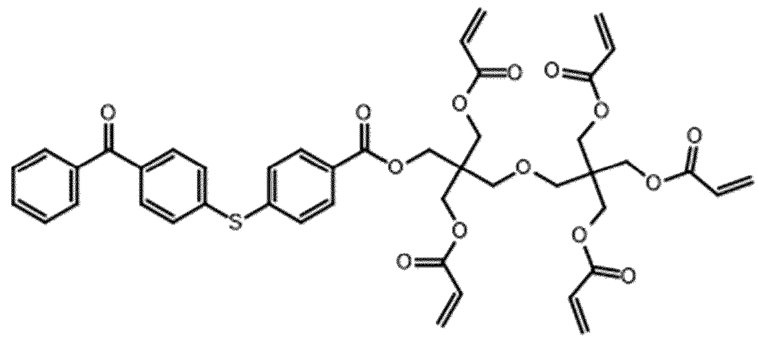

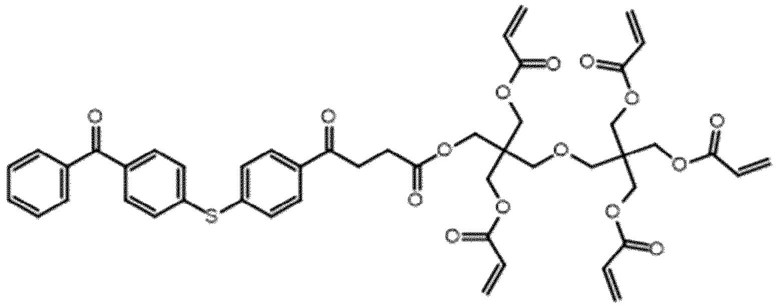

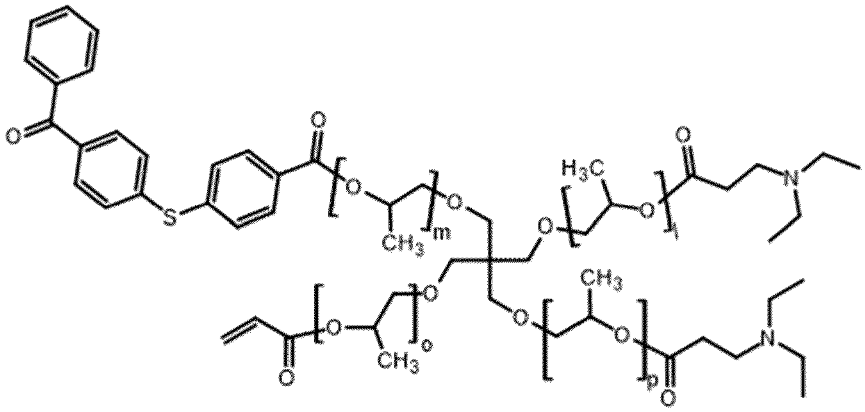

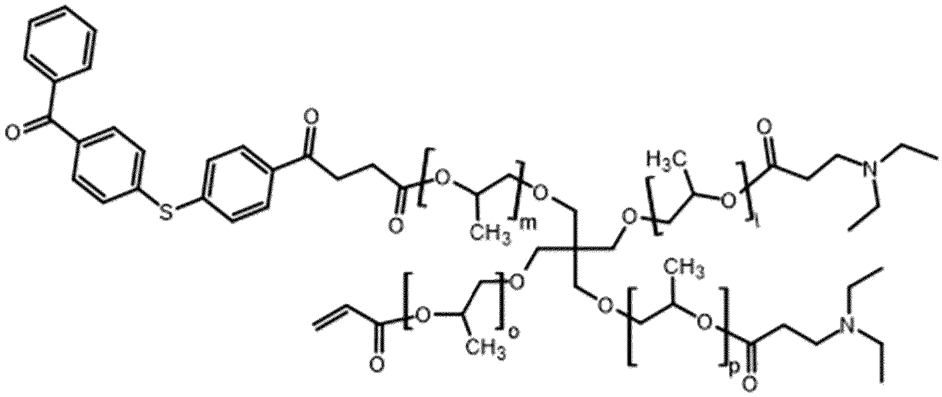

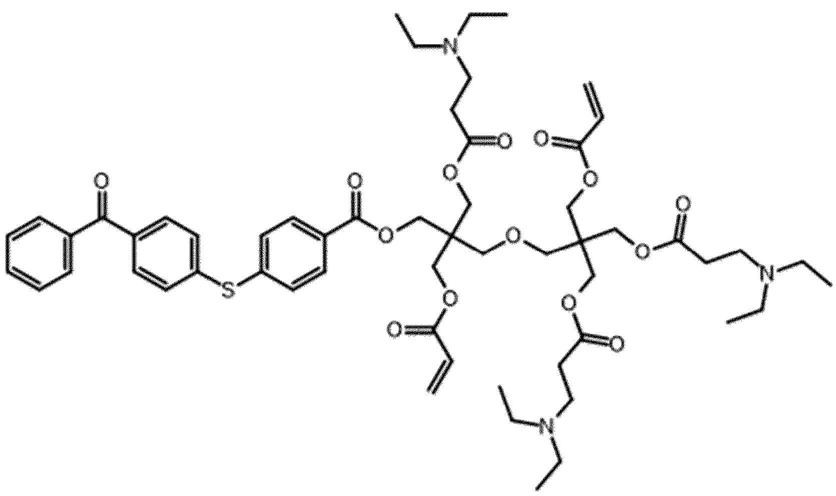

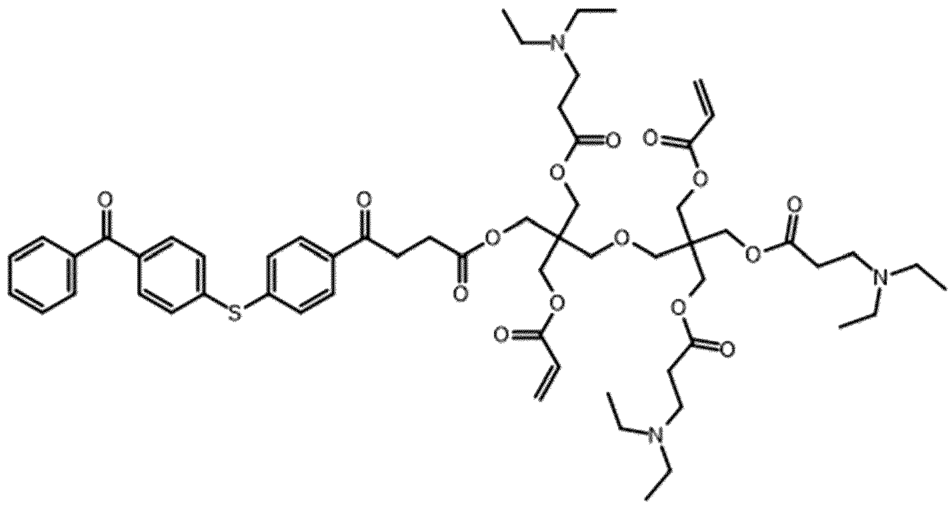

wherein

each n is independently an integer from 1 to 30;
each l, m, o, p, q, and r is independently an integer from 0 to 30.

In the formulas above, one or more of the oxyethylene units may indifferently be replaced by an oxypropylene unit and/or one or more of the oxypropylene units may indifferently be replaced by an oxyethylene unit.
The photoinitiator of formula (1) may be prepared by a process as detailed below.

## Preparation process

All the particular and preferred embodiments described above for the photoinitiator of formula (1) equally apply to the following process.
The photoinitiator of formula (1) as described above may be prepared by a process comprising:

- reacting at least one precursor of formula (3) with at least one compound of formula (4) to provide a compound of formula (1a) and optionally reacting the compound of formula (1a) with at least one amine of formula (8); or
- reacting at least one precursor of formula (3) with at least one compound of formula (5a) to provide a compound of formula (1b); or
- reacting at least one precursor of formula (3) with at least one compound of formula (5) and at least one compound of formula (6) to provide a compound of formula (1a) and optionally reacting the compound of formula (1a) with at least one amine of formula (8); or
- reacting at least one precursor of formula (3) with at least one compound of formula (7) to provide a compound of formula (1c); or
- reacting at least one precursor of formula (3) with at least one compound of formula (7) and at least one compound of formula (6) to provide a compound of formula (1d) and optionally reacting the compound of formula (1d) with at least one amine of formula (8) to provide a compound of formula (1e);

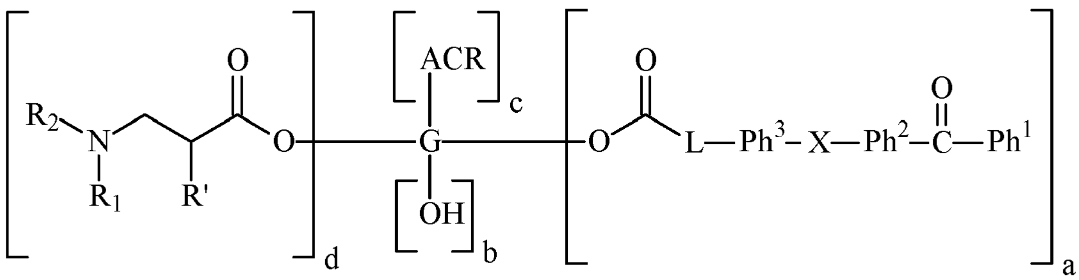

(1)

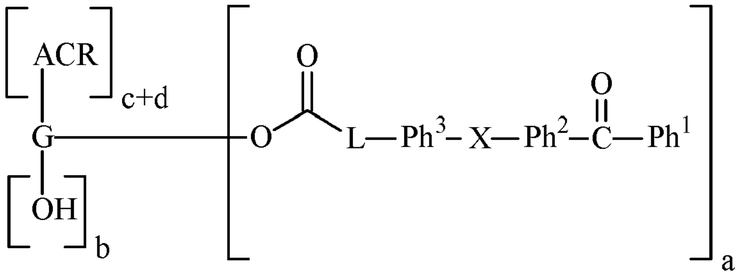
(1a)

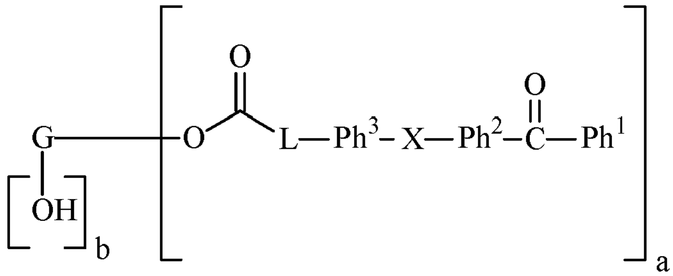
(1b)

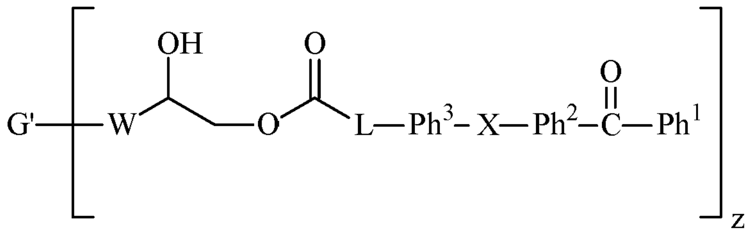
(1c)

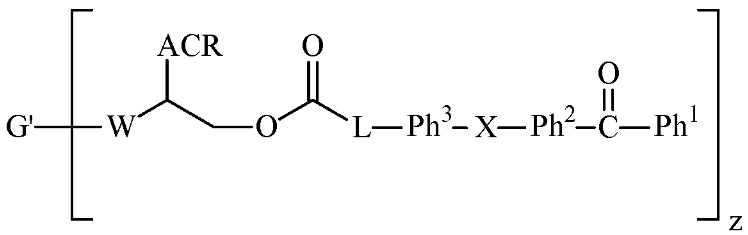
(1d)

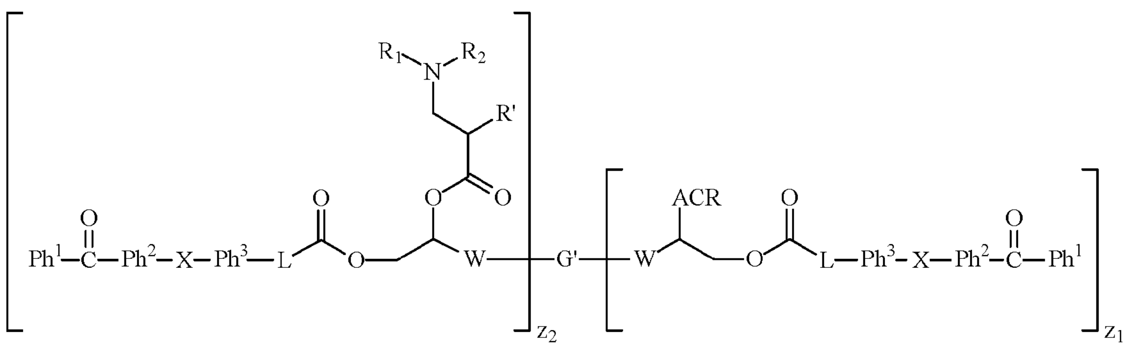
(1e)

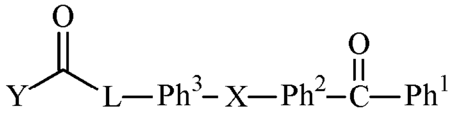
(3)

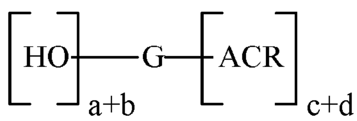
(4)

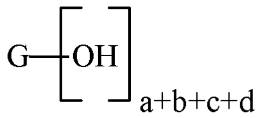 (5) 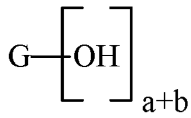 (5a) 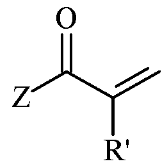 (6)

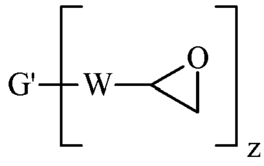 (7) 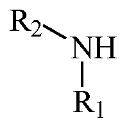 (8)

wherein:

G, G', L, ACR, $Ph^1$, $Ph^2$, $Ph^3$, R', $R_1$, $R_2$, W, X, a, b, c, d and z are as defined herein;
each Y and Z is independently OH, a halogen atom or -O-C(=O)-J;
J is alkyl or aryl, in particular tert-butyl
$z_1+z_2=z$.

The reaction of the at least one precursor of formula (3) with at least one product of formula (5) or (7) and at least one product of formula (6) may be carried out simultaneously or in successive steps (i.e. in a first step the at least one precursor of formula (3) reacts with at least one product of formula (5) or (7) and in a second step at least one product of formula (6) is added to the resulting product).

The above reactions may be carried out in the presence of one or more compounds selected from:

- a catalyst, in particular a catalyst chosen from a zirconium catalyst, a titanium catalyst, a quaternary ammonium salt and an acid catalyst, preferably chosen from a zirconium (IV) catalyst, a titanium (IV) catalyst, a tetraalkylammonium salt, sulfuric acid or a sulfonic acid, more preferably chosen from zirconium(IV) acetylacetonate, titanium(IV) isopropoxide, titanium (IV) oxyacetylacetonate, tetrabutylammonium bromide, tetrabutylammonium chloride, sulfuric acid, methanesulfonic acid, para-toluenesulfonic acid or trifluoromethanesulfonic acid;
- a solvent, in particular a solvent selected from toluene, xylene, acetonitrile, acetone, tetrahydrofuran and mixtures thereof;
- a co-solvent such as N-methylpyrrolidinone, N,N-dimethylformamide, *N*,*N*-dimethylacetamide, dimethyl sulfoxide, sulfolane and mixtures thereof.
- a stabilizer/polymerization inhibitor, in particular a polymerization inhibitor selected from hydroquinone (HQ), hydroquinone monomethyl ether (MEHQ, 4-methoxyphenol), 4-tert-butylcatechol (TBC), 3,5-di-tertiobutyl-4-hydroxytoluene (BHT), phenothiazine (PTZ), TEMPOL (4-hydroxy-2,2,6,6-tetramethylpiperidin-1-oxyl) and mixtures thereof.

Said reactions may be carried out in the presence of a zirconium catalyst, a titanium catalyst or an acid catalyst when the reaction corresponds to an esterification, amidification or thioesterification reaction. Said reactions may be carried out in the presence of a quaternary ammonium salt as the catalyst when the reaction corresponds to the opening of an epoxy ring. Said reactions may be carried out at a temperature from 70 to 140°C and preferably from 90 to 130°C. Said reaction may be carried out for a duration from 1 to 72 hours and preferably from 4 to 24 hours. Said reactions may be carried out so as to eliminate the water that is formed during the reaction. For example, the reaction may be carried out in a reactor equipped with a condenser (i.e. Dean-Stark) and the reaction medium may be heated at a temperature sufficient to evaporate the water (optionally as an azeotropic mixture with a solvent). Once the reaction is finished, the reaction medium may be washed one or more times with an aqueous solution, for example an aqueous solution of sodium chloride. The solvent(s) may be evaporated from the resulting organic phase.

In one embodiment, the photoinitiator of formula (1) may be prepared by a process comprising reacting at least one precursor of formula (3) with at least one compound of formula (4):

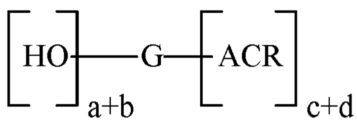 (4)

wherein G, ACR, a, b, c and d are as defined above.

Preferably, the compound of formula (4) is a partially (meth)acrylated polyol and the polyol is as listed above for $P_{OH}$.

Such a process provides a photoinitiator of formula (1a):

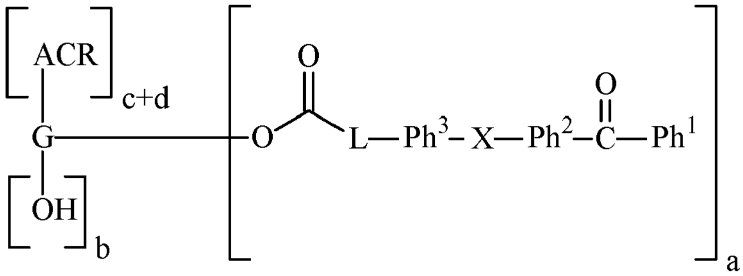

(1a)

wherein G, L, ACR, $Ph^1$, $Ph^2$, $Ph^3$, X, a, b, c and d are as defined herein.

When the photoinitiator of formula (1) has a group of formula -O-C(=O)-CH(R')-$CH_2$-$NR_1R_2$ (i.e. d is other than 0), the process may further comprise a step of reacting at least part of the ACR groups of the photoinitiator of formula (1a) with an amine of formula (8):

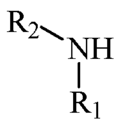

(8)

wherein $R_1$ and $R_2$ are as defined above.

This step corresponds to a Michael addition of an amine of formula (8) on at least part of the ACR groups of the photoinitiator of formula (1a). In such a case, the ACR groups are preferably acrylate groups.

Examples of suitable amines of formula (8) are amines bearing a primary or secondary amine group and optionally a tertiary amine group. The amine-containing compound may comprise more than one primary and/or secondary amine groups. The amine-containing compound may be selected from monoethanolamine (2-aminoethanol), 2-ethylhexylamine, octylamine, cyclohexylamine, sec-butylamine, isopropylamine, diethylamine, diethanolamine, dipropylamine, dibutylamine, 2-(methylamino)ethanol, 2-methoxyethylamine, bis(2-hydroxypropyl)amine, diisopropylamine, dipentylamine, dihexylamine, bis(2-ethylhexyl)amine, 1,2,3,4-tetrahydroisoquinoline, N-benzylmethylamine, morpholine, piperidine, dioctylamine, di-cocoamine, dimethylaminopropylamine, N,N-bis-(dimethylaminopropyl)-N-(3-aminopropyl)-amine, 1-[3-(dimethylamino)propyl]piperazine, (aminomethyl)cyclohexane, an optionally substituted aniline, an optionally substituted N-alkylaniline and an optionally substituted benzocaine (ethyl-4-aminobenzoate), and mixtures thereof. Preferably, the amine of formula (8) is selected from diethylamine, dipropylamine, dibutylamine, diethanolamine, dioctylamine, bis(2-ethylhexyl)amine, piperidine, morpholine, and mixtures thereof.

Alternatively, the photoinitiator of formula (1) may be prepared by a process comprising reacting at least one precursor of formula (3) with at least one compound of formula (5a):

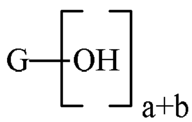

(5a)

wherein G, a and b are as defined herein.

Preferably, the compound of formula (4a) is a polyol as listed above for $P_{OH}$.

Such a process provides a photoinitiator of formula (1b):

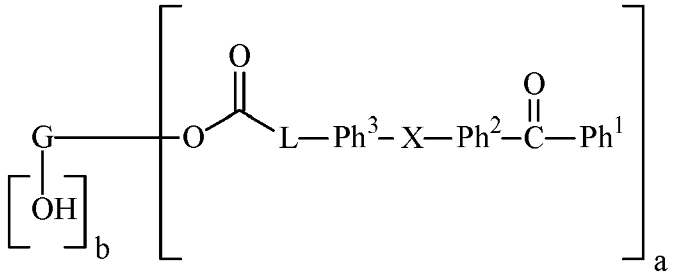

(1b)

wherein G, L, $Ph^1$, $Ph^2$, $Ph^3$, X, a, b, c and d are as defined herein.

The photoinitiator of formula (1b) corresponds to a photoinitiator of formula (1) wherein c and d are both 0.

Alternatively, the photoinitiator of formula (1) may be prepared by a process comprising reacting at least one precursor of formula (3) with at least one compound of formula (6) and at least one compound of formula (7):

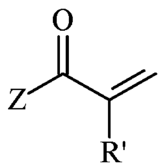

(6)

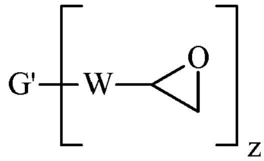

(7)

wherein G', R', W, Z and z are as defined above.

Preferably, the compound of formula (7) is a polyepoxide as listed above for $P_{EPOX}$.

Such a process provides a photoinitiator of formula (1c):

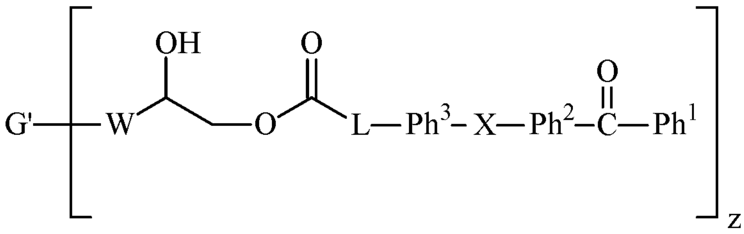

(1c)

wherein G', L, $Ph^1$, $Ph^2$, $Ph^3$, W, X and z are as defined herein.

The photoinitiator of formula (1c) corresponds to a photoinitiator of formula (1) wherein c and d are both 0 and G is according to formula (34).

Alternatively, the process may comprise reacting at least one precursor of formula (3) with at least one compound of formula (7) and at least one compound of formula (6) to provide a photoinitiator of formula (1d):

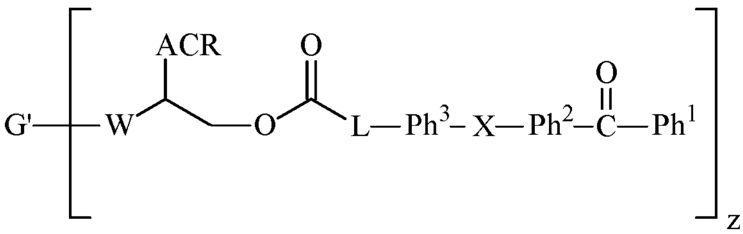

(1d)

wherein G', ACR, L, $Ph^1$, $Ph^2$, $Ph^3$, W, X and z are as defined herein.

The photoinitiator of formula (1c) corresponds to a photoinitiator of formula (1) wherein b and d are both 0, c is at least 2 and G is according to formula (34).

The process may further comprise a step of reacting at least part of the ACR groups of the photoinitiator of formula (1d) with an amine of formula (8) to provide a photoinitiator of formula (1e):

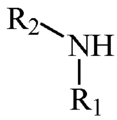 (8)

wherein $R_1$ and $R_2$ are as defined above.

This step corresponds to a Michael addition of an amine of formula (8) on at least part of the ACR groups of the photoinitiator of formula (1d). In such a case, the ACR groups are preferably acrylate groups.

## Precursor

All the particular and preferred embodiments described above for the photoinitiator of formula (1) equally apply to the following precursor.

The invention also relates to a precursor that can be used to obtain the photoinitiator of formula (1). The precursor is according to formula (3):

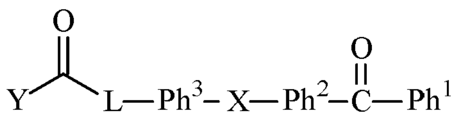 (3)

wherein:

- L, $Ph^1$, $Ph^2$, $Ph^3$, X are as defined herein;
- Y is OH, a halogen atom or -O-C(=O)-J;
- J is alkyl or aryl, in particular tert-butyl.

Non-limiting examples of photoinitiators of formula (3) are detailed below:

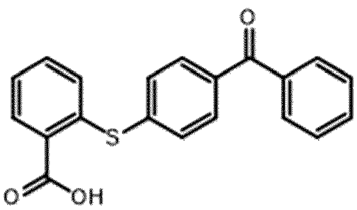
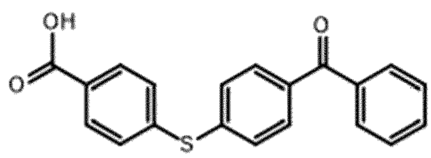
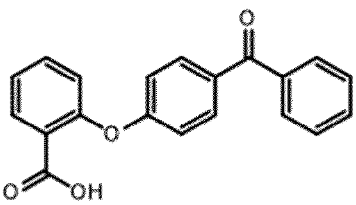
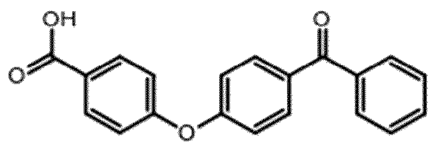
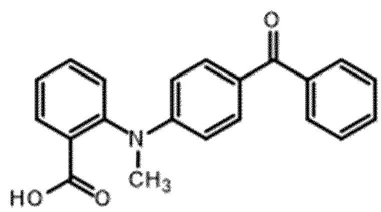
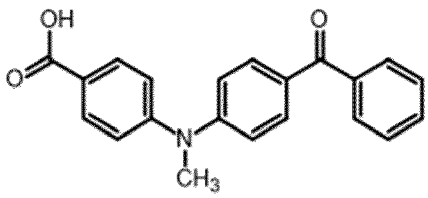

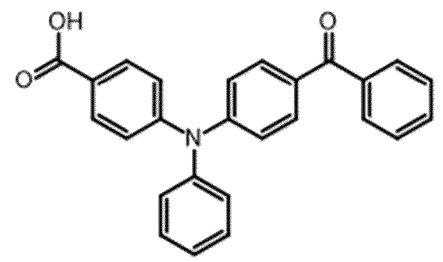
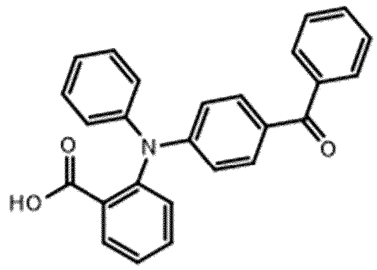

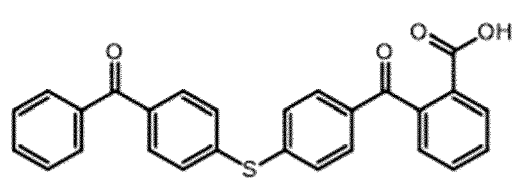
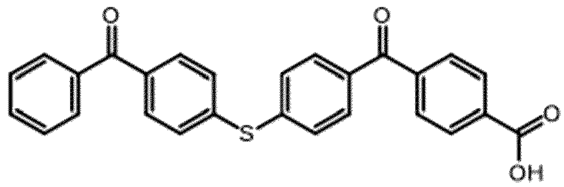

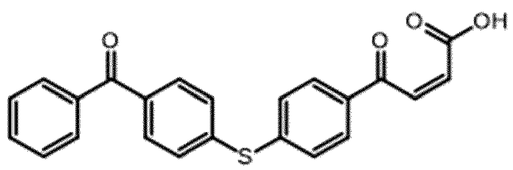
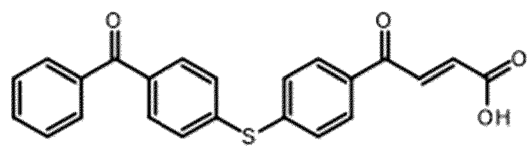

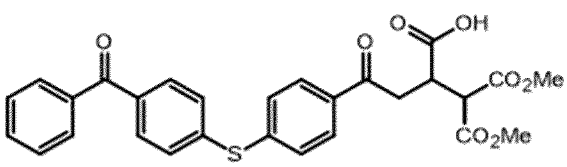
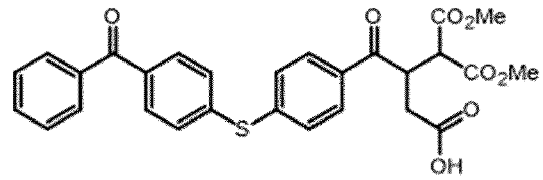

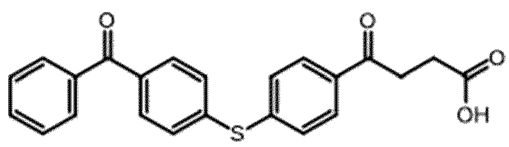
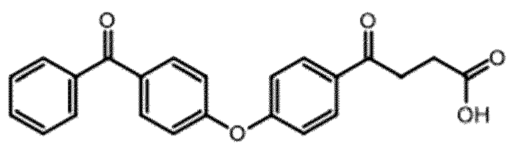

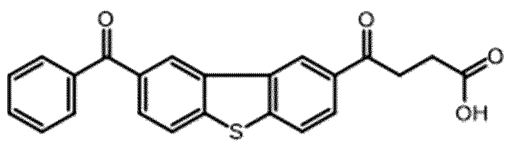
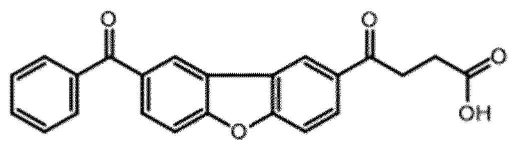

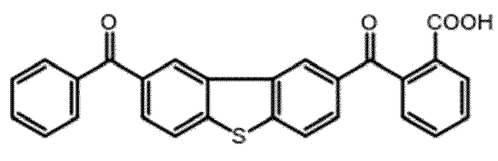
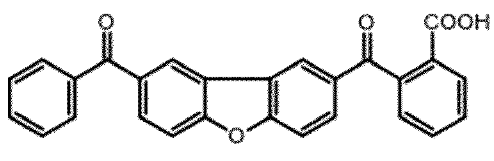

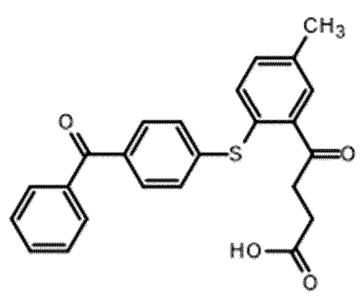
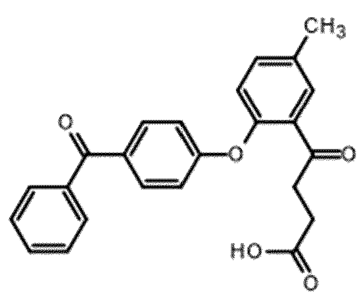
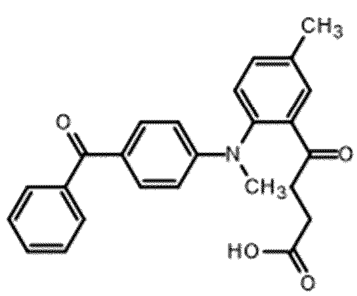

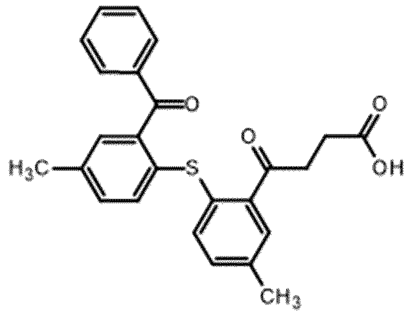

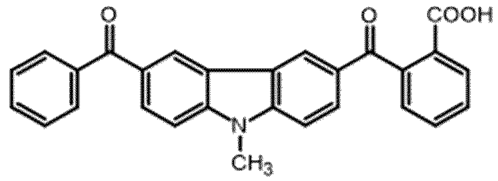

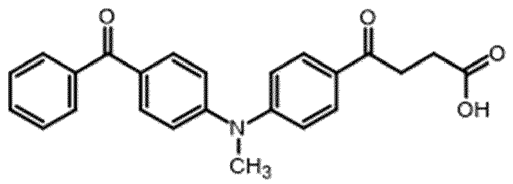

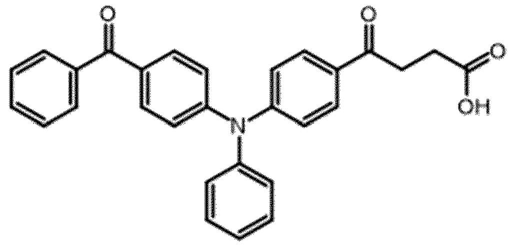

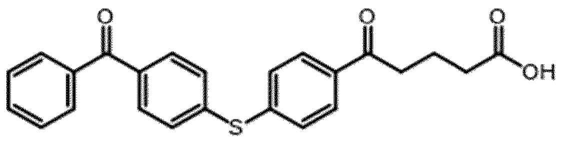

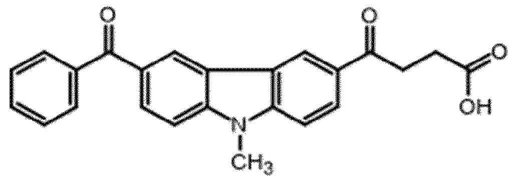

**Photoinitiator composition**

The present disclosure also relates to a photoinitiator composition comprising a mixture of at least two photoinitiators of formula (1) according to the invention.

Advantageously, a photoinitiator composition comprising a mixture of such photoinitiators may be liquid at 20°C.

In addition, the present disclosure relates to a photoinitiator composition comprising a photoinitiator of formula (1) according to the invention and a photoinitiator other than a photoinitiator of formula (1).

The photoinitiator other than a photoinitiator of formula (1) may be a photoinitiator having Norrish type I activity and/or Norrish type II activity, more particularly a photoinitiator having Norrish type II activity.

In particular, the other photoinitiator may be used to sensitize the photoinitiator of formula (1), i.e. to improve the photoinitiating activity of the photoinitiator of formula (1)and/or to shift the photoabsorbance of the photoinitiator of formula (1) to a wavelength in the range of from 340 to 420 nm.

Non-limiting types of other photoinitiators suitable for use in the photoinitiator composition of the invention include, for example, benzoins, benzoin ethers, acetophenones, α-hydroxy acetophenones, benzil, benzil ketals, anthraquinones, phosphine oxides, acylphosphine oxides, α-hydroxyketones, phenylglyoxylates, α-aminoketones, benzophenones, thioxanthones, xanthones, acridine derivatives, phenazine derivatives, quinoxaline derivatives, triazine compounds, benzoyl formates, aromatic oximes, metallocenes, acylsilyl or acylgermanyl compounds, camphorquinones, polymeric derivatives thereof, and mixtures thereof.

Examples of suitable other photoinitiators include, but are not limited to, 2-methylanthraquinone, 2-ethylanthraquinone, 2-chloroanthraquinone, 2-benzyanthraquinone, 2-t-butylanthraquinone, 1,2-benzo-9,10-anthraquinone, benzoin ethers, benzoin, benzoin methyl ether, benzoin ethyl ether, benzoin isopropyl ether, alpha-methylbenzoin, alpha-phenylbenzoin, Michler's ketone, acetophenones such as 2,2-dialkoxybenzophenones and 1-hydroxyphenyl ketones, benzophenone, 4,4'-bis-(diethylamino) benzophenone, acetophenone, 2,2-diethyloxyacetophenone, , 2-isopropylthioxanthone, thioxanthone, diethyl thioxanthone, 1,5-acenaphthylene, benzil, α-hydroxyketone, 2,4,6-trimethylbenzoyldiphenyl phosphine oxide, , 2,2-dimethoxy-1,2-diphenylethanone, 1-hydroxycyclohexyl phenyl ketone, 2-methyl-1-[4-(methylthio) phenyl]-2-morpholinopropanone, 2-hydroxy-2-methyl-1-phenyl-propanone, oligomeric α-hydroxy ketone, benzoyl phosphine oxides, phenylbis(2,4,6-trimethylbenzoyl)phosphine oxide, ethyl(2,4,6-trimethylbenzoyl)phenyl phosphinate, anisoin, anthraquinone, anthraquinone-2-sulfonic acid sodium salt monohydrate, (benzene) tricarbonylchromium, , benzoin isobutyl ether, benzophenone/1-hydroxycyclohexyl phenyl ketone 50/50 blend, 3,3',4,4'-benzophenonetetracarboxylic dianhydride, 4-benzoylbiphenyl, 2-benzyl-2-(dimethylamino)-4'-morpholinobutyrophenone, 4,4'-bis(diethylamino)benzophenone, 4,4'-bis(dimethylamino)benzophenone, camphorquinone, 2-chlorothioxanthen-9-one, dibenzosuberenone, 4,4'-dihydroxybenzophenone, , 4-(dimethylamino)benzophenone, 4,4'-dimethylbenzil, 2,5-dimethylbenzophenone, 3,4-dimethylbenzophenone, diphenyl(2,4,6-trimethylbenzoyl)phosphine oxide /2-hydroxy-2-methylpropiophenone 50/50 blend, 4'-ethoxyacetophenone, 2,4,6-trimethylbenzoyldiphenylphosphine oxide, , 3'-hydroxyacetophenone, 4'-hydroxyacetophenone, 3-hydroxybenzophenone, 4-hydroxybenzophenone, 2-methylbenzophenone, 3-methylbenzophenone, methybenzoylformate, phenanthrenequinone, 4'-phenoxyacetophenone, (cumene) cyclopentadienyl iron(ii) hexafluorophosphate, 9,10-diethoxy and 9,10-dibutoxyanthracene, 2-ethyl-9,10-dimethoxyanthracene, and combinations thereof.

In particular, the other photoinitiator may be a thioxanthone, more particularly a thioxanthone selected from a polymeric thioxanthone (such as SpeedCure® 7010), a polymerizable thioxanthone (such as those disclosed in WO 2022/157274), isopropyl thioxanthone (such as SpeedCure® ITX), 2,4-diethylthioxanthone (such as SpeedCure® DETX), 1-chloro-4-propoxythioxanthone (such as SpeedCure® CPTX) and mixtures thereof.

## Photopolymerization process

The photoinitiator of formula (1) as defined above or the photoinitiator composition as defined above may be used in a photopolymerization process, i.e. a process for photopolymerizing (e.g. curing) one or more ethylenically unsaturated compounds.

The process for photopolymerizing one or more ethylenically unsaturated compounds comprises contacting one or more ethylenically unsaturated compounds with a photoinitiator of formula (1) according to the invention or a photoinitiator composition according to the invention, and irradiating the mixture, in particular with UV, near-UV, visible, infrared and/or near-infrared radiation.

The ethylenically unsaturated compound(s) may be as defined below.

## Curable composition

The curable (or polymerizable) composition of the invention comprises a photoinitiator of formula (1) according to the invention or a photoinitiator composition according to the invention, referred to as component a). The curable composition of the invention further comprises an ethylenically unsaturated component, referred to as component b).

The curable composition of the invention may comprise:

- from 0.5 to 30%, preferably from 1 to 25%, more preferably from 2 to 20%, more preferably 3 to 15%, of component a);
- from 70 to 99.5%, preferably from 75 to 99%, more preferably from 80 to 98%, more preferably from 85 to 97%, of component b);

the % being % by weight based on the total weight of components a) and b).

The curable composition of the invention may further comprise one or more compounds selected from:

- an amine synergist;
- an additive; and
- a solvent.

### *Ethylenically unsaturated component*

Component b) is an ethylenically unsaturated component. An ethylenically unsaturated component comprises at least one ethylenically unsaturated compound. An ethylenically unsaturated component may comprise a mixture of ethylenically unsaturated compounds.

As used herein, the term "ethylenically unsaturated compound" means a compound that comprises a polymerizable carbon-carbon double bond. A polymerizable carbon-carbon double bond is a carbon-carbon double bond that can react with another carbon-carbon double bond in a polymerization reaction. A polymerizable carbon-carbon double bond is generally comprised in a group selected from acrylate, methacrylate, cyanoacrylate, acrylamide, methacrylamide, styrene, maleate, fumarate, itaconate, allyl, propenyl, vinyl and combinations thereof, preferably selected from acrylate, methacrylate, cyanoacrylate, allyl and vinyl, more preferably selected from acrylate and methacrylate. The carbon-carbon double bonds of a phenyl ring are not considered as polymerizable carbon-carbon double bonds.

Component b) may comprise at least one ethylenically unsaturated compound selected from a (meth)acrylate-functionalized monomer, a (meth)acrylate-functionalized oligomer, an amine-modified acrylate and mixtures thereof. Preferably, component b) comprise at least one ethylenically unsaturated compound selected from a (meth)acrylate-functionalized monomer, an amine-modified acrylate and mixtures thereof.

As used herein, the term "(meth)acrylate-functionalized monomer" means a monomer comprising a (meth) acrylate group, in particular an acrylate group. The term "(meth)acrylate-functionalized oligomer" means an oligomer comprising a (meth)acrylate group, in particular an acrylate group. The term "(meth)acrylate group" encompasses acrylate groups ($-O-CO-CH=CH_2$) and methacrylate groups ($-O-CO-C(CH_3)=CH_2$).

The total amount of ethylenically unsaturated component (including (meth)acrylate functionalized monomer, (meth)acrylate functionalized oligomer and amine-modified acrylate) in the curable composition may be from 40 to 99.5%, in particular 50 to 95%, more particularly 60 to 90%, by weight based on the weight of the composition. In particular, the curable composition may comprise 40 to 80%, or 40 to 75% or 40 to 70% or 40 to 65% or 40 to 60% by weight of ethylenically unsaturated component based on the weight of the composition. Alternatively, the curable composition may comprise 60 to 99.5%, or 65 to 99.5% or 70 to 99.5% or 75 to 99.5% or 80 to 99.5% by weight of ethylenically unsaturated component based on the weight of the composition.

Component b) may comprise at least one (meth)acrylate-functionalized monomer. Component b) may comprise a mixture of (meth)acrylate-functionalized monomers.

A (meth)acrylate-functionalized monomer may have a molecular weight of less than 600 g/mol, in particular from 100 to 550 g/mol, more particularly 200 to 500 g/mol.

A (meth)acrylate-functionalized monomer may have 1 to 6 (meth)acrylate groups, in particular 1 to 3 (meth) acrylate groups.

Component b) may comprise a mixture of (meth)acrylate-functionalized monomers having different functionalities. For example, component b) may comprise a mixture of a (meth)acrylate-functionalized monomer containing a single acrylate or methacrylate group per molecule (referred to herein as "mono(meth)acrylate-functionalized compounds") and a (meth)acrylate-functionalized monomer containing 2 or more, preferably 2 or 3, acrylate and/or methacrylate groups per molecule.

In one embodiment, component b) comprises a mono(meth)acrylate-functionalized monomer. The mono(meth) acrylate-functionalized monomer may advantageously function as a reactive diluent and reduce the viscosity of the composition.

Examples of suitable mono(meth)acrylate-functionalized monomers include, but are not limited to, mono-(meth) acrylate esters of aliphatic alcohols (wherein the aliphatic alcohol may be straight chain, branched or alicyclic and may be a mono-alcohol, a di-alcohol or a polyalcohol, provided only one hydroxyl group is esterified with (meth)acrylic acid); mono-(meth)acrylate esters of aromatic alcohols (such as phenols, including alkylated phenols); mono-(meth)acrylate esters of alkylaryl alcohols (such as benzyl alcohol); mono-(meth)acrylate esters of oligomeric and polymeric glycols such as diethylene glycol, triethylene glycol, dipropylene glycol, tripropylene glycol, polyethylene glycol, and polypropylene glycol); mono-(meth)acrylate esters of monoalkyl ethers of glycols and oligoglycols; mono-(meth)acrylate esters of alkoxylated (e.g., ethoxylated and/or propoxylated) aliphatic alcohols (wherein the aliphatic alcohol may be straight chain, branched or alicyclic and may be a mono-alcohol, a di-alcohol or a polyalcohol, provided only one hydroxyl group of the alkoxylated aliphatic alcohol is esterified with (meth)acrylic acid); mono-(meth)acrylate esters of alkoxylated (e.g., ethoxylated and/or propoxylated) aromatic alcohols (such as alkoxylated phenols); caprolactone mono(meth)acrylates; and the like.

The following compounds are specific examples of mono(meth)acrylate-functionalized monomers suitable for use in component b): methyl (meth)acrylate; ethyl (meth)acrylate; n-propyl (meth)acrylate; n-butyl (meth)acrylate; isobutyl (meth)acrylate; n-hexyl (meth)acrylate; 2-ethylhexyl (meth)acrylate; n-octyl (meth)acrylate; isooctyl (meth)acrylate; n-decyl (meth)acrylate; n-dodecyl (meth)acrylate; tridecyl (meth)acrylate; tetradecyl (meth)acrylate; hexadecyl (meth) acrylate; 2-hydroxyethyl (meth)acrylate; 2- and 3-hydroxypropyl (meth)acrylate; 2-methoxyethyl (meth)acrylate; 2-ethoxyethyl (meth)acrylate; 2- and 3-ethoxypropyl (meth)acrylate; tetrahydrofurfuryl (meth)acrylate; alkoxylated tetrahydrofurfuryl (meth)acrylate; 2-(2-ethoxyethoxy)ethyl (meth)acrylate; cyclohexyl (meth)acrylate; glycidyl (meth)acrylate; isodecyl (meth)acrylate; lauryl (meth)acrylate; 2-phenoxyethyl (meth)acrylate; alkoxylated phenol (meth)acrylates; alkoxylated nonylphenol (meth)acrylates; cyclic trimethylolpropane formal (meth)acrylate; isobornyl (meth)acrylate; tricyclodecanemethanol (meth)acrylate; tert-butylcyclohexanol (meth)acrylate; trimethylcyclohexanol (meth)acrylate; diethylene glycol monomethyl ether (meth)acrylate; diethylene glycol monoethyl ether (meth)acrylate; diethylene glycol monobutyl ether (meth)acrylate; triethylene glycol monoethyl ether (meth)acrylate; ethoxylated lauryl (meth)acrylate; methoxy polyethylene glycol (meth)acrylates; hydroxyl ethyl-butyl urethane (meth)acrylates; 3-(2-hydroxyalkyl)oxazolidinone (meth)acrylates; and combinations thereof.

In one embodiment, component b) may comprise a (meth)acrylate-functionalized monomer containing two or more (meth)acrylate groups per molecule.

Examples of suitable (meth)acrylate-functionalized monomers containing two or more (meth)acrylate groups per molecule include acrylate and methacrylate esters of polyols. Examples of suitable polyols are as listed above for $P_{OH}$. Such polyols may be fully or partially esterified (with (meth)acrylic acid, (meth)acrylic anhydride, (meth)acryloyl chloride or the like), provided they contain at least two (meth)acrylate functional groups per molecule.

Exemplary (meth)acrylate-functionalized monomers containing two or more (meth)acryloyloxy groups per

molecule may include bisphenol A di(meth)acrylate; hydrogenated bisphenol A di(meth)acrylate; ethylene glycol di(meth) acrylate; diethylene glycol di(meth)acrylate; triethylene glycol di(meth)acrylate; tetraethylene glycol di(meth)acrylate; polyethylene glycol di(meth)acrylate; propylene glycol di(meth)acrylate; dipropylene glycol di(meth)acrylate; tripropylene glycol di(meth)acrylate; tetrapropylene glycol di(meth)acrylate; polypropylene glycol di(meth)acrylate; polytetramethylene glycol di(meth)acrylate; 1,2-butanediol di(meth)acrylate; 2,3-butanediol di(meth)acrylate; 1,3-butanediol di(meth) acrylate; 1,4-butanediol di(meth)acrylate; 1,5-pentanediol di(meth)acrylate; 1,6-hexanediol di(meth)acrylate; 1,8-octanediol di(meth)acrylate; 1,9-nonanediol di(meth)acrylate; 1,10-nonanediol di(meth)acrylate; 1,12-dodecanediol di(meth) acrylate; neopentyl glycol di(meth)acrylate; 2-methyl-2,4-pentanediol di(meth)acrylate; polybutadiene di(meth)acrylate; cyclohexane-1,4-dimethanol di(meth)acrylate; tricyclodecane dimethanol di(meth)acrylate; metallic di(meth)acrylates; modified metallic di(meth)acrylates; glyceryl di(meth)acrylate; glyceryl tri(meth)acrylate; trimethylolethane tri(meth) acrylate; trimethylolethane di(meth)acrylate; trimethylolpropane tri(meth)acrylate; trimethylolpropane di(meth)acrylate; pentaerythritol di(meth)acrylate; pentaerythritol tri(meth)acrylate; pentaerythritol tetra(meth)acrylate, di(trimethylolpropane) diacrylate; di(trimethylolpropane) triacrylate; di(trimethylolpropane) tetraacrylate, sorbitol penta(meth)acrylate; di(pentaerythritol) tetraacrylate; di(pentaerythritol) pentaacrylate; di(pentaerythritol) hexa(meth)acrylate; tris (2-hydroxyethyl) isocyanurate tri(meth)acrylate; as well as the alkoxylated (e.g., ethoxylated and/or propoxylated) derivatives thereof; and combinations thereof.

Component b) may comprise 0 to 99.5%, in particular 5 to 90%, more particularly 10 to 80%, even more particularly 15 to 75%, more particularly still 20 to 70% by weight of (meth)acrylate-functionalized monomer based on the total weight of the component b). In particular, component b) may comprise 5 to 50% or 10 to 50% or 15 to 50% or 20 to 50% or 25 to 50% or 30 to 50%, by weight of (meth)acrylate-functionalized monomer based on the total weight of component b). Alternatively, component b) may comprise 50 to 99.5% or 55 to 99.5% or 60 to 99.5% or 65 to 99.5% or 70 to 99.5%, by weight of (meth)acrylate-functionalized monomer based on the total weight of component b).

Component b) may comprise at least one (meth)acrylate-functionalized oligomer. Component b) may comprise a mixture of (meth)acrylate-functionalized oligomers.

A (meth)acrylate-functionalized oligomer may be selected in order to enhance the flexibility, strength and/or modulus, among other attributes, of a cured product obtained by curing the curable composition.

A (meth)acrylate functionalized oligomer may have 1 to 18 (meth)acrylate groups, in particular 2 to 6 (meth) acrylate groups, more particularly 2 to 6 acrylate groups. A (meth)acrylate functionalized oligomer may have a number average molecular weight equal or more than 600 g/mol, in particular 800 to 15,000 g/mol, more particularly 1,000 to 5,000 g/mol.

In particular, component b) may comprise at least one (meth)acrylate-functionalized oligomer selected from the group consisting of (meth)acrylate-functionalized urethane oligomers (sometimes also referred to as "urethane (meth) acrylate oligomers," "polyurethane (meth)acrylate oligomers" or "carbamate (meth)acrylate oligomers"), (meth)acrylate-functionalized epoxy oligomers (sometimes also referred to as "epoxy (meth)acrylate oligomers"), (meth)acrylate-functionalized polyether oligomers (sometimes also referred to as "polyether (meth)acrylate oligomers"), (meth)acrylate-functionalized polydiene oligomers (sometimes also referred to as "polydiene (meth)acrylate oligomers"), (meth) acrylate-functionalized polycarbonate oligomers (sometimes also referred to as "polycarbonate (meth)acrylate oligomers"), and (meth)acrylate-functionalized polyester oligomers (sometimes also referred to as "polyester (meth)acrylate oligomers") and mixtures thereof.

Preferably, component b) comprises at least one (meth)acrylate-functionalized urethane oligomer, more preferably at least one acrylate-functionalized urethane oligomer.

Advantageously, component b) comprises at least one (meth)acrylate-functionalized urethane oligomer having two (meth)acrylate groups, more preferably at least one acrylate-functionalized urethane oligomer having two acrylate groups.

Exemplary polyester (meth)acrylate oligomers include the reaction products of acrylic or methacrylic acid or mixtures or synthetic equivalents thereof with hydroxyl group-terminated polyester polyols. The reaction process may be conducted such that all or essentially all of the hydroxyl groups of the polyester polyol have been (meth)acrylated, particularly in cases where the polyester polyol is difunctional. The polyester polyols can be made by polycondensation reactions of polyols (in particular, diols) and polycarboxylic acid functional compounds (in particular, dicarboxylic acids and anhydrides). The polyol and polycarboxylic acid functional compounds can each have linear, branched, cycloaliphatic or aromatic structures and can be used individually or as mixtures.

Examples of suitable epoxy (meth)acrylates include the reaction products of acrylic or methacrylic acid or mixtures thereof with an epoxy resin (polyglycidyl ether or ester). The epoxy resin may be as disclosed herein for $P_{EPOX}$.

Suitable polyether (meth)acrylate oligomers include, but are not limited to, the condensation reaction products of acrylic or methacrylic acid or synthetic equivalents or mixtures thereof with polyetherols which are polyether polyols (such as polyethylene glycol, polypropylene glycol or polytetramethylene glycol). Suitable polyetherols can be linear or branched substances containing ether bonds and terminal hydroxyl groups. Polyetherols can be prepared by ring opening polymerization of cyclic ethers such as tetrahydrofuran or alkylene oxides (e.g., ethylene oxide and/or propylene

oxide) with a starter molecule. Suitable starter molecules include water, polyhydroxyl functional materials, polyester polyols and amines.

Polyurethane (meth)acrylate oligomers (sometimes also referred to as "urethane (meth)acrylate oligomers") suitable for use in the curable composition include urethanes based on aliphatic, cycloaliphatic and/or aromatic polyester polyols and polyether polyols and aliphatic, cycloaliphatic and/or aromatic polyester diisocyanates and polyether diisocyanates capped with (meth)acrylate end-groups. Suitable polyurethane (meth)acrylate oligomers include, for example, aliphatic polyester-based urethane di- and tetra-acrylate oligomers, aliphatic polyether-based urethane di- and tetra-acrylate oligomers, as well as aliphatic polyester/polyether-based urethane di- and tetra-acrylate oligomers.

The polyurethane (meth)acrylate oligomers may be prepared by reacting an aliphatic, cycloaliphatic and/or aromatic polyisocyanate (e.g., diisocyanate, triisocyanate) with a polyol (e.g. a polyester polyol, polyether polyol, polycarbonate polyol, polycaprolactone polyol, polyorganosiloxane polyol, polydiene polyol or combinations thereof) to form isocyanate-functionalized oligomers which are then reacted with hydroxyl-functionalized (meth)acrylates (such as hydroxyethyl (meth)acrylate or 2- or 3-hydroxypropyl (meth)acrylate) to provide terminal (meth)acrylate groups. For example, the polyurethane (meth)acrylate oligomers may contain two, three, four or more (meth)acrylate functional groups per molecule. Other orders of addition may also be practiced to prepare the polyurethane (meth)acrylate, as is known in the art. For example, the hydroxyl-functionalized (meth)acrylate may be first reacted with the polyisocyanate to obtain an isocyanate-functionalized (meth)acrylate, which may then be reacted with the polyol. In yet another embodiment, the polyisocyanate may be first reacted with the polyol to obtain an isocyanate-functionalized polyol, which is thereafter reacted with the hydroxyl-functionalized (meth)acrylate. Alternatively, all the components may be combined and reacted at the same time.

Suitable acrylic (meth)acrylate oligomers (sometimes also referred to in the art as "acrylic oligomers") include oligomers which may be described as substances having an oligomeric acrylic backbone which is functionalized with one or (meth)acrylate groups (which may be at a terminus of the oligomer or pendant to the acrylic backbone). The acrylic backbone may be a homopolymer, random copolymer or block copolymer comprised of repeating units of acrylic monomers. The acrylic monomers may be any monomeric (meth)acrylate such as C1-C6 alkyl (meth)acrylates as well as functionalized (meth)acrylates such as (meth)acrylates bearing hydroxyl, carboxylic acid and/or epoxy groups. Acrylic (meth)acrylate oligomers may be prepared using any procedures known in the art, such as by oligomerizing monomers, at least a portion of which are functionalized with hydroxyl, carboxylic acid and/or epoxy groups (e.g., hydroxyalkyl(meth) acrylates, (meth)acrylic acid, glycidyl (meth)acrylate) to obtain a functionalized oligomer intermediate, which is then reacted with one or more (meth)acrylate-containing reactants to introduce the desired (meth)acrylate functional groups.

Component b) may comprise 0 to 99.5 %, in particular 5 to 90 %, more particularly 10 to 80 %, even more particularly 15 to 75 %, more particularly still 20 to 70 % by weight of (meth)acrylate-functionalized oligomer based on the total weight of the component b). In particular, component b) may comprise 5 to 50 % or 10 to 50 % or 15 to 50 % or 20 to 50 % or 25 to 50 % or 30 to 50 %, by weight of (meth)acrylate-functionalized oligomer based on the total weight of component b). Alternatively, component b) may comprise 50 to 99.5 % or 55 to 99.5 % or 60 to 99.5 % or 65 to 99.5 % or 70 to 99.5 %, by weight of (meth)acrylate-functionalized oligomer based on the total weight of component b).

Component b) may comprise at least one amine-modified acrylate. Component b) may comprise a mixture of amine-modified acrylates.

An amine-modified acrylate is obtained by reacting an acrylate-functionalized compound with an amine-containing compound (aza-Michael addition). An amine-modified acrylate comprises at least one remaining acrylate group (i.e. an acrylate group that has not reacted with the amine-containing compound during the aza-Michael addition) and/or at least one (meth)acrylate group (which may not be reactive towards primary or secondary amines).

The acrylate-functionalized compound may be an acrylate-functionalized monomer and/or acrylate-functionalized oligomer as defined above.

The amine-containing compound may be as defined above for the amine of formula (8).

Examples of commercially available amine-modified acrylates include CN3705, CN3715, CN3755, CN381, CN386 and LM7401, all available from Arkema. Polymeric or multi-amino versions are also suitable.

Component b) may comprise from 0% to 25%, in particular 2.5% to 20%, more particularly 5 to 15%, by weight of amine-modified acrylate based on the total weight of component b).

Component b) may comprise at least one ethylenically unsaturated compound other than a (meth)acrylate-functionalized monomer or oligomer. Examples of such ethylenically unsaturated compounds include:

- polyvinylic and/or polyallylic monomers (in particular divinyl benzene, 1,4-butanediol divinyl ether, tri(ethylene glycol) divinyl ether, diallyl ether, glycerol diallyl ether, glycerol triallyl ether, trimethylolpropane diallyl ether, trimethylolpropane triallyl ether, pentaerythritol triallyl ether, pentaerythritol tetraallyl ether, diallyl phthalate, triallyl isocyanurate, 2,4,6-triallyloxy-1,3,5-triazine, glyoxal bis(diallyl acetal) and mixtures thereof);
- vinyl esters of carboxylic acids (in particular vinyl acetate, vinyl propionate, vinyl hexanoate, vinyl 2-ethylhexanoate, vinyl octanoate, vinyl pelargonate, vinyl laurate, vinyl stearate, a vinyl ester of versatic acid and mixtures thereof);

- vinyl ethers (in particular vinyl methyl ether, vinyl ethyl ether, vinyl n-butyl ether, vinyl iso-butyl ether and mixtures thereof, ethylene glycol divinyl ether, triethylene glycol divinyl ether and trimethylolpropane trivinyl ether);
- cycloaliphatic vinyl monomers (in particular vinylcyclohexane);
- olefins (in particular ethylene, propene, 1-butene, isobutylene, diisobutylene, 1-nonene, 1-decene and mixtures thereof);
- conjugated dienes (in particular butadiene, isoprene, pentadiene, chlorodiene and mixtures thereof);
- vinyl aromatic monomers (in particular styrene, alpha-methylstyrene, tert-butylstyrene, ortho-, meta-, and para-methylstyrene, ortho-, meta- and para-ethylstyrene, o-methyl-p-isopropylstyrene, p-chlorostyrene, p-bromostyrene, o,p-dichlorostyrene, o,p-dibromostyrene, ortho-, meta- and para-methoxystyrene, optionally substituted indenes, optionally substituted vinylnaphthalenes, acenaphthylene, diphenylethylene, vinyl anthracene and mixtures thereof);
- mono- or dicarboxylic acid monomers, cyclic anhydride monomers and salts thereof (in particular 3-butenoic acid, crotonic acid, vinyl acetic acid, fumaric acid, maleic acid, maleic anhydride, tetrahydrophthalic acid, tetrahydrophthalic anhydride, itaconic acid, mesaconic acid, citraconic acid, glutaconic acid, muconic acid and mixtures thereof);
- unsaturated polymers such as polybutadiene;
- as well as the alkoxylated (e.g., ethoxylated and/or propoxylated) derivatives thereof
- and mixtures thereof.

*Amine synergist*

The curable composition of the present invention may comprise an amine synergist. The curable composition may comprise a mixture of amine synergists. When the compound of formula (1) has a group of formula -O-C(=O)-CH(R')-$CH_2$-$NR_1R_2$ (i.e. d is other than 0), the curable composition may not require an amine synergist. Indeed, in such a case, the group of formula -O-C(=O)-CH(R')-$CH_2$-$NR_1R_2$ may advantageously act as an amine synergist.

Amine synergists may be introduced in the curable composition of the present invention in order to act synergistically with Norrish Type II photoinitiators and/or reduce oxygen inhibition. Amine synergists are typically tertiary amines. When used in conjunction with Norrish Type II photoinitiators, the tertiary amine provides an active hydrogen donor site for the excited triple state of the photoinitiator, thus producing a reactive alkyl-amino radical which can subsequently initiate polymerization. Tertiary amines are able to convert unreactive peroxy species, formed by reaction between oxygen and free radicals, to reactive alkyl-amino radicals, thus reducing the effects of oxygen on curing.

When the curable composition comprises an amine-modified acrylate monomer or oligomer as defined above, an amine synergist may not need to be added to the composition.

Examples of suitable amine synergists include low-molecular weight tertiary amines (i.e. having a molecular weight of less than 200 g/mol) such as triethanol amine, N-methyldiethanol amine. Other types of amine synergists are aminobenzoates, polymerizable aminobenzoates, polymeric aminobenzoates and mixtures thereof. Examples of aminobenzoates include ethyl 4-(dimethylamino)benzoate (SpeedCure® EDB), pentyl 4-(dimethylamino)benzoate, 2-ethylhexyl 4-(dimethylamino)benzoate (SpeedCure® EHA), 2-butoxyethyl 4-(dimethylamino)benzoate (SpeedCure® BEDB), 2-(dimethylamino)ethyl benzoate (SpeedCure® DMB). Examples of polymeric aminobenzoates are SpeedCure® 7040, SpeedCure® 7045 and the compounds disclosed in WO2021074363A1.

The concentration of amine synergist in the curable composition will vary depending on the type of compound that is used. Typically, however, the curable composition is formulated to comprise from 0% to 25%, in particular 0.5% to 20%, more particularly 1 to 15%, by weight of amine synergist based on the total weight of the curable composition.

*Additives*

The curable composition of the present invention may further comprise an additive. The curable composition may comprise a mixture of additives.

In particular, the additive may be selected from stabilizers, antioxidants, light blockers, polymerization inhibitors, foam inhibitors, flow or leveling agents, colorants, pigments, dispersants (wetting agents, surfactants), slip additives, fillers, thixotropic agents, matting agents, impact modifiers, waxes and mixtures thereof; and any other additive conventionally used in the coating, sealant, adhesive, molding, 3D printing or ink arts.

According to some embodiments, the curable composition may comprise a stabilizer.

Stabilizers may be introduced in the curable composition of the present invention in order to provide adequate storage stability and shelf life. Further, stabilizers may be used during the preparation of the curable composition, to protect against unwanted reactions during processing of the ethylenically unsaturated components of the curable composition. A stabilizer may be a compound or substance which retards or prevents reaction or curing of actinically-polymerizable functional groups present in a composition in the absence of actinic radiation. However, it will be advantageous to select an amount and type of stabilizer such that the composition remains capable of being cured when exposed to actinic radiation (that is, the stabilizer does not prevent radiation curing of the composition). The stabilizer may, in particular be a free radical

stabilizer (i.e. a stabilizer which functions by inhibiting free radical reactions).

Any of the stabilizers known in the art related to (meth)acrylate-functionalized compounds may be utilized in the present invention. Quinones represent a particularly preferred type of stabilizer which can be employed in the context of the present invention. As used herein, the term *"quinone"* includes both quinones and hydroquinones as well as ethers thereof such as monoalkyl, monoaryl, monoaralkyl and bis(hydroxyalkyl) ethers of hydroquinones. Hydroquinone monomethyl ether is an example of a suitable stabilizer which can be utilized. Other stabilizers known in the art such as hydroquinone (HQ), 4-methoxyphenol (MEHQ), 4-tert-butylcatechol (TBC), 3,5-di-tertiobutyl-4-hydroxytoluene (BHT), phenothiazine (PTZ), pyrogallol, phosphite compounds, triphenyl antimony and tin(II) salts.

The concentration of stabilizer in the curable composition will vary depending upon the particular stabilizer or combination of stabilizers selected for use and also on the degree of stabilization desired and the susceptibility of components in the curable compositions towards degradation in the absence of stabilizer. Typically, however, the curable composition is formulated to comprise from 5 to 5000 ppm stabilizer. According to certain embodiments of the invention, the reaction mixture during each stage of the method employed to make the curable composition contains at least some stabilizer, e.g., at least 10 ppm stabilizer.

The curable composition may comprise a colorant. A colorant may be a dye, a pigment and mixtures thereof. The term "dye", as used herein means a colorant having a solubility of 10 mg/L or more in the medium in which it is introduced at 25°C. The term "pigment" is defined in DIN 55943, as a colorant that is practically insoluble in the application medium under the pertaining ambient conditions, hence having a solubility of less than 10 mg/L therein at 25°C. The term "C.I." is used as an abbreviation for Colour Index.

The colorant may be a pigment. Organic and/or inorganic pigments may be used. If the colorant is not a self-dispersible pigment, the inkjet inks preferably also contain a dispersant, more preferably a polymeric dispersant. The pigment may be black, cyan, magenta, yellow, red, orange, violet, blue, green, brown and mixtures thereof. Pigments may be chosen from those disclosed by HERBST, Willy, et al. Industrial Organic Pigments, Production, Properties, Applications. 3rd edition. Wiley - VCH , 2004. ISBN 3527305769.

Particular pigments include:

- Carbon black;
- C.I. Pigment White 1, 3, 4, 5, 6, 7, 10, 11, 12, 14, 17, 18, 19, 21, 24, 25, 27, 28 and 32;
- C.I. Pigment Yellow 1, 3, 10, 12, 13, 14, 17, 55, 65, 73, 74, 75, 83, 93, 97, 109, 111, 120, 128, 138, 139, 150, 151, 154, 155, 180, 185 and 213;
- C.I. Pigment Red 17, 22, 23, 41, 48:1 , 48:2, 49:1, 49:2, 52:1,57:1,81 :1, 81 :3, 88, 112, 122, 144, 146, 149, 169, 170, 175, 176, 184, 185, 188, 202, 206, 207, 210, 216, 221, 248, 251, 254, 255, 264, 270 and 272;
- C.I. Pigment Violet 1, 2, 19, 23, 32, 37 and 3;
- C.I. Pigment Blue 15:1, 15:2, 15:3, 15:4, 15:6, 16, 56, 61 and (bridged) aluminum phthalocyanine pigments;
- C.I. Pigment Orange 5, 13, 16, 34, 40, 43, 59, 66, 67, 69, 71 and 73;
- C.I. Pigment Green 7 and 36;
- C.I. Pigment Brown 6 and 7;

and mixtures thereof.

The curable composition of the invention may comprise a dispersant. The dispersant may be used to disperse an insoluble material such as a pigment or filler in the curable composition.

The dispersant may be a polymeric dispersant, a surfactant and mixtures thereof.

Typical polymeric dispersants are copolymers of two, three, four, five or even more monomers. The properties of polymeric dispersants depend on both the nature of the monomers and their distribution in the polymer. Copolymeric dispersants preferably have the following polymer compositions:

- random copolymer (e.g. ABBAABAB);
- alternating copolymer (e.g. ABABABAB);
- gradient copolymer (e.g. AAABAABBABBB) ;
- block copolymers (e.g. AAAAABBBBBB);
- graft copolymers (polymeric backbone with polymeric side chains attached to the backbone);

and mixed forms of these copolymers.

The polymeric dispersant may have a number average molecular weight Mn between 500 and 30,000 g/mol, more preferably between 1,500 and 10,000 g/mol.

Commercial examples of polymeric dispersants include:

- DISPERBYK® dispersants available from BYK CHEMIE GMBH;

- SOLSPERSE® dispersants available from LUBRIZOL;
- TEGO® DISPERSE dispersants from EVONIK;
- DISPEX®, EFKA® and JONCRYL® dispersants from BASF;
- DISPONER® dispersants from ELEMENTIS.

*Solvents*

The curable composition of the invention may comprise a solvent. As used herein, the term "solvent" means a non-reactive organic solvent, i.e. a solvent comprising carbon and hydrogen atom that does not react when exposed to the actinic radiation used to cure the curable compositions described herein.

Advantageously, the curable composition of the present invention may be formulated to be solvent-free. For example, the curable composition of the present invention may contain little or no solvent, e.g., less than 10 %, or less than 5 %, or less than 1 %, or even 0 % by weight of solvent, based on the total weight of the curable composition.

According to some embodiments, the curable composition is a liquid at 25°C. In various embodiments of the invention, the curable compositions described herein are formulated to have a viscosity of less than 10,000 mPa.s, or less than 5,000 mPa.s, or less than 1,000 mPa.s, or less than 500 mPa.s, or less than 250 mPa.s, or even less than 100 mPa.s as measured at 25°C using a Brookfield viscometer, model DV-II, using a 27 spindle (with the spindle speed varying typically between 20 and 200 rpm, depending on viscosity). In advantageous embodiments of the invention, the viscosity of the curable composition is from 10 to 10,000 mPa.s, or from 10 to 5,000 mPa.s, or from 10 to 1,000 mPa.s, or from 10 to 500 mPa.s, or from 10 to 250 mPa.s, or from 10 to 100 mPa.s at 25°C.

*Formulations*

The curable compositions described herein may be compositions that are to be subjected to curing by means of free radical polymerization. In particular embodiments, the curable compositions may be photocured (i.e., cured by exposure to actinic radiation, in particular UV, near-UV, visible, infrared and/or near-infrared radiation).

The curable composition of the invention may be an ink composition, a coating composition, an adhesive composition, a sealant composition, a molding composition, a dental composition, a nail polish composition or a 3D-printing composition.

End use applications for the curable compositions include, but are not limited to, inks, coatings, adhesives, additive manufacturing resins (such as 3D printing resins), molding resins, sealants, composites, antistatic layers, electronic applications, recyclable materials, smart materials capable of detecting and responding to stimuli, packaging materials, personal care articles, nail polishes, articles for use in agriculture, water or food processing, or animal husbandry, and biomedical materials. The curable compositions of the invention thus find utility in the production of biocompatible articles. Such articles may, for example, exhibit high biocompatibility, low cytotoxicity and/or low extractables.

The composition according to the invention may, in particular, be used to obtain a cured product according to the following processes.

## Process for the preparation of a cured product

The process for the preparation of a cured product according to the invention comprises curing the curable composition of the invention. In particular, the curable composition may be cured by exposing the composition to radiation. More particularly, the curable composition may be cured by exposing the composition to UV, near-UV, visible, infrared and/or near-infrared radiation. The curable composition may advantageously be cured by exposing the composition to a LED light source.

Curing may be accelerated or facilitated by supplying energy to the curable composition, such as by heating the curable composition. Thus, the cured product may be deemed as the reaction product of the curable composition, formed by curing. A curable composition may be partially cured by exposure to actinic radiation, with further curing being achieved by heating the partially cured article. For example, a product formed from the curable composition may be heated at a temperature of from 40°C to 120°C for a period of time from 5 minutes to 12 hours.

Prior to curing, the curable composition may be applied to a substrate surface in any known conventional manner, for example, by spraying, jetting, knife coating, roller coating, casting, drum coating, dipping, and the like and combinations thereof. Indirect application using a transfer process may also be used.

The substrate on which the curable composition is applied and cured may be any kind of substrate. Suitable substrates are detailed below. When used as an adhesive, the curable composition may be placed between two substrates and then cured, the cured composition thereby bonding the substrates together to provide an adhered article. Curable compositions in accordance with the present invention may also be formed or cured in a bulk manner (e.g., the curable

composition may be cast into a suitable mold and then cured).

The substrate may be a ceramic, metallic, mineral, cellulosic, animal-based or polymeric substrate. The substrate may also be a part of a human body, such as a tooth or a nail.

The substrate may be porous or substantially non-porous. The substrates may be transparent, translucent or opaque.

Examples of ceramic substrates include alumina-based ceramics and zirconia-based ceramics.

Examples of metallic substrates include titanium, gold, silver, copper, brass, steel and bronze.

Examples of mineral substates include glass, asbestos and basalt.

Examples of cellulosic substrates include plain paper or resin coated paper (e.g. polyethylene or polypropylene coated paper). There is no real limitation on the type of paper and it includes newsprint paper, magazine paper, office paper, wallpaper but also paper of higher grammage, usually referred to as boards, such as white lined chipboard, corrugated board and packaging board. Further examples of cellulosic substrates include bamboo, cotton, flax, hemp, jute, lyocell, modal, rayon, raffia, ramie and sisal.

Examples of cellulosic substrates include wool, fur, silk and leather.

Examples of polymeric substrates include polyethylene, polypropylene, polycarbonate, polyvinyl chloride, polyethylene terephthalate, polyethylene naphthalate, polylactide, polyamide, polyimide, polyacrylonitrile, polyurethane, acrylonitrile butadiene styrene.

There is no restriction on the shape of the substrate. It can be a sheet, a film, a nonwoven or woven fiber mat or a three-dimensional object.

In particular, the substrate may be selected from a food and beverage packaging, a pharmaceutical packaging, a textile, a nail, a tooth, a medical device, a food and beverage processing equipment, a water pipe.

The cured product obtained with the process of the invention may be an ink, a coating, an adhesive, a sealant, a molded article, a dental material, a nail polish or a 3D-printed article.

## Process of 3D printing

The curable compositions as described herein may be 3D-printing compositions and may be used in a 3D printing process. Accordingly, the cured product of the invention may be a 3D-printed article obtained with a 3D printing process.

The invention thus also relates to a process of 3D printing comprising printing a 3D article with the curable composition of the invention.

Three-dimensional (3D) printing (also referred to as additive manufacturing) is a process in which a 3D article is manufactured by accretion of construction material. The 3D printed article is created by utilizing the computer-aided design (CAD) data of an object through sequential construction of two dimensional (2D) layers or slices that correspond to cross-sections of 3D objects. Such 3D printed articles may be free-standing/self-supporting and may comprise, consist essentially of, or consist of an at least partially cured composition as described herein. The three-dimensional article may also be a composite, comprising at least one component consisting essentially of or consisting of at least partially cured composition as described herein as well as at least one additional component comprised of one or more materials other than such a cured composition (for example, a metal component, a thermoplastic component, an inorganic filler and/or a fibrous reinforcement).

The 3D printed article may be obtained with a process that comprises printing a 3D article with the curable compositions as described herein. In particular, the process may comprise printing a 3D article layer by layer or continuously.

The process of 3D printing may comprise curing the curable compositions of the invention. In particular, the curable compositions may be cured by exposing the curable compositions to actinic radiation. Specifically, the actinic radiation may include UV, near-UV, visible, infrared, near-infrared and/or electron beam radiation.

A plurality of layers of a curable composition as described herein may be applied to a substrate surface; the plurality of layers may be simultaneously cured (by exposure to a single dose of radiation, for example) or each layer may be successively cured before application of an additional layer of the curable composition. In some embodiments, each layer may be successively cured before the application of an additional layer, then the plurality of layers may be simultaneously cured.

The curable compositions may be partially cured during the 3D printing process to form a green composition. As described below, the green composition may then be post-cured to form a post-cured composition. It should be understood that the cured composition may include both the green composition and the post-cured composition. Accordingly, the curable compositions may be used to construct a 3D printed article which may comprise the curable compositions, the green composition, the post-cured compositions, or a combination of these.

In particular, the process of 3D printing may comprise the steps of:

a) providing (e.g., coating) a first layer of the curable composition as described herein onto a surface;

b) curing the first layer, at least partially, to provide a cured first layer;
c) providing (e.g., coating) a second layer of the curable composition as described herein onto the cured first layer;
d) curing the second layer, at least partially, to provide a cured second layer adhered to the cured first layer; and
e) repeating steps c) and d) a desired number of times to build up the 3D printed article.

The process may further comprise step f) exposing the 3D printed article to actinic radiation, wherein the actinic radiation is of sufficient intensity and duration to cause at least 50 wt. %, at least 60 wt. %, at least 70 wt. %, at least 80 wt. %, at least 90 wt. %, at least 95 wt. %, or even at least 99 wt. % of the polymerizable components in the curable composition to cure, based on the total weight of polymerizable components in the curable composition. The entirety of, or portions of, the 3D printed article may be exposed to the actinic radiation.
Although the curing steps may be carried out by any suitable means, which will in some cases be dependent upon the components present in the curable composition, in certain embodiments of the disclosure the curing is accomplished by exposing the layer to be cured to an effective amount of radiation, in particular actinic radiation as described above.
After the 3D printed article has been printed, it may be subjected to one or more post-processing steps. The post-processing steps can be selected from one or more of the following steps: removal of any printed support structures, washing with water and/or organic solvents to remove residual resins, and post-curing using thermal treatment and/or actinic radiation either simultaneously or sequentially. The post-processing steps may be used to transform the freshly printed article into a finished, functional article ready to be used in its intended application.
In some embodiments of the one or more post-processing steps, curing may be accelerated or facilitated by supplying energy to the curable composition, such as by heating the curable composition. An actinically-curable composition may be cured by exposure to actinic radiation, with further curing being achieved by heating the partially cured article. For example, an article formed from the curable composition (e.g., a 3D printed article) may be heated at a temperature of from 40°C to 250°C for a period of time of from 5 minutes to 12 hours.
Suitable 3D printing processes include digital light printing (DLP), stereolithography (SLA), inkjet, multi-jet printing, piezoelectric printing, actinically-cured extrusion, liquid crystal display (LCD) printing, gel deposition printing, continuous liquid interface (or interphase) printing (CLIP), high viscosity printing using a carrier film, and combinations of any of these.

## Process of inkjet printing

The curable compositions as described herein may be ink compositions and may be used in an inkjet printing process. Accordingly, the cured product of the invention may be an ink obtained with an inkjet printing process.
The invention thus also relates to a process of inkjet printing comprising jetting the curable composition of the invention onto a substrate.
The substrate on which the curable composition is jetted may be any kind of substrate. Suitable substrates are as detailed above.
The curable composition may be jetted by one or more print heads ejecting small droplets in a controlled manner through nozzles onto a substrate moving relative to the print head(s).
The print head may be a piezoelectric head or a continuous type print head.
The inkjet printing process may be carried out in a single pass or with a multi-pass printing mode.
The inkjet printing process may further comprise a UV-curing step. In inkjet printing, the UV curing device may be arranged in combination with the print head of the inkjet printer, travelling therewith so that the liquid UV curable inkjet ink is exposed to curing radiation very shortly after been jetted.
In a particularly preferred embodiment, the UV curing step is performed using UV LED light sources.
For facilitating curing, the inkjet printer may include one or more oxygen depletion units. The oxygen depletion units place a blanket of nitrogen or other relatively inert gas (e.g. $CO_2$), with adjustable position and adjustable inert gas concentration, in order to reduce the oxygen concentration in the curing environment.

## Process of coating a nail

The curable compositions as described herein may be nail polish compositions and may be used in a nail coating process. Accordingly, the cured product of the invention may be a nail polish obtained with a nail coating process.
The invention thus also relates to a process of coating a nail comprising applying the curable composition of the invention on a nail and curing the composition on the nail.

## Uses

The compound of formula (1) of the invention or the photoinitiator composition of the invention may be used as a

photoinitiator or a photoinitiating system in a radiation curable composition, in particular in a UV or LED-curable composition.

As used herein, *"UV curable composition"* means a composition cured by exposure to UV light emitted by a mercury light source, in particular a mercury-vapor lamp, and *"LED-curable composition"* means curing by exposure to UV light emitted by a LED light source, in particular a LED light source having an emission band in the range from 340 to 420 nm.

The compound of formula (1) of the invention or the photoinitiator composition of the invention may be used in a photopolymerization reaction. The photopolymerization reaction may be used to cure one or more ethylenically unsaturated compounds as defined above.

The compound of formula (1) of the invention or the photoinitiator composition of the invention may be used to obtain a cured product having a reduced amount of extractables. In particular, the cured product may be an ink, an overprint varnish, a coating, an adhesive, a sealant, a molded article, a dental material, a nail-polish or a 3D-printed article.

The reduction in the amount of extractables may be assessed in comparison with a cured product obtained with a conventional photoinitiator.

The extractables may be any component that migrates from the cured product. In particular, the extractables may be a photoinitiator or a residue thereof.

Migration in inkjet inks may occur in different ways:

- Penetration Migration - through the substrate to the reverse-side of the print;
- Set-off Migration - from the printed side of a substrate to the reverse side of the substrate while stacked or stored on a roll;
- Vapor-phase migration - evaporation of volatile compounds when heated;
- Condensation Extraction - condensation of critical compounds when cooked or sterilized.

The amount of extractables may be determined quantitatively using a suitable analytical method such as liquid chromatography mass spectroscopy (LC-MS). For example, the curable composition can be applied in 12 $\mu$m thickness film on a glass substrate, and crosslinked using a UV Hg lamp. Resulting cured films are removed from the glass plate, weighed and soaked in solvent such as acetonitrile or dichloromethane. The liquid fraction is finally evaporated and the residue, corresponding to the extractables part, is weighed, allowing to determine the amount of product that is uncured (not trapped within the photocured network).

Analytical methods such as nuclear magnetic resonance (NMR), liquid chromatography mass spectroscopy (LC-MS) or gas chromatography mass spectroscopy (GC-MS) may then be used to identify the nature of the extractables and refine their corresponding content.

In particular, the cured product may have less than 5 %, less than 2 %, less than 1 %, less than 0.5 %, less than 0.25 % or less than 0.1 %, by weight of extractables based on the weight of the cured product.

The curable composition of the invention may be used to obtain an ink, an overprint varnish, a coating, an adhesive, a sealant, a molded article, a dental material or a 3D-printed article, in particular an ink.

## ASPECTS

The invention may be as described in any one of the following Aspects:

Aspect 1. A photoinitiator according to formula (1):

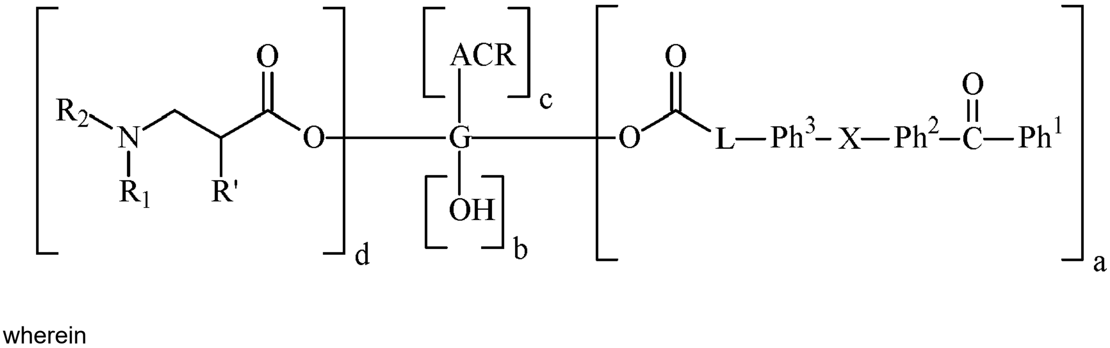

(1)

wherein

each ACR is independently a (meth)acrylate group;
each R’ is independently H or methyl;

each $R_1$ and $R_2$ is independently H or an optionally substituted group selected from alkyl, cycloalkyl and aryl; or $R_1$ and $R_2$ may, with the nitrogen atom to which they are attached, form a 5-7-membered ring; or one of $R_1$ and $R_2$ is an optionally substituted group selected from alkyl, cycloalkyl and aryl and the other of $R_1$ and $R_2$ is a moiety bearing a $-O-C(=O)-L-Ph^3-X-Ph^2-C(=O)-Ph^1$ group;
G is a (a+b+c+d)valent linker;
each L is independently a direct bond or a divalent linker;
each $Ph^1$ is independently an optionally substituted phenyl;
each $Ph^2$ and $Ph^3$ is independently an optionally substituted phenylene;
each X is independently -S-, -S(=O)-, -O- or -NR-;
each R is independently H or an optionally substituted group selected from alkyl and aryl;
a is from 1 to 7;
b is from 0 to 7;
c is from 0 to 7;
d is from 0 to 7;
a+b+c+d is from 1 to 12.

Aspect 2. The photoinitiator according to Aspect 1, wherein each $Ph^1$ is independently according to formula (2a):

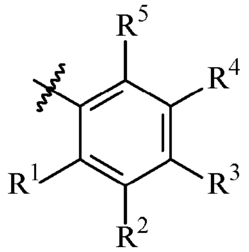

(2a)

wherein

each $R^1$, $R^2$, $R^3$, $R^4$ and $R^5$, is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, $-NO_2$, -CN, $-C(=O)OR_a$, $-OC(=O)R_b$, $-C(=O)R_c$, $-NR_dR_e$ and $-C(=O)NR_dR_e$; or any two of $R^1$ to $R^5$ may, with the carbon atoms to which they are attached, form a 5 to 6-membered ring;
each $R_a$, $R_b$ and $R_c$ is independently an optionally substituted group selected from alkyl and aryl;
each $R_d$ and $R_e$ is independently H or an optionally substituted group selected from alkyl and aryl.

Aspect 3. The photoinitiator according to Aspect 2, wherein $R^1$, $R^2$, $R^3$, $R^4$ and $R^5$ are all H.

Aspect 4. The photoinitiator according to any one of Aspects 1 to 3, wherein each $Ph^2$ is independently selected from one of the following formulae (2b) and (2c):

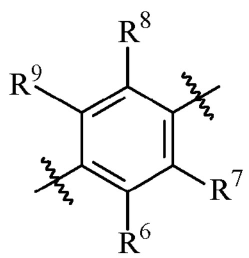

(2b)

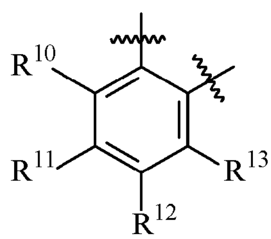

(2c)

wherein

each $R^6$, $R^7$, $R^8$, $R^9$, $R^{10}$, $R^{11}$, $R^{12}$ and $R^{13}$ is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, $-NO_2$, -CN, - C(=O)ORa, $-OC(=O)R_b$, $-C(=O)R_c$, $-NR_dR_e$ and $-C(=O)NR_dR_e$; or any two of $R^6$ to $R^9$ and $R^{10}$ to $R^{13}$ may, with the carbon atoms to which they are attached, form a 5 to 6-membered ring; or one of $R^6$,

$R^9$, $R^{10}$ and $R^{13}$ may form a 5 to 6-membered ring with $Ph^3$;
each $R_a$, $R_b$ and $R_c$ is independently an optionally substituted group selected from alkyl and aryl;
each $R_d$ and $R_e$ is independently H or an optionally substituted group selected from alkyl and aryl.

Aspect 5. The photoinitiator according to Aspect 4, wherein $R^6$, $R^7$, $R^8$, $R^9$, $R^{10}$, $R^{11}$, $R^{12}$ and $R^{13}$ are all H.

Aspect 6. The photoinitiator according to any one of Aspects 1 to 5, wherein each $Ph^3$ is independently selected from one of the following formulae (2d) and (2e):

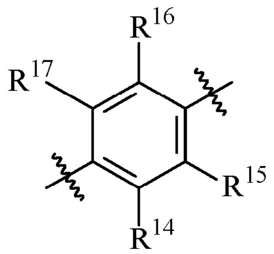
(2d)

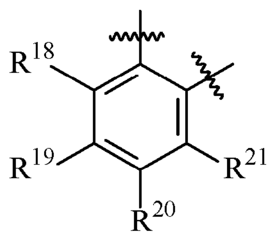
(2e)

wherein

each $R^{14}$, $R^{15}$, $R^{16}$, $R^{17}$, $R^{18}$, $R^{19}$, $R^{20}$ and $R^{21}$ is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, $-NO_2$, -CN, $-C(=O)OR_a$, $-OC(=O)R_b$, $-C(=O)R_c$, $-NR_dR_e$ and $-C(=O)NR_dR_e$; or any two of $R^{14}$ to $R^{17}$ and $R^{18}$ to $R^{21}$ may, with the carbon atoms to which they are attached, form a 5 to 6-membered ring; or one of $R^{15}$, $R^{16}$, $R^{18}$ and $R^{21}$ may form a 5 to 6-membered ring with $Ph^2$;
each $R_a$, $R_b$ and $R_c$ is independently an optionally substituted group selected from alkyl and aryl;
each $R_d$ and $R_e$ is independently H or an optionally substituted group selected from alkyl and aryl.

Aspect 7. The photoinitiator according to Aspect 6, wherein $R^{14}$, $R^{15}$, $R^{16}$, $R^{17}$, $R^{18}$, $R^{19}$, $R^{20}$ and $R^{21}$ are all H.

Aspect 8. The photoinitiator according to any one of Aspects 1 to 7, wherein each L is a direct bond or $*-C(=O)-L_1-$, wherein $L_1$ is an optionally substituted group selected from alkylene, alkenylene and arylene, any of which may be optionally interrupted with one or more of -O-, -S-, and -C(=O)-; the symbol * representing the point of attachment to $-Ph^3-$.

Aspect 9. The photoinitiator according to Aspect 8 wherein $L_1$ is according to one of the following formulae:

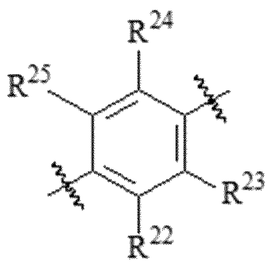

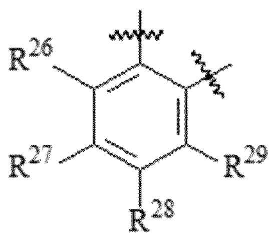

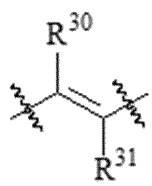

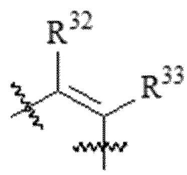

-ALK-

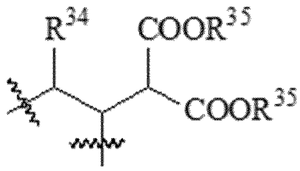 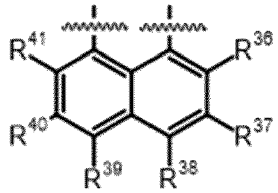 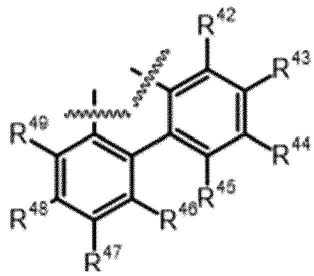

wherein

each $R^{22}$, $R^{23}$, $R^{24}$, $R^{25}$, $R^{26}$, $R^{27}$, $R^{28}$, $R^{29}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$ and $R^{49}$ is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, $-NO_2$, -CN, $-C(=O)OR_a$, $-OC(=O)R_b$, $-C(=O)R_c$, $-NR_dR_e$ and $-C(=O)NR_dR_e$; or any two of $R^{22}$ to $R^{25}$ and $R^{26}$ to $R^{29}$ and $R^{36}$ to $R^{41}$ and $R^{42}$ to $R^{49}$ may, with the carbon atoms to which they are attached, form a 5 to 6-membered ring;
each $R_a$, $R_b$ and $R_c$ is independently an optionally substituted group selected from alkyl and aryl;
each $R_d$ and $R_e$ is independently H or an optionally substituted group selected from alkyl and aryl;
each $R^{30}$, $R^{31}$, $R^{32}$, $R^{33}$ and $R^{34}$ is independently H or an optionally substituted group selected from alkyl and aryl;
ALK is an optionally substituted alkylene;
each $R^{35}$ is independently an optionally substituted group selected from alkyl and aryl.

Aspect 10. The photoinitiator according to any one of Aspects 1 to 9, wherein X is -S-.

Aspect 11. The photoinitiator according to any one of Aspects 1 to 9, wherein X is -S(=O)-.

Aspect 12. The photoinitiator according to any one of Aspects 1 to 9, wherein X is -O-.

Aspect 13. The photoinitiator according to any one of Aspects 1 to 9, wherein X is -NR- and R is preferably alkyl or aryl.

Aspect 14. The photoinitiator according to any one of Aspects 1 to 13, wherein G is selected from an aromatic linker, an aliphatic linker, a cycloaliphatic linker, a polyether linker, a polythioether linker, a polyalkylene imine linker, a polyester linker, a polycarbonate linker, a polyurethane linker, a polyorganosiloxane linker, a polybutadiene linker, and combinations thereof;
preferably G is selected from an aromatic linker, an aliphatic linker, a cycloaliphatic linker, a polyether linker, a polythioether linker, a polyalkylene imine linker, a polyester linker, a polyorganosiloxane linker, and combinations thereof.

Aspect 15. The photoinitiator according to any one of Aspects 1 to 14, wherein G is selected from:

- a divalent moiety according to any one of formulae (9) to (18):

-(CR4R'4)e- (9)

$-CH_2-O-CH_2-$ (10)

$-[(CR_5R'_5)_f-W]_g-(CR_5R'_5)_f-$ (11)

$-[(CR_6R'_6)_h-W]_i-(CR_7R'_7)_j-[W-(CR_6R'_6)_h]_i-$ (12)

$-[(CR_8R'_8)_k-C(=O)O]_l-(CR_9R'_9)_m-$ (13)

$-(CR_9R'_9)_m-[OC(=O)-(CR_8R'_8)_k]_l-$ (14)

$-[(CR_{10}R'_{10})_n-O-C(=O)-(CR_{11}R'_{11})_o-C(=O)-O]_p-(CR_{10}R'_{10})_n-$ (15)

$-(CR_{12}R'_{12})_q-C(=O)-O-(CR_{13}R'_{13})_r-O-C(=O)-(CR_{12}R'_{12})_q-$ (16)

$-(CR_{14}R'_{14})_s-Cy-[L_0-Cy]_t-(CR_{14}R'_{14})_s-$ (17)

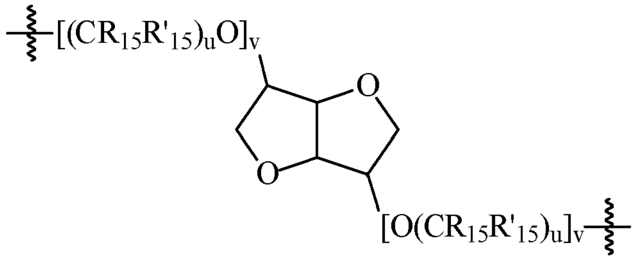

(18)

wherein:

- each $R_4$, $R'_4$, $R_7$, $R'_7$, $R_9$, $R'_9$, $R_{10}$, $R'_{10}$, $R_{11}$, $R'_{11}$, $R_{12}$, $R'_{12}$, $R_{13}$, $R'_{13}$, $R_{14}$ and $R'_{14}$ is independently H or alkyl;
- each $R_5$, $R'_5$, $R_6$, $R'_6$, $R_8$, $R'_8$, $R_{15}$ and $R'_{15}$ is independently H or methyl;
- each W is independently -O- or -S-;
- each Cy is independently an optionally substituted ring, in particular an optionally substituted cyclohexylene or phenylene;
- each $L_0$ is independently a bond or a linker such as Alk, -C(=O)-, -C(=O)-O-Alk-O-C(=O)-, -SO-, $-SO_2-$, $-C(=CCl_2)-$ and -Alk-Ph'-Alk-;
- Alk is an optionally substituted alkylene;
- Ph' is an optionally substituted phenylene;
- each e, j, m, n, o and r is independently an integer from 2 to 20;
- each s is independently an integer from 0 to 20;
- each f, h and u is independently an integer from 2 to 4;
- t is an integer equal to 0 or 1;
- each g, l, p and q is independently an integer from 1 to 20;
- each v is independently an integer from 0 to 10, in particular from 1 to 6;
- each i is independently an integer from 0 to 20 with the proviso that at least one i is not 0;
- k is an integer from 3 to 12;

- a trivalent moiety according to any one of formulae (19) to (24):

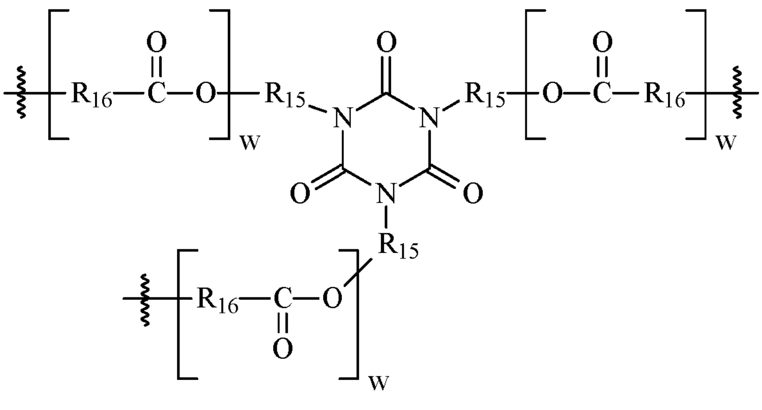

(19)

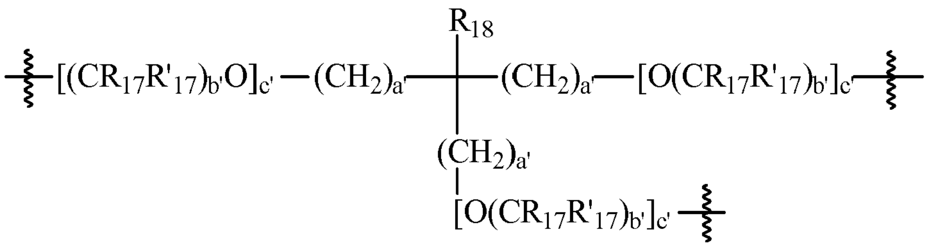

(20)

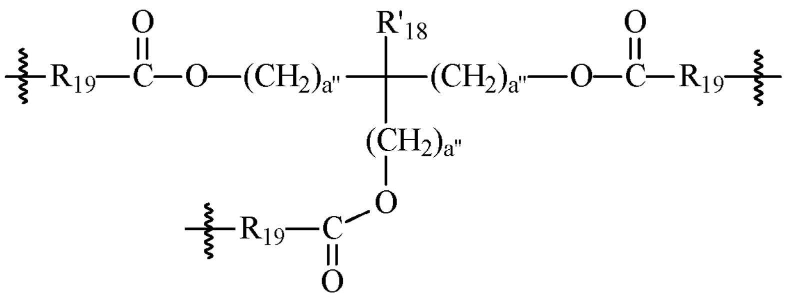

(21)

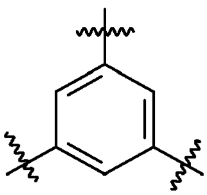

(22)

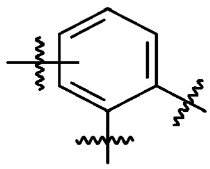

(23)

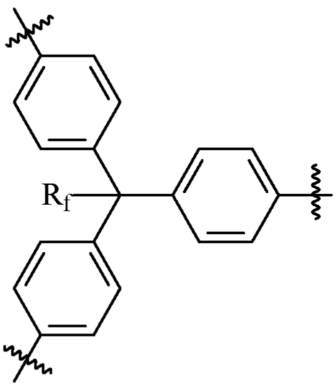

(24)

wherein:

- each $R_{15}$ and $R_{16}$ is independently a linear or branched alkylene;
- each $R_{17}$ and $R'_{17}$ is independently H or methyl;
- each $R_{18}$ and $R'_{18}$ is independently H, alkyl or alkoxy, preferably $R_{18}$ and $R'_{18}$ are alkyl;
- each $R_{19}$ is independently a linear or branched alkylene;
- $R_f$ is H or methyl;
- w is an integer equal to 0 or 1;
- each a' is independently an integer from 0 to 2 with the proviso that not more than one a' is equal to 0, preferably each a' is equal to 1 or one a' is equal to 0 and the two other a' are equal to 1;
- each a" is independently an integer from 0 to 2 with the proviso that not more than one a" is equal to 0, preferably each a" is equal to 1 or one a" is equal to 0 and the two other a" are equal to 1;
- each b' is independently an integer from 2 to 4, in particular 2;
- each c' is independently an integer from 0 to 10, in particular from 1 to 6;

- a tetravalent moiety according to any one of formulae (25) to (28):

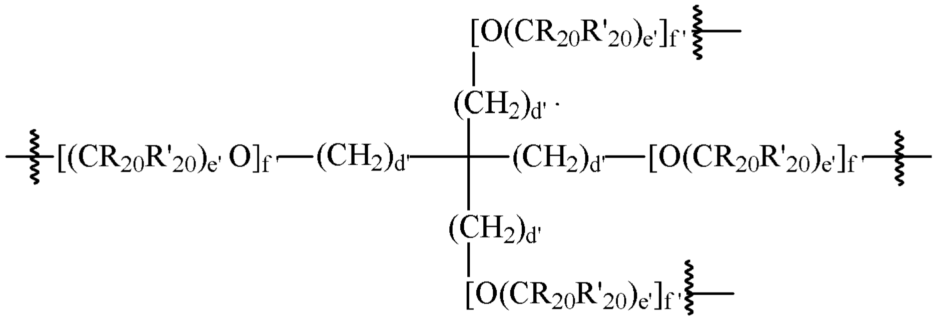

(25)

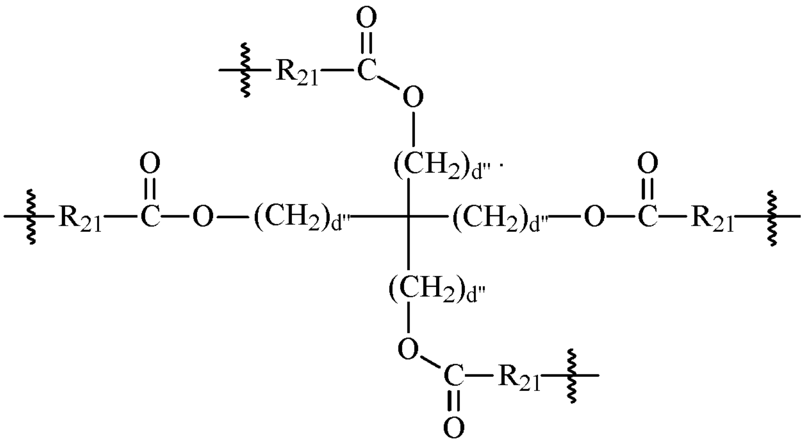

(26)

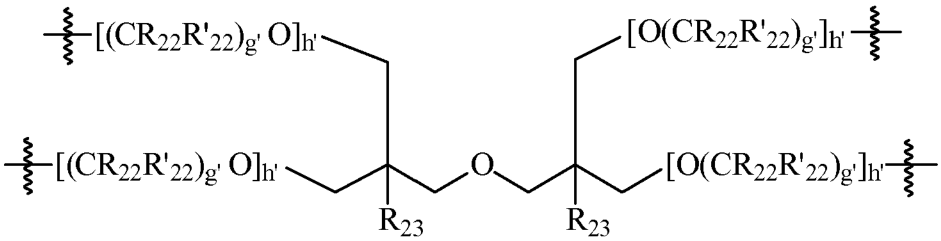

(27)

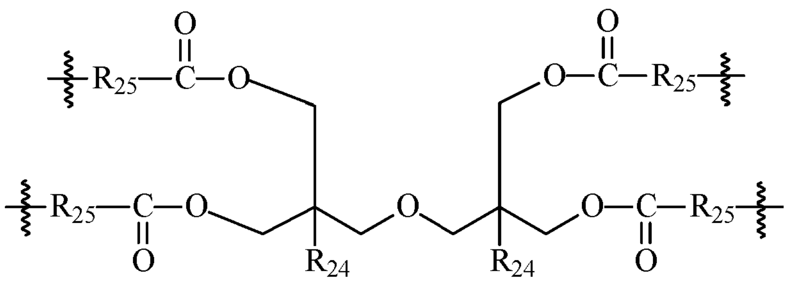

(28)

wherein:

- each $R_{20}$, $R'_{20}$, and $R'_{22}$ is independently H or methyl;
- each $R_{21}$ and $R_{25}$ is independently a linear or branched alkylene;
- each $R_{23}$ and $R_{24}$ is independently H, alkyl or alkoxy, preferably alkyl;
- each d' is independently an integer from 0 to 2 with the proviso that not more than one d' is equal to 0, preferably each d' is equal to 1;

- each d" is independently an integer from 0 to 2 with the proviso that not more than one d" is equal to 0, preferably each d" is equal to 1;
- each e' and g' is independently an integer from 2 to 4, in particular 2;
- each f' and h' is independently an integer from 0 to 10, in particular from 1 to 6;

- a tetra-, penta- or hexavalent moiety according to formula (29):

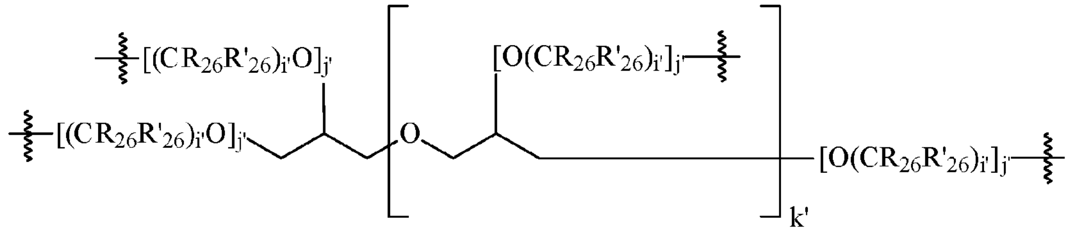

(29)

wherein

- each $R_{26}$ and $R'_{26}$ is independently H or methyl;
- each i' is independently an integer from 2 to 4, in particular 2;
- each j' is independently an integer from 0 to 10, in particular from 1 to 6;
- k' is an integer from 1 to 3;

- a tri-, tetra-, penta- or hexavalent moiety according to formula (30):

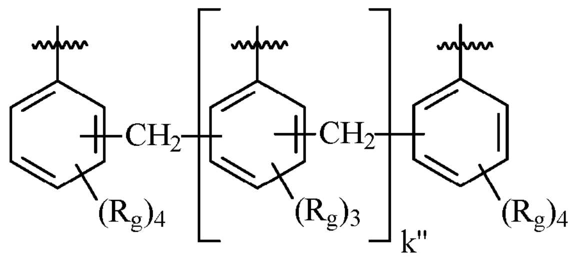

(30)

wherein

- each $R_g$ is independently selected from H, alkyl, cycloalkyl, aryl, alkaryl, aralkyl, alkoxy, -C(=O)O-Alkyl and a halogen atom;
- k" is an integer from 1 to 4.

- a hexavalent moiety according to any one of formulae (31) to (33):

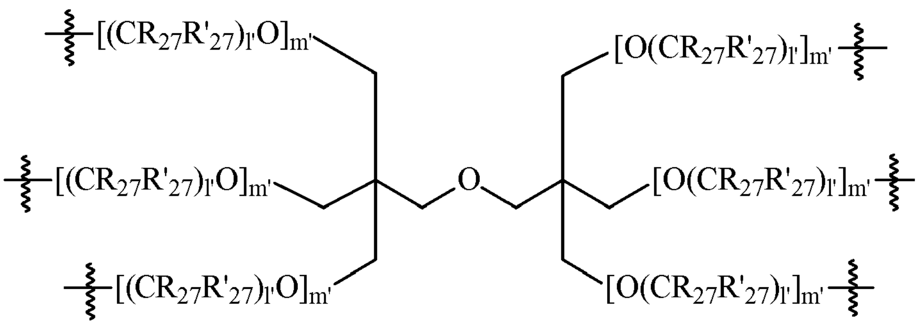

(31)

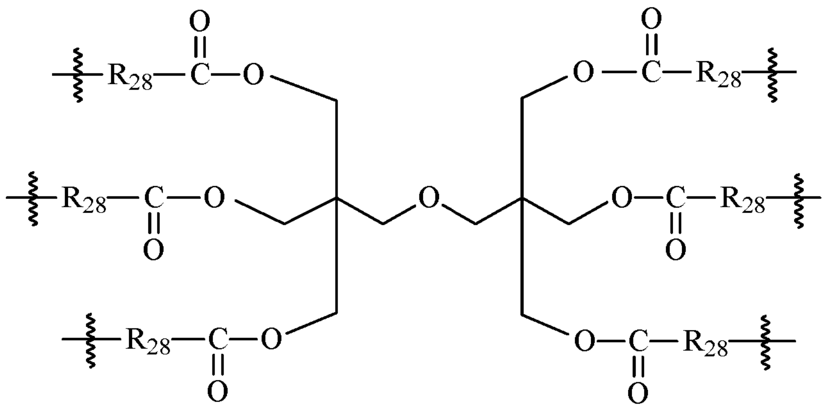

(32)

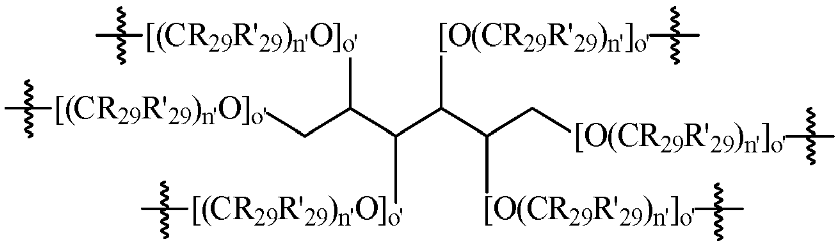

(33)

wherein

- each $R_{27}$, $R'_{27}$, $R_{29}$ and $R'_{29}$ is independently H or methyl;
- each $R_{28}$ is independently a linear or branched alkylene;
- each l' and n' is independently an integer from 2 to 4, in particular 2;
- each m' and o' is independently an integer from 0 to 10, in particular from 1 to 6.

Aspect 16. The photoinitiator according to any one of Aspects 1 to 15, wherein G corresponds to the following formula (33):

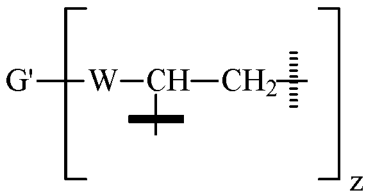

(34)

wherein

- G' is a z-valent linker;
- W is a bond, #-O-$CH_2$- or #-C(=O)-O-$CH_2$-;
- z is from 2 to 6;
- the symbol # represents a point of attachment to G';
- each bold line represents a point of attachment to a hydroxyl group, to a ACR group or to a group of formula -O-C(=O)-CH(R')-$CH_2$-$NR_1R_2$;
- each hashed line represents a point of attachment to a -O-C(=O)-L-$Ph^3$-X-$Ph^2$-C(=O)-$Ph^1$ group or to a ACR group or to a group of formula -O-C(=O)-CH(R')-$CH_2$-$NR_1R_2$, provided that at least one of the hashed lines represents a point of attachment to a -O-C(=O)-L-$Ph^3$-X-$Ph^2$-C(=O)-$Ph^1$ group.

Aspect 17. The photoinitiator according to Aspect 16, wherein G' is selected from:

- a divalent moiety according to any one of formulae (9) to (18) as defined in Aspect 16;
- a trivalent moiety according to any one of formulae (19) to (24) as defined in Aspect 16;

- a tetravalent moiety according to any one of formulae (25) to (28) as defined in Aspect 16;
- a tetra-, penta- or hexavalent moiety according to formula (29) as defined in Aspect 16;
- a tri-, tetra-, penta- or hexavalent moiety according to formula (30) as defined in Aspect 16;
- a hexavalent moiety according to any one of formulae (31) to (33) as defined in Aspect 16.

Aspect 18. The photoinitiator according to any one of Aspects 1 to 17, wherein a is an integer equal to 1, 2, 3, 4, 5 or 6, in particular a is an integer equal to 1, 2, 3 or 4.

Aspect 19. The photoinitiator according to any one of Aspects 1 to 18, wherein b is an integer equal to 0, 1 or 2, in particular b is an integer equal to 0 or 1.

Aspect 20. The photoinitiator according to any one of Aspects 1 to 19, wherein c is an integer equal to 0, 1, 2, 3, 4 or 5, in particular, c is an integer equal to 0, 1, 2 or 3.

Aspect 21. The photoinitiator according to any one of Aspects 1 to 20, wherein the photoinitiator is according to one of the following formulae:

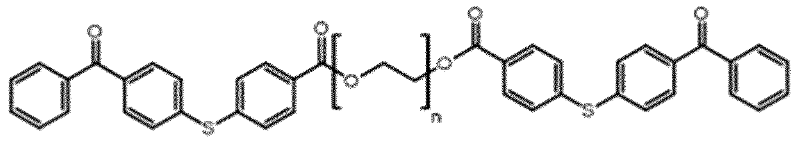

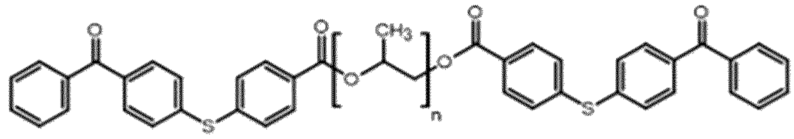

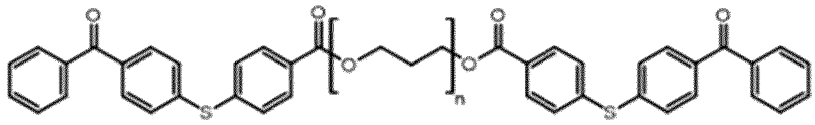

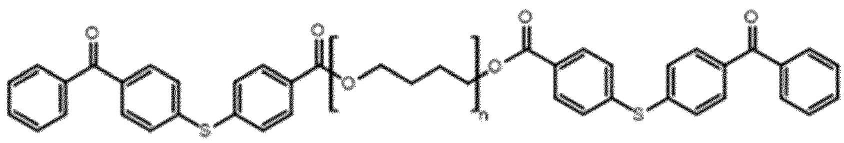

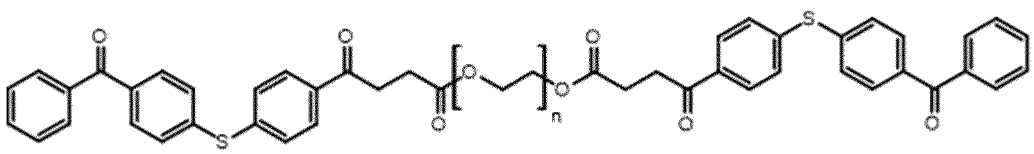

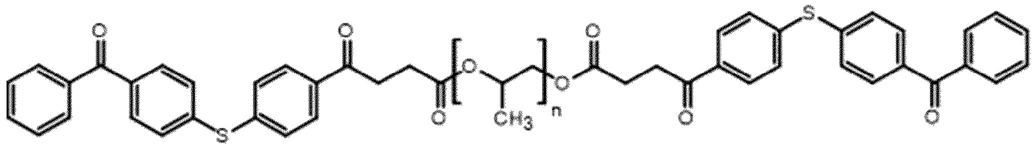

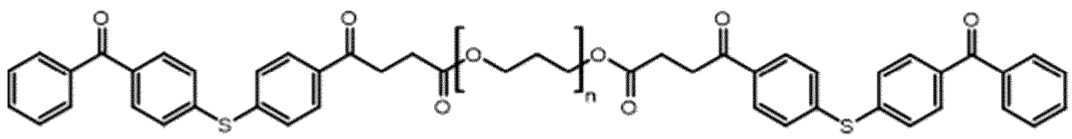

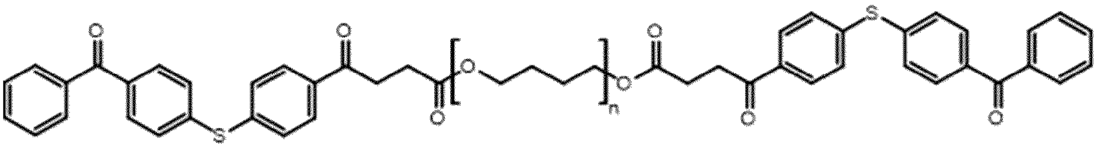

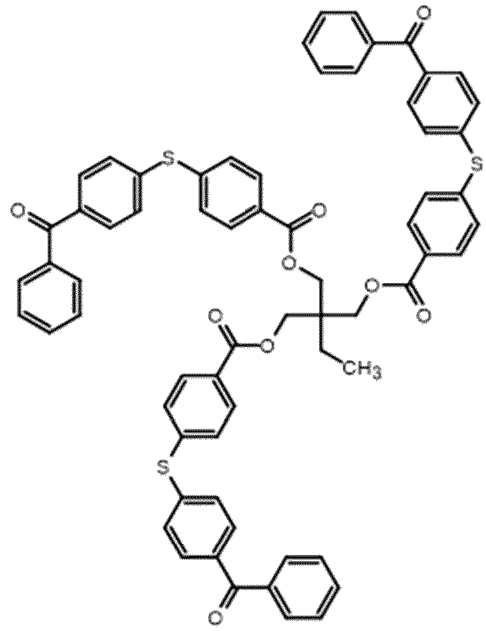

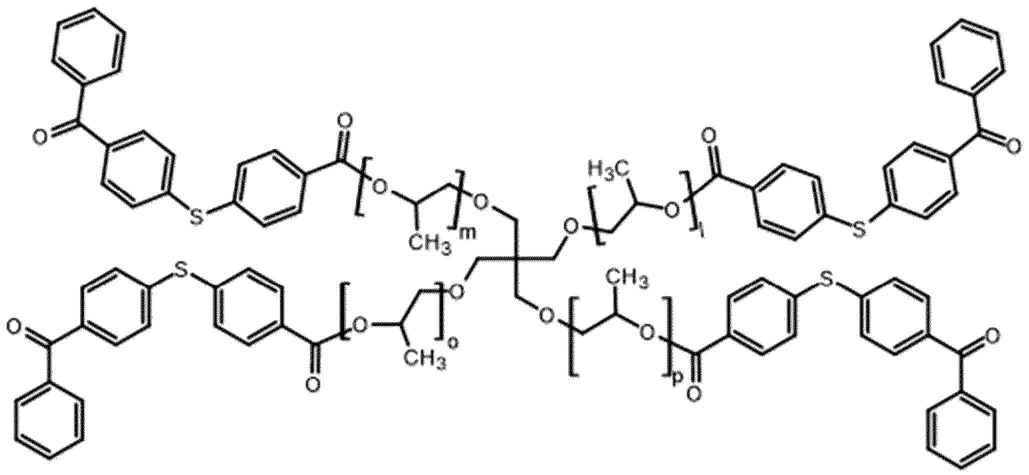

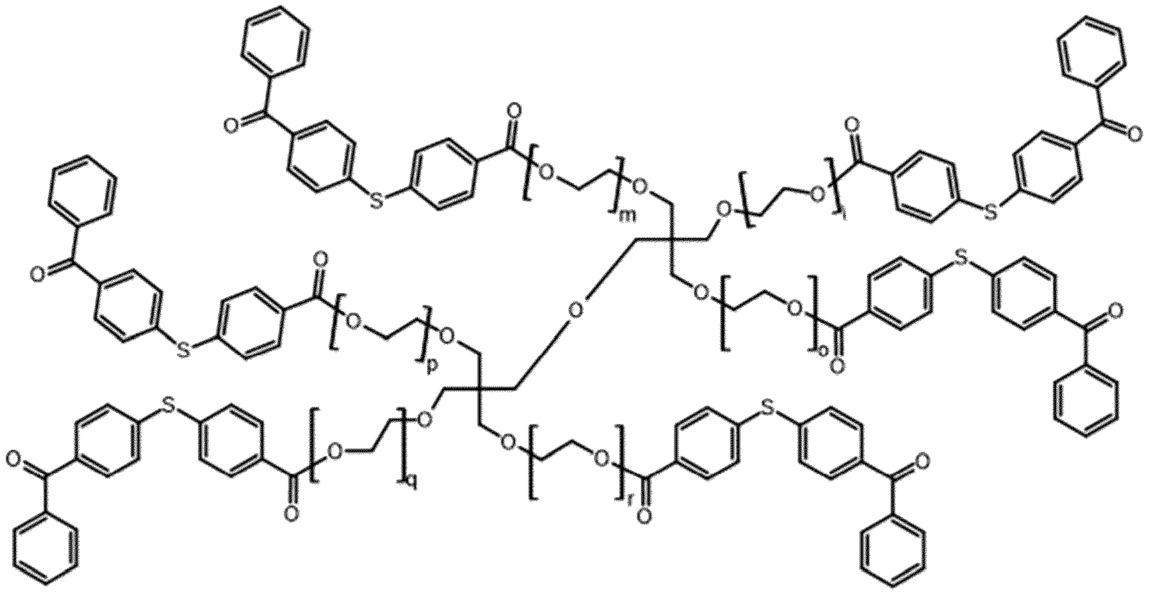

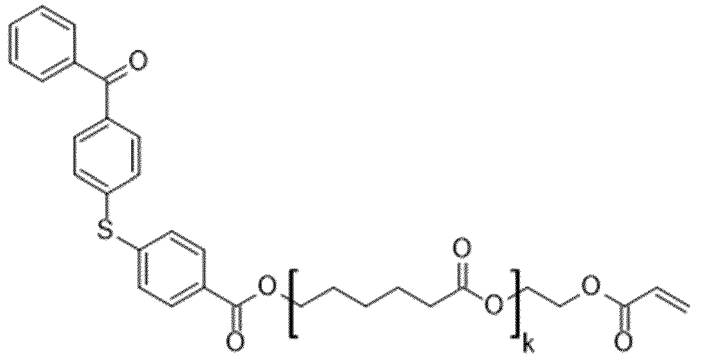

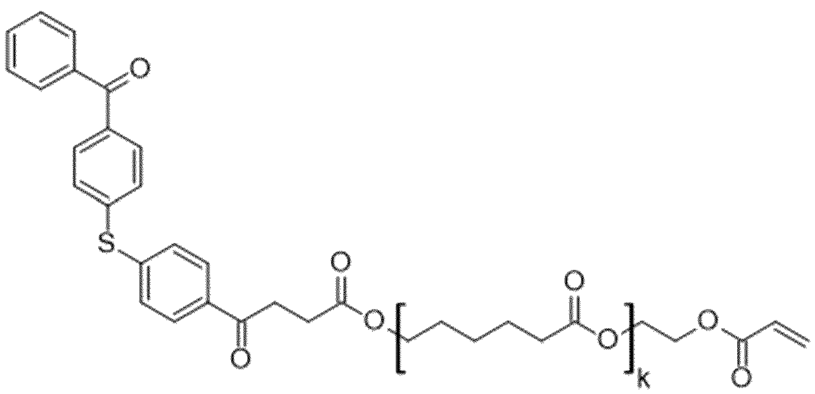

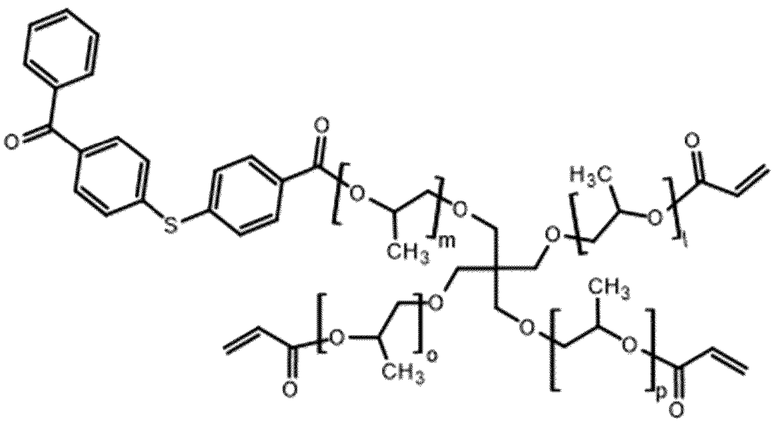

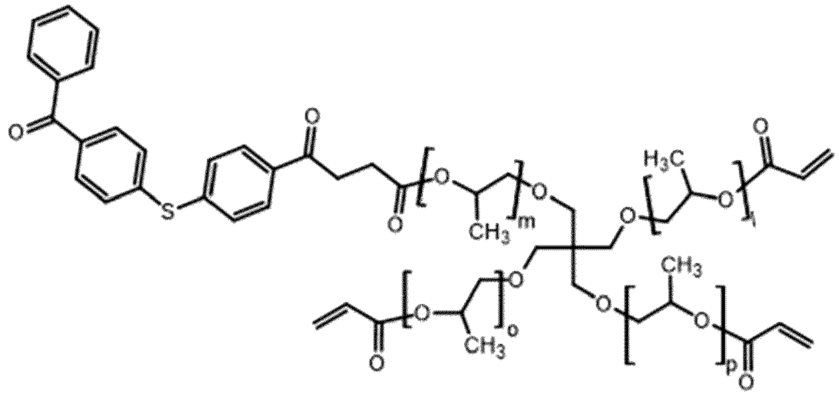

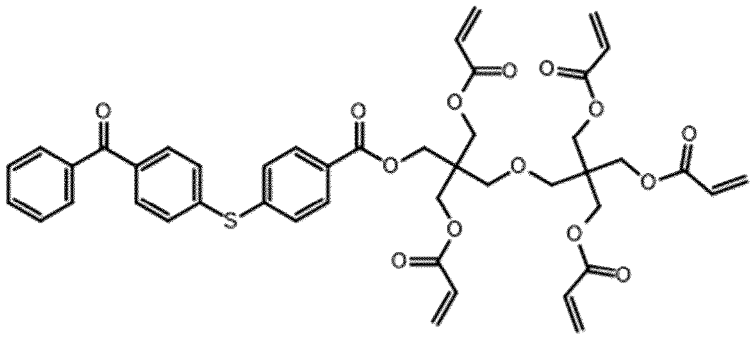

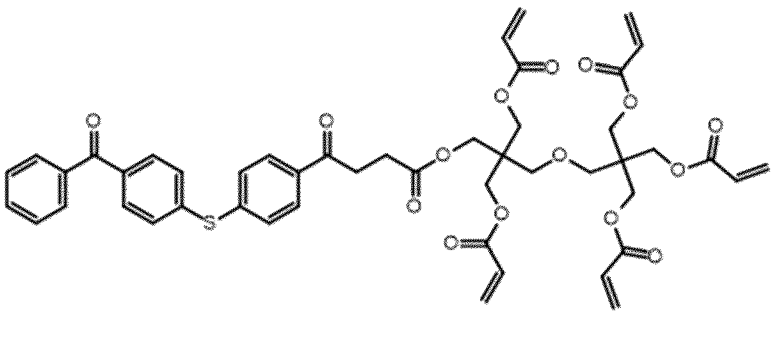

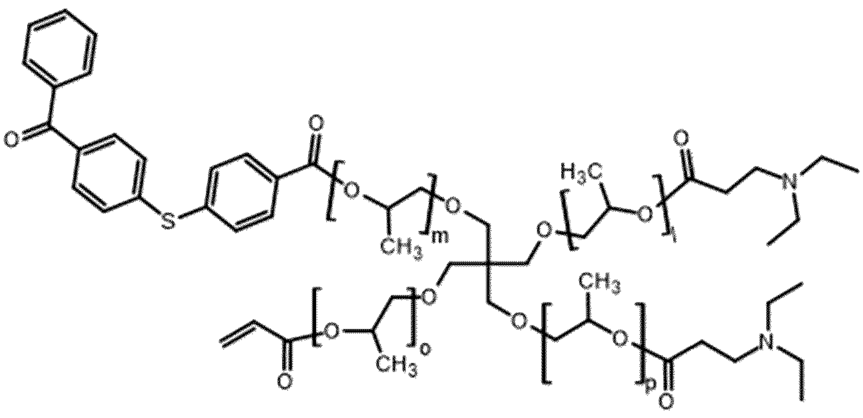

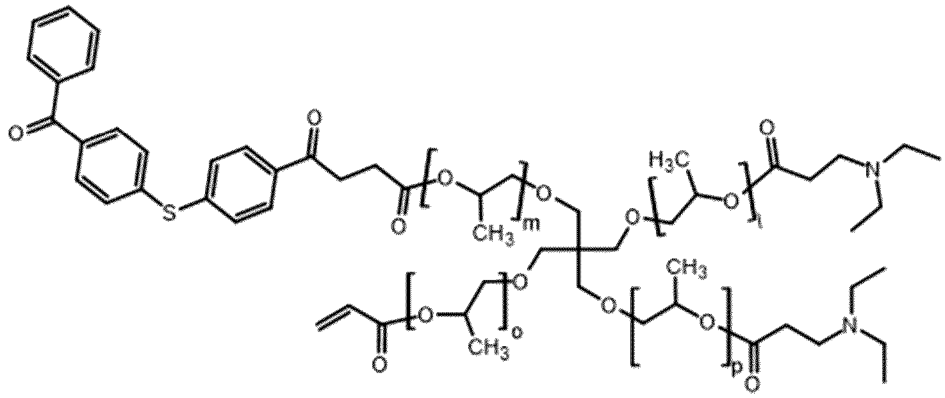

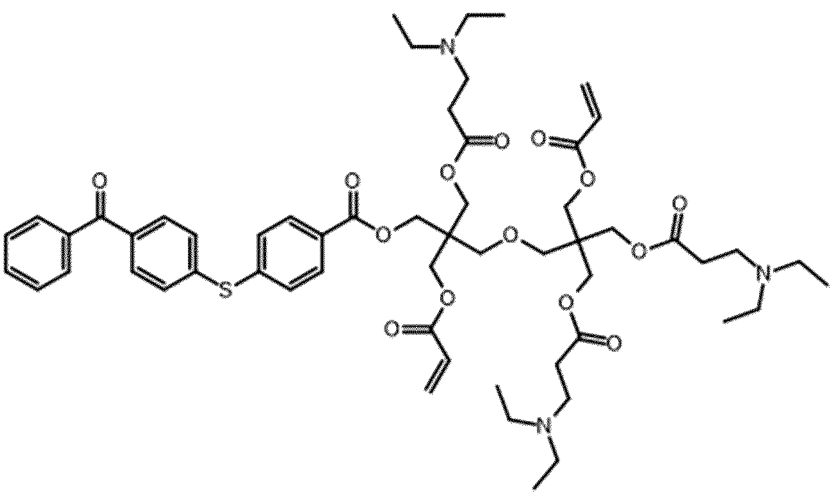

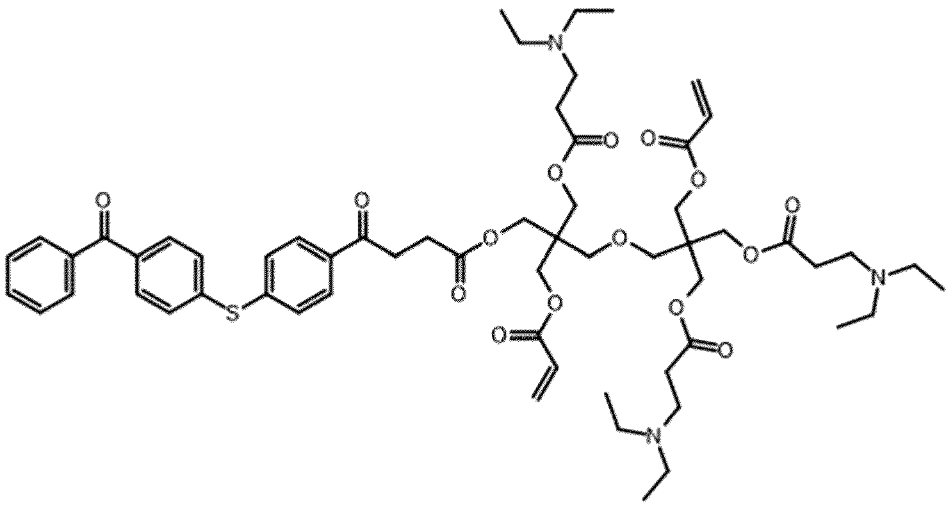

wherein

each n is independently an integer from 1 to 30;
each l, m, o, p, q, r and s is independently an integer from 0 to 30.

Aspect 22. A process for the preparation of a photoinitiator of formula (1) according to any one of Aspects 1 to 21, wherein the process comprises:

- reacting at least one precursor of formula (3) with at least one compound of formula (4) to provide a compound of formula (1a) and optionally reacting the compound of formula (1a) with at least one amine of formula (8); or
- reacting at least one precursor of formula (3) with at least one compound of formula (5a) to provide a compound of formula (1b); or
- reacting at least one precursor of formula (3) with at least one compound of formula (5) and at least one compound of formula (6) to provide a compound of formula (1a) and optionally reacting the compound of formula (1a) with at least one amine of formula (8); or
- reacting at least one precursor of formula (3) with at least one compound of formula (7) to provide a compound of formula (1c); or
- reacting at least one precursor of formula (3) with at least one compound of formula (7) and at least one compound of formula (6) to provide a compound of formula (1d) and optionally reacting the compound of formula (1d) with at least one amine of formula (8) to provide a compound of formula (1e);

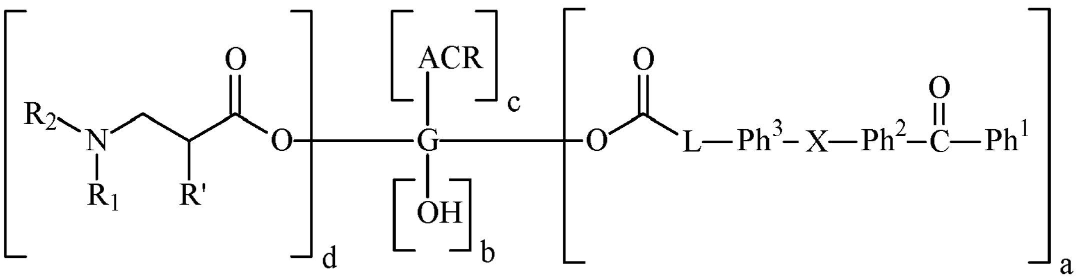
(1)

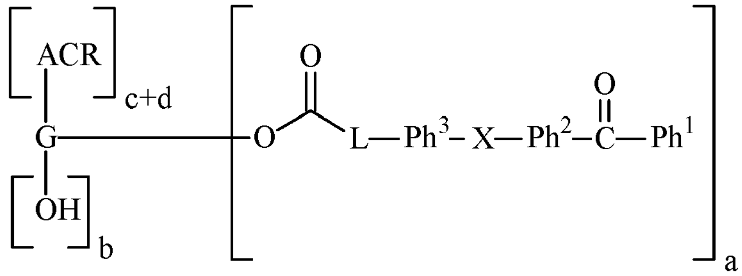
(1a)

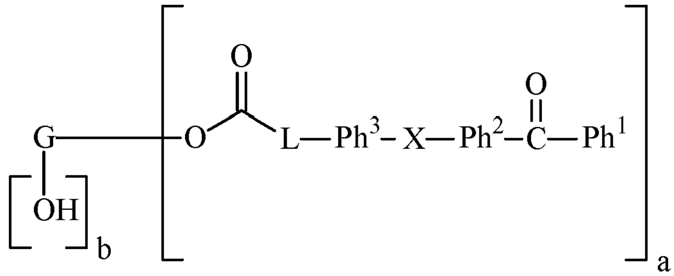
(1b)

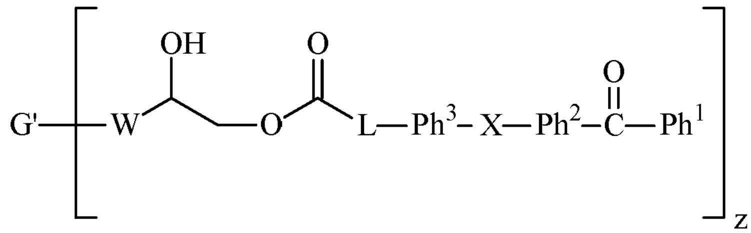
(1c)

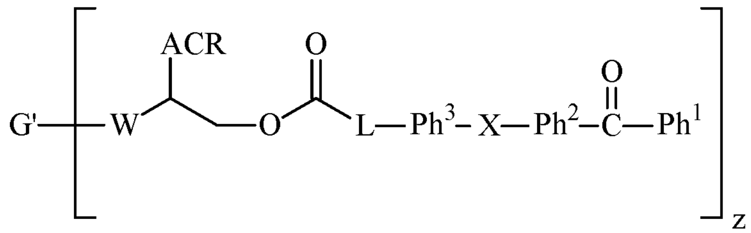
(1d)

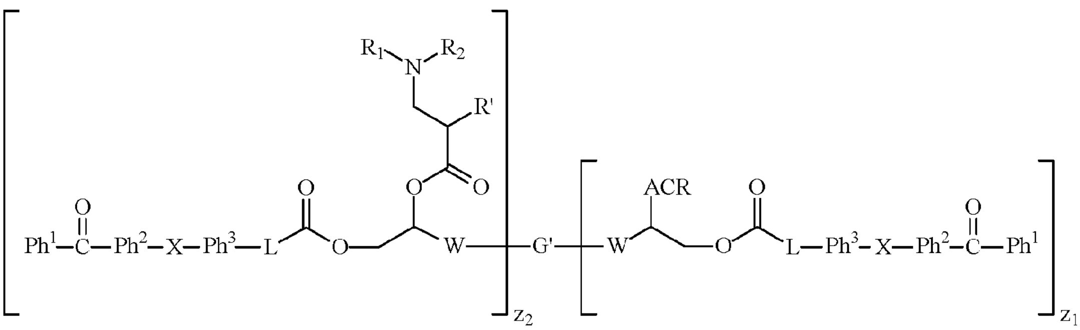
(1e)

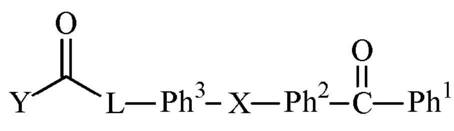
(3)

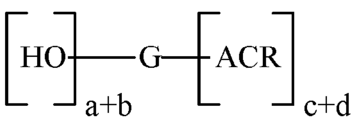
(4)

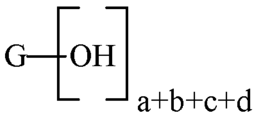 (5) 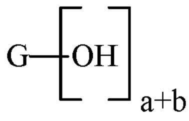 (5a) 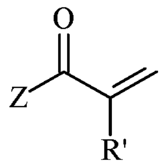 (6)

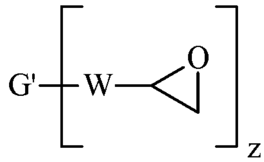 (7) 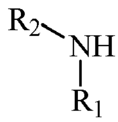 (8)

wherein:

G, G', L, ACR, $Ph^1$, $Ph^2$, $Ph^3$, R', $R_1$, $R_2$, W, X, a, b, c, d and z are as defined in any one of Aspects 1 to 20;
each Y and Z is independently OH, a halogen atom or -O-C(=O)-J;
J is alkyl or aryl, in particular tert-butyl
$Z_1 + Z_2 = Z$.

Aspect 23. A precursor according to formula (3):

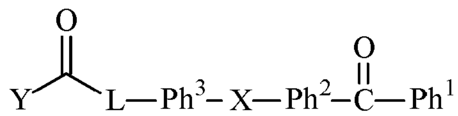 (3)

wherein:

L, $Ph^1$, $Ph^2$, $Ph^3$, X are as defined in any one of Aspects 1 to 21;
Y is OH, a halogen atom or -O-C(=O)-J;
J is alkyl or aryl, in particular tert-butyl.

Aspect 24. A precursor according to Aspect 23, wherein the precursor is according to one of the following formulae:

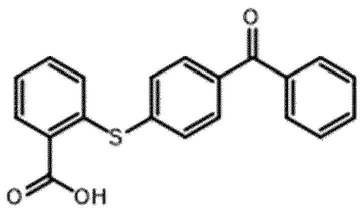 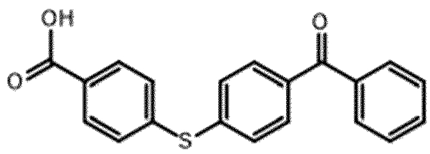

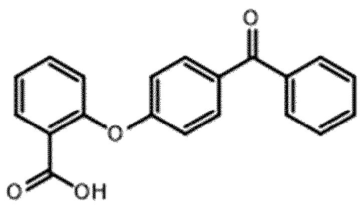 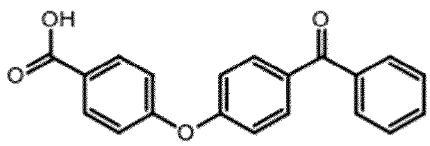

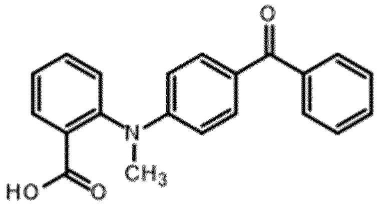

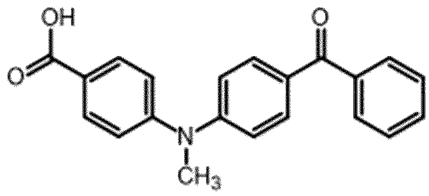

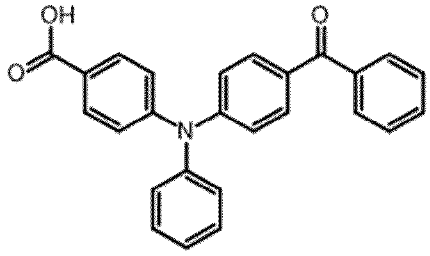

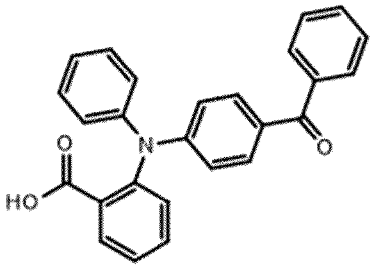

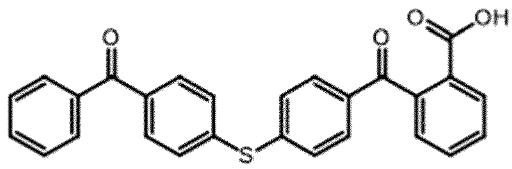

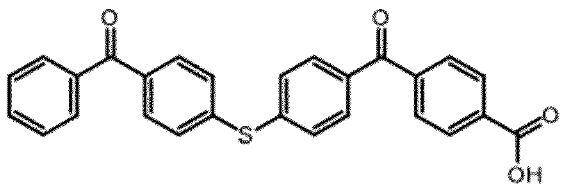

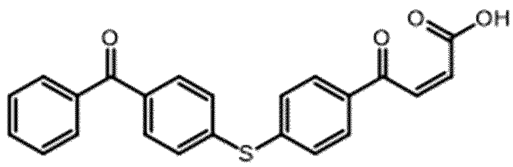

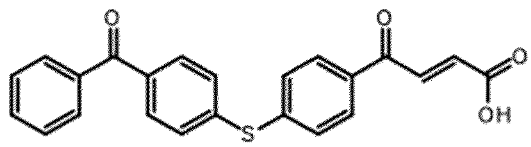

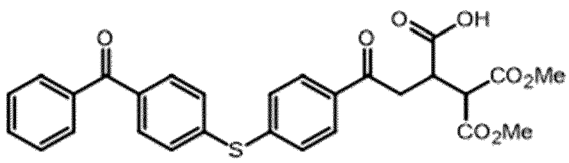

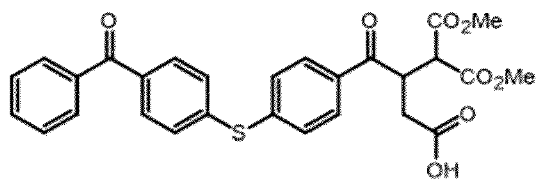

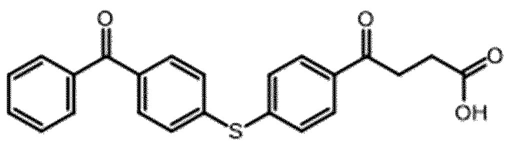

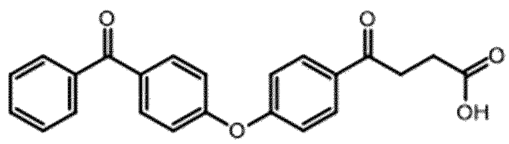

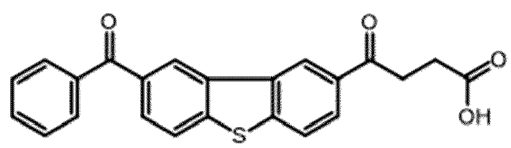

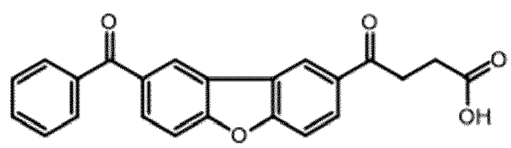

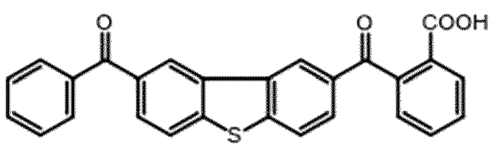

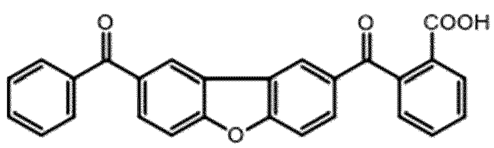

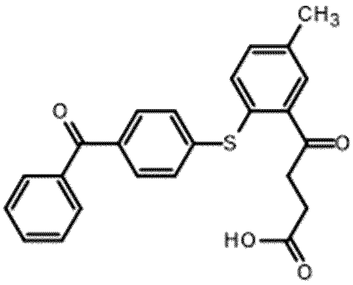
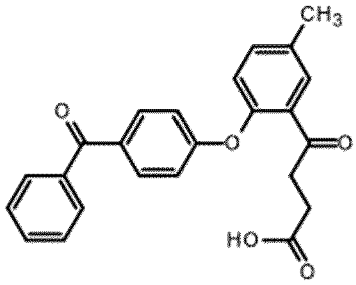
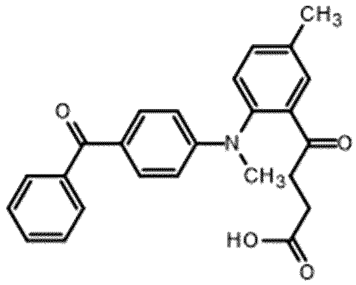

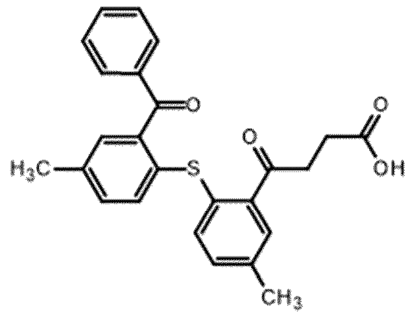
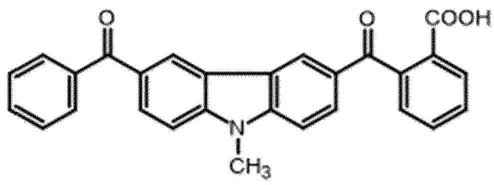

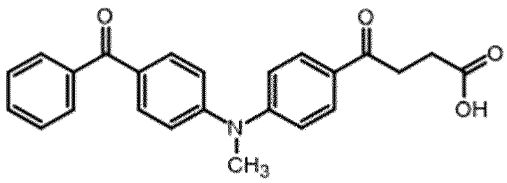
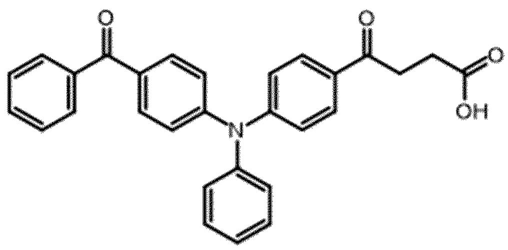

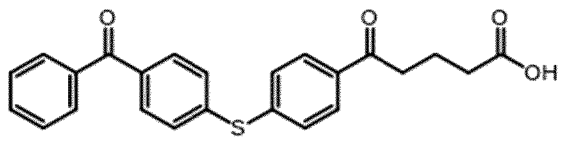
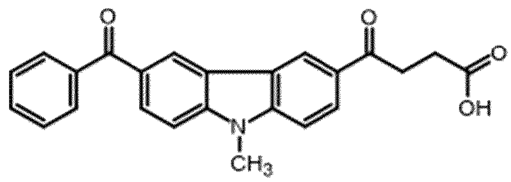

Aspect 25. A photoinitiator composition comprising a mixture of at least two photoinitiators of formula (1) according to any one of Aspects 1 to 21.

Aspect 26. A photoinitiator composition comprising a photoinitiator of formula (1) according to any one of Aspects 1 to 21, and a photoinitiator other than a photoinitiator of formula (1), in particular a photoinitiator which is a thioxanthone, more particularly a thioxanthone selected from a polymeric thioxanthone, a polymerizable thioxanthone, isopropyl thioxanthone, 2,4-diethylthioxanthone, 1-chloro-4-propoxythioxanthone and mixtures thereof.

Aspect 27. A process for photopolymerizing one or more ethylenically unsaturated compounds, wherein the process comprises contacting one or more ethylenically unsaturated compounds with a photoinitiator of formula (1) according to any one of Aspects 1 to 21 or a photoinitiator composition according to Aspect 25 or 26, and irradiating the mixture, in particular with UV, near-UV, visible, infrared and/or near-infrared radiation.

Aspect 28. A curable composition comprising:

a) a photoinitiator of formula (1) according to any one of Aspects 1 to 21 or a photoinitiator composition according to Aspect 25 or 26; and
b) an ethylenically unsaturated component.

Aspect 29. The curable composition of Aspect 28, wherein component b) comprise at least one ethylenically

unsaturated compound selected from a (meth)acrylate-functionalized monomer, an amine-modified acrylate and mixtures thereof.

Aspect 30. The curable composition of Aspect 28 or 29, wherein the composition further comprises an amine synergist, in particular an amine synergist selected from aminobenzoates, polymerizable aminobenzoates, polymeric aminobenzoates and mixtures thereof.

Aspect 31. The curable composition of any one of Aspects 28 to 30, wherein the composition further comprises an additive, in particular an additive selected from stabilizers, antioxidants, light blockers, polymerization inhibitors, foam inhibitors, flow or leveling agents, colorants, pigments, dispersants (wetting agents, surfactants), slip additives, fillers, thixotropic agents, matting agents, impact modifiers, waxes and mixtures thereof.

Aspect 32. The curable composition of any one of Aspects 28 to 31, wherein the composition is an ink composition, a coating composition, an adhesive composition, a sealant composition, a molding composition, a dental composition, a nail polish composition or a 3D-printing composition.

Aspect 33. A process for the preparation of a cured product, comprising curing the curable composition according to any one of Aspects 28 to 32, preferably by exposing the curable composition to radiation such as UV, near-UV, visible, infrared and/or near-infrared radiation, more preferably by exposing the polymerizable composition to a LED light source.

Aspect 34. A process of 3D printing comprising printing a 3D article with the curable composition according to any one of Aspects 28 to 32, in particular layer by layer or continuously.

Aspect 35. A process of inkjet printing comprising jetting the curable composition according to any one of Aspects 28 to 32 onto a substrate.

Aspect 36. A process of coating a nail, wherein the process comprises applying the curable composition according to any one of Aspects 28 to 32 on a nail and curing the composition on the nail.

Aspect 37. A use of a photoinitiator of formula (1) according to any one of Aspects 1 to 21 or a photoinitiator composition according to Aspect 25 or 26, as a photoinitiator or a photoinitiating system in a radiation curable composition, preferably in a UV or LED-curable composition.

Aspect 38. A use of a photoinitiator of formula (1) according to any one of Aspects 1 to 21 or a photoinitiator composition according to Aspect 25 or 26, to cure one or more ethylenically unsaturated compounds.

Aspect 39. A use of a photoinitiator of formula (1) according to any one of Aspects 1 to 21 or a photoinitiator composition according to Aspect 25 or 26, to obtain a cured product having a reduced amount of extractables.

Within this specification, embodiments have been described in a way which enables a clear and concise specification to be written, but it is intended and will be appreciated that embodiments may be variously combined or separated without departing from the invention. For example, it will be appreciated that all preferred features described herein are applicable to all aspects of the invention described herein.

Although the invention is illustrated and described herein with reference to specific embodiments, the invention is not intended to be limited to the details shown. Rather, various modifications may be made in the details within the scope and range of equivalents of the claims and without departing from the invention.

## EXAMPLES

The following examples illustrate the invention without limiting it.

### Comparative example 1

*Step 1: Preparation of precursor **(Comp Ia)***

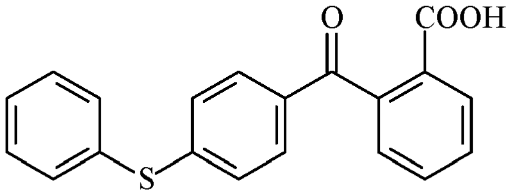

**(Comp Ia)**

Precursor **(Comp Ia**) was prepared as described in WO2023161049A1.

*Step 2: Preparation of compound **(Comp I)***

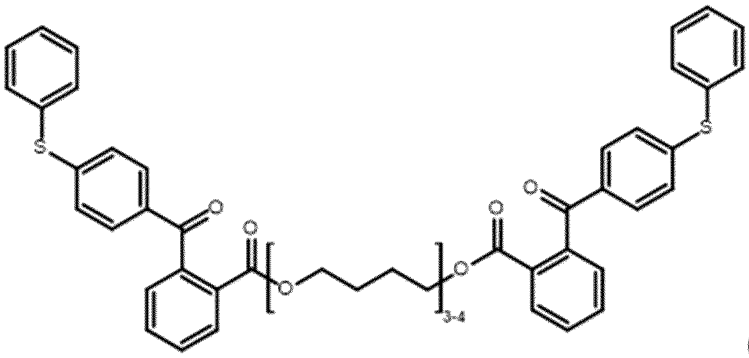

**(Comp I)**

A mixture of precursor **(Comp Ia)** (5.00 g; 14.95 mmol), poly(tetrahydrofuran) 250 (1.87g; 7.48 mmol), methanesulfonic acid (0.2 mL) and toluene (50 mL) was heated to reflux under inert atmosphere for 16 h, the water formed was collected using Dean-Stark apparatus. The reaction mixture was cooled to ambient temperature and washed with saturated sodium bicarbonate solution (50 mL), water (50 mL) and saturated brine (50 mL). The solution was treated with anhydrous magnesium sulphate and activated carbon, filtered and the solvent removed under vacuum. The crude product was then dissolved in a mixture of toluene (75 mL) and ethyl acetate (55 mL) and washed three times with a solution of potassium bicarbonate (3.3 g) in water (80 mL), then with water (100 mL). The solvents were removed under vacuum at 70°C to provide precursor **(Comp I)** as a pale orange viscous liquid. Yield 3.71 g (56%).

**Example 1**

*Step 1: Preparation of precursor **(Ia)***

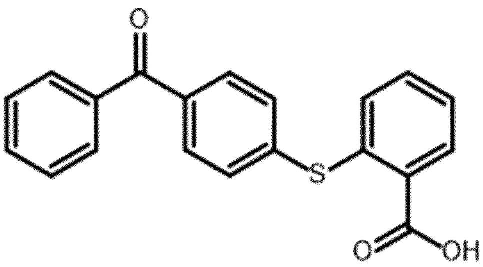

**(Ia)**

4-fluorobenzophenone (10.00 g, 49.95 mmol) and thiosalicylic acid (7.71 g; 49.95 mmol) were dissolved in N,N-dimethylacetamide (70 mL) under inert atmosphere. Potassium carbonate (20.76 g; 150.2 mmol) was added slowly with stirring. The reaction mixture was heated to 80°C for 3.5 h, then to 125°C for 6 h. Further thiosalicylic acid (0.77 g; 5.0 mmol) and potassium carbonate (2.14 g; 15.5 mmol) were added and the reaction was continued at 138°C for 5.5 h. The reaction mixture was cooled to 117°C and added slowly into a mixture of ice/water (400 g). The obtained solution was slowly acidified with 37% hydrochloric acid (40 mL) with vigorous stirring and extracted with ethyl acetate (500 mL). The organic

phase was washed with water (2*100 mL) and the solvent removed under vacuum. The obtained crude product was recrystallized from toluene/ethyl acetate (2:1, v/v) to provide precursor **(Ia)** as an off-white crystalline powder. Yield 14.55 g (87 %).

*Step 2: Preparation of compound **(I)***

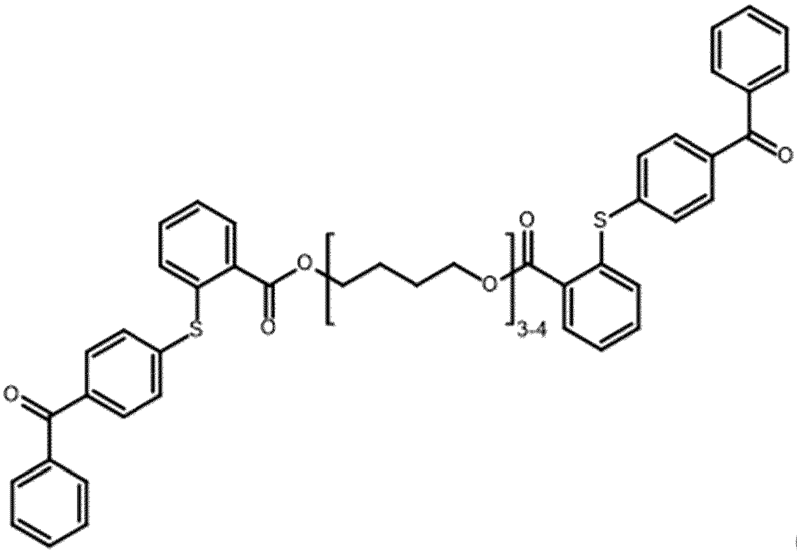

**(I)**

A mixture of precursor (**Ia**) (12.00 g; 35.89 mmol), poly(tetrahydrofuran) 250 (4.08 g; 16.31 mmol), methanesulfonic acid (0.288 g; 3.0 mmol) and toluene (75 mL) was heated to reflux under inert atmosphere for 14 h, the water formed was collected using Dean-Stark apparatus. Further methanesulfonic acid (0.900 g; 9.36 mmol) was added and the reaction was continued at reflux for a further 18 h. The reaction mixture was cooled to ambient temperature, diluted with ethyl acetate (250 mL) and washed with 2.7% solution of potassium bicarbonate (7*250 mL) and then water (3*100 mL). The solvents were removed under vacuum at 70°C to provide compound (**I**) as an orange viscous liquid. Yield 8.55 g (59 %). The product was a mixture of mono and di-substituted species.

**Example 2**

*Step 1: Preparation of precursor **(IIa)***

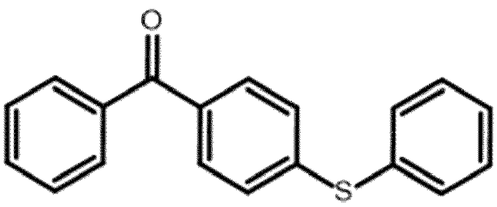

**(IIa)**

4-fluorobenzophenone (10.01 g, 50.0 mmol) and thiophenol (6.10 g; 55.4 mmol) were dissolved in N,N-dimethylacetamide (70 mL) under inert atmosphere. Potassium carbonate (11.40 g; 82.5 mmol) was added and the reaction mixture was heated to 80°C for 4 h, then to 100°C for additional 3 h. The reaction mixture was cooled to ambient temperature and added slowly into water (350 mL) with stirring. The precipitated crude product was filtered off and washed with water (2*150 mL). The product was dried in vacuum and recrystallized from n-heptane (93 mL) to provide precursor (**IIa**) as a white crystalline powder. Yield 12.13 g (84 %).

*Step 2: Preparation of precursor (**IIb**)*

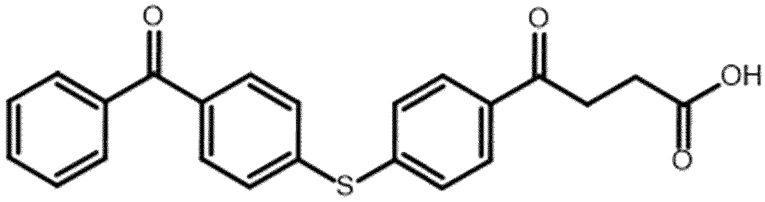

**(IIb)**

Aluminium chloride (14.3 g; 107 mmol) was suspended in dichloromethane (77 mL) under inert atmosphere. The reaction mixture was cooled to 0-5°C and precursor (**IIa**) (10.0 g; 34.4 mmol) was added followed by succinic anhydride (3.79 g; 37.9 mmol). The reaction mixture was stirred at 20-25°C for 16 h and then added into a mixture of ice/water (350 g) and 1M hydrochloric acid (50 mL). The obtained suspension was partially evaporated to remove dichloromethane and filtered. The crude solid product was washed with water (2*150 mL) and petroleum ether (100 mL) and dried in vacuum to provide precursor (**IIb**) as an off-white solid. Yield 12.89 g (96 %).

*Step 3: Preparation of compound **(II)***

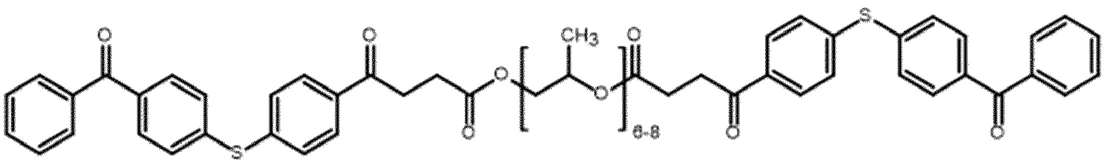

**(II)**

A mixture of precursor (**IIb**) (10.00 g; 25.61 mmol), polypropylene glycol) (average MW 425; 5.183 g; 12.20 mmol), methanesulfonic acid (0.240 g; 2.5 mmol) and toluene (75 mL) was heated to reflux under inert atmosphere for 22 h, the water formed was collected using Dean-Stark apparatus. The reaction mixture was cooled to ambient temperature, diluted with toluene (20 mL), ethyl acetate (200 mL) and treated with 1.5% solution of potassium bicarbonate in water (200 mL). The obtained mixture was filtered and allowed to phase separate, the organic phase was washed with 1% solution of potassium bicarbonate in water (150 mL) and water (100 mL). The solvents were removed under vacuum at 65°C to provide compound **(II)** as an orange viscous liquid which solidifies upon cooling. Yield 14.2 g (99 %).

**Example 3**

*Preparation of compound **(III)***

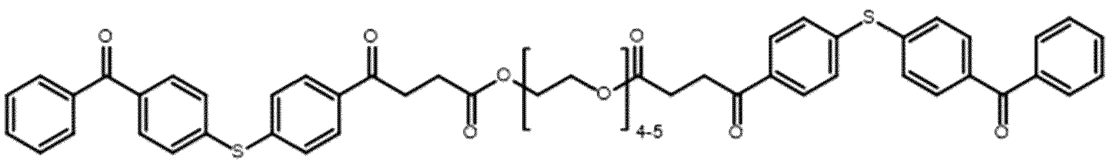

**(III)**

A mixture of precursor (**IIb**) obtained as described in Example 2 (6.00 g; 15.37 mmol), poly(ethylene glycol) (average MW 200; 1.464 g; 7.32 mmol), methanesulfonic acid (0.150 g; 1.56 mmol) and toluene (60 mL) was heated to reflux under inert atmosphere for 23 h, the water formed was collected using Dean-Stark apparatus. The reaction mixture was cooled to ambient temperature, diluted with toluene (60 mL), ethyl acetate (130 mL) and washed with 2% potassium carbonate solution in water (2*75 mL) and water (50 mL). The solvents were removed under vacuum at 65°C to provide compound **(III)** as an orange viscous liquid which solidifies upon cooling. Yield 6.77 g (98 %).

**Example 4**

*Preparation of compound **(IV)***

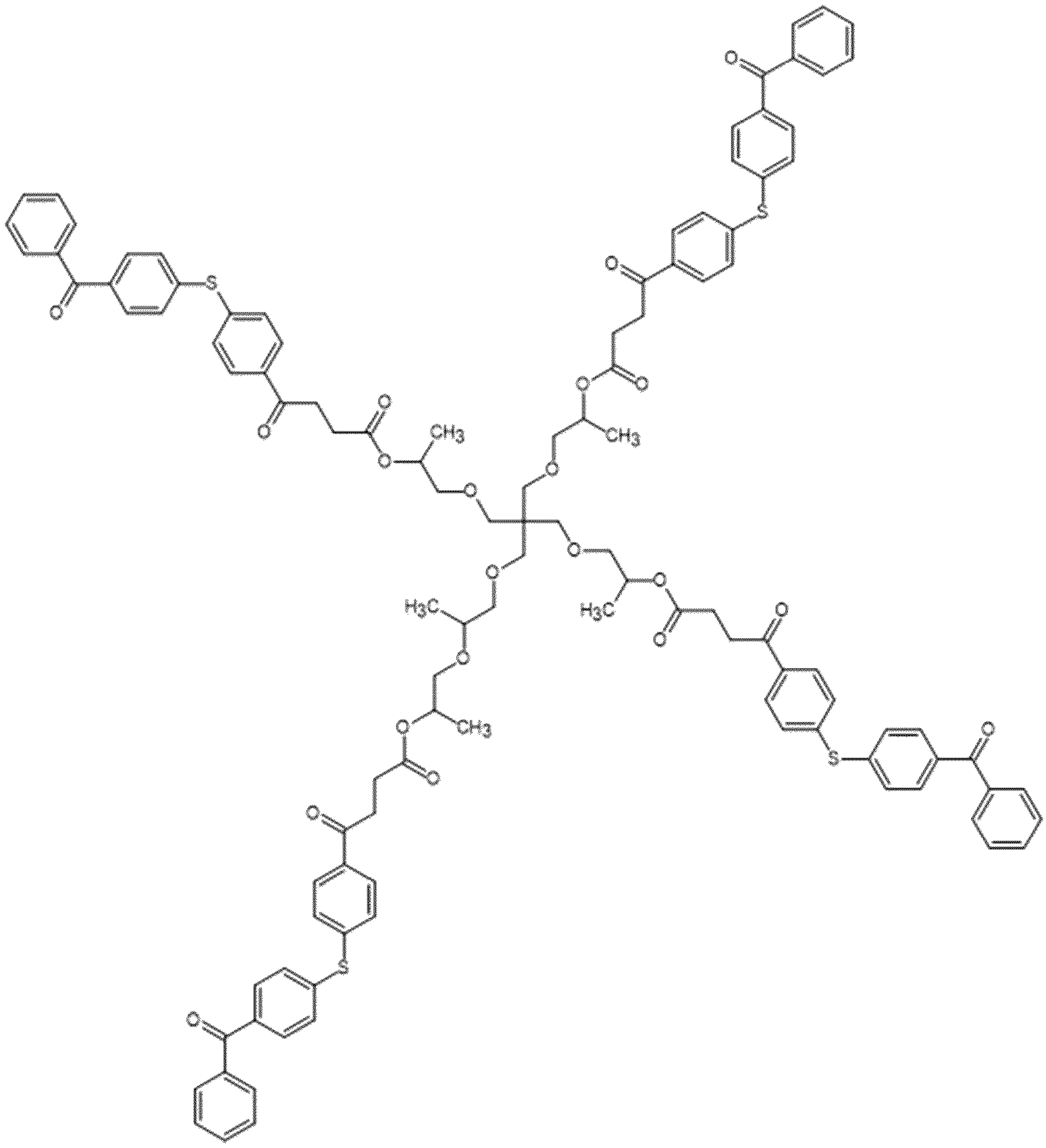

**(IV)**

A mixture of precursor (**IIb**) obtained as described in Example 2 (6.00 g; 15.37 mmol), Perstorp Polyol 4525 (average MW 430; 1.573 g; 3.66 mmol), methanesulfonic acid (0.150 g; 1.56 mmol) and toluene (64 mL) was heated to reflux under inert atmosphere for 18 h, the water formed was collected using Dean-Stark apparatus. The reaction mixture was cooled to ambient temperature, diluted with toluene (120 mL), treated with 2.4% solution of potassium bicarbonate in water (100 mL), filtered and allowed to phase separate. The organic phase was washed with 2% solution of potassium bicarbonate in water (50 mL) and water (50 mL). The solvent was removed under vacuum at 70 °C to provide product (IV) as an orange/brown semisolid. Yield 7.17 g (102 %).

**Example 5**

*Preparation of compound **(V)***

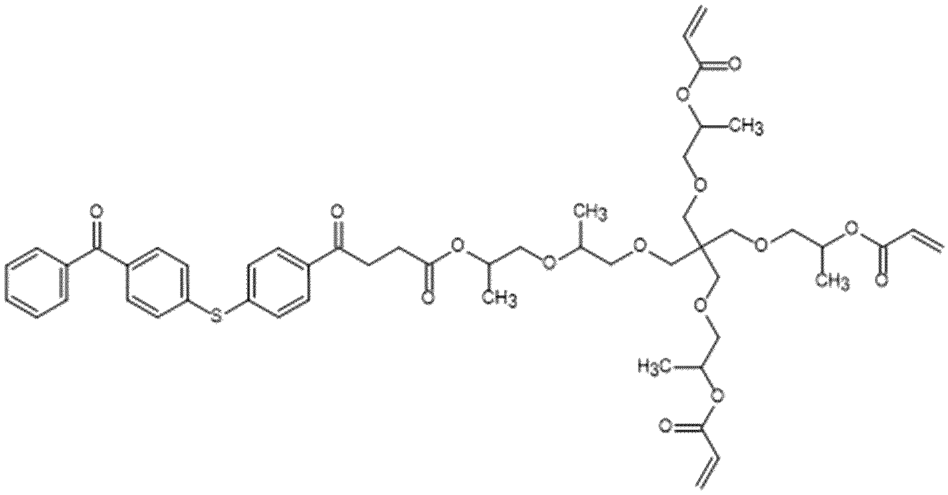

**(V)**

A mixture of precursor (**IIb**) obtained as described in Example 2 (5.46 g; 14.0 mmol), pentaerythritol propoxylate triacrylate (Sartomer SR043LM) (12.00 g; 20.38 mmol), 4-methoxyphenol (80 mg; 0.644 mmol), BHT (16 mg; 0.073 mmol), 4-hydroxy-TEMPO (3.2 mg; 0.019 mmol), methanesulfonic acid (0.150 g; 1.56 mmol) and a mixture of toluene and n-heptane (4:1, w/w) (70 mL) was heated to reflux for 17 h, the water formed was collected using Dean-Stark apparatus. The reaction mixture was cooled to ambient temperature, diluted with a mixture of toluene and n-heptane (4:1, w/w) (50 mL) and washed with 2.3% solution of potassium bicarbonate in water (100 mL), 1% solution of potassium bicarbonate in water (100 mL) and water (100 mL). After removal of solvents in vacuum, the crude product was redissolved in toluene (100 mL), treated with activated carbon at 80°C for 1h, filtered and washed with 1% solution of ammonium bicarbonate in water (2*100 mL) and water (100 mL). The solvent was removed under vacuum at 60°C to provide compound **(V)** as a yellow liquid. Yield 13.44 g (78 %).

## UV spectra

The UV spectra of Precursors **(Comp Ia),** (**Ia**) and **(Ib)** were recorded using a Shimadzu UV spectrophotometer at approx. 10 ppm concentration.

| Precursor | solvent | $\lambda_{max}$ (nm) | $E_{1cm}^{1\%}$ |
|---|---|---|---|
| Precursor **(Comp Ia)** of Comparative Example 1 | MeCN | 310 | 546 |
| Precursor (**Ia**) of Example 1 | MeCN | 253 | 689 |
| | | 319 | 341 |
| Precursor (**IIb**) of Example 2 | MeOH | 247 | 643 |
| | | 298 | 467 |
| | | 316 | 465 |

## Curing experiments

The following example illustrates the curing properties of photoinitiators disclosed in this invention.

Testing Protocol: a formulation was prepared by combining all materials listed in Table 1 in the given proportions (in % by weight based on the total weight of the formulation) and then stirring at 30-40°C until the sample was fully homogeneous; the formulation was then allowed to cool to 20°C. All components remained fully dissolved in the resin formulation throughout the experiment.

| | **Formulation 1** | | | **Formulation 2** | | | **Formulation 3** | | | **Formulation 4** | | |
|---|---|---|---|---|---|---|---|---|---|---|---|---|
| | **1A** | **1B** | **1C** | **2A** | **2B** | **2C** | **3A** | **3B** | **3C** | **4A** | **4B** | **4C** |
| SR349 | 60.9 | 60.6 | 60.2 | 60.9 | 60.6 | 60.2 | 59.4 | 59.1 | 58.7 | 59.6 | 59.2 | 58.9 |

(continued)

| | Formulation 1 | | | Formulation 2 | | | Formulation 3 | | | Formulation 4 | | |
|---|---|---|---|---|---|---|---|---|---|---|---|---|
| | 1A | 1B | 1C | 2A | 2B | 2C | 3A | 3B | 3C | 4A | 4B | 4C |
| SR306 | 26.1 | 25.9 | 25.8 | 26.1 | 25.9 | 25.8 | 25.5 | 25.3 | 25.2 | 25.5 | 25.4 | 25.2 |
| LM7401 | 8 | 8 | 8 | 8 | 8 | 8 | 8 | 8 | 8 | 8 | 8 | 8 |
| BDMB | 5 | 5 | 5 | - | - | - | - | - | - | - | - | - |
| BMS | - | - | - | 5 | 5 | 5 | - | - | - | - | - | - |
| **(Comp I)** of Comp. Example 1 | - | - | - | - | - | - | 7.1 | 7.1 | 7.1 | - | - | - |
| (**I**) of Example 1 | - | - | - | - | - | - | - | - | - | 6.9 | 6.9 | 6.9 |
| **(II)** of Example 2 | - | - | - | - | - | - | - | - | - | - | - | - |
| **(III)** of Example 3 | - | - | - | - | - | - | - | - | - | - | - | - |
| **(IV)** of Example 4 | - | - | - | - | - | - | - | - | - | - | - | - |
| DETX | - | 0.5 | 1 | - | 0.5 | 1 | - | 0.5 | 1 | - | 0.5 | 1 |

| | Formulation 5 | | | Formulation 6 | | | Formulation 7 | | |
|---|---|---|---|---|---|---|---|---|---|
| | 5A | 5B | 5C | 6A | 6B | 6C | 7A | 7B | 7C |
| SR349 | 57.7 | 57.3 | 57.0 | 58.6 | 58.2 | 57.9 | 58.6 | 58.2 | 57. 9 |
| SR306 | 24.7 | 24.6 | 24.4 | 25.1 | 25.0 | 24.8 | 25.1 | 25.0 | 24. 8 |
| LM7401 | 8 | 8 | 8 | 8 | 8 | 8 | 8 | 8 | 8 |
| BOMB | - | - | - | - | - | - | - | - | - |
| BMS | - | - | - | - | - | - | - | - | - |
| **(Comp I)** of Comp. Example 1 | - | - | - | - | - | - | - | - | - |
| (**I**) of Example 1 | - | - | - | - | - | - | - | - | - |
| **(II)** of Example 2 | 9.6 | 9.6 | 9.6 | - | - | - | - | - | - |
| **(III)** of Example 3 | - | - | - | 8.3 | 8.3 | 8.3 | - | - | - |
| **(IV)** of Example 4 | - | - | - | - | - | - | 8.3 | 8.3 | 8.3 |
| DETX | - | 0.5 | 1 | - | 0.5 | 1 | - | 0.5 | 1 |

*SR349 = Ethoxylated bisphenol A diacrylate from Arkema*
*SR306 = Tripropylene glycol diacrylate from Arkema*
*LM7401 = Acrylated amine synergist from Arkema*
*BDMB = 2-Benzyl-2-(dimethylamino)-4'-morpholinobutyrophenone from Arkema*
*BMS = 4-benzoyl-4'-methyldiphenyl sulphide from Arkema*
*DETX : 2,4-diethylthioxanthone from Arkema*

A 12 $\mu$m film was prepared on a contrast card (Leneta Form 3NT-31 Gloss Finish Coated Book Printing Ink Drawdown Sheet) with the aid of suitable K-bar coating rollers, then curing of the film was achieved a 365 nm LED lamp. The minimum passage speed necessary (in m/min) to obtain a dry film to the touch was measured.

Surface curing was checked by lightly scratching the surface of the film. Average curing speed was calculated using the belt speed divided by the passing number recorded when full cured resin was achieved. Duplicate or triplicate curing was done for the same formulation, average curing speed was used for final comparisons.

Yellowness index was determined by measuring the L* and b* values of the cured coating using a PCE colorimeter. The yellowness index (YI) was calculated from the equation: YI = 142.86 b*/L* (Francis and Clydesdale, 1975).

Pitting was assessed visually.

The surface cure results are detailed in Figure 1.

The Yellowness Index results are detailed in the Table below:

Yellowness Index:

| | Yellowness Index | | |
|---|---|---|---|
| | A (0% DETX) | B (0.5% DETX) | C (1% DETX) |
| Formulation 1 (BDMB) | 13.56 | 11.64 | 15.51 |
| Formulation 2 (BMS) | 3.65 | 5.85 | 9.54 |
| Formulation 3 **(Comp I)** | 4.21 | 5.6 | 12.15 |
| Formulation 4 (**I**) | 4.12 | 6.21 | 12.58 |
| Formulation 5 (**II**) | 4.94 | 7.12 | 12.65 |
| Formulation 6 **(III)** | 5.87 | 7.54 | 10.30 |
| Formulation 7 **(IV)** | 5.42 | 8.45 | 10.73 |

As can be seen from these results, the benzophenone compounds according to the invention are effective photoinitiators for curing of ethylenically unsaturated compounds under LED lamp conditions. Formulations comprising compounds **(I)**, **(II)**, **(III)** and **(IV)** according to the invention have enhanced curing performance compared to formulations comprising compound **(Comp I)** as disclosed in WO2023161049A1. Formulations comprising compounds **(I)**, **(II)**, **(III)** and **(IV)** according to the invention exhibit less yellowing compared to formulations comprising BDMB.

All of the compounds exhibit sensitization by thioxanthones.

## Flexographic Ink Formulations

The following example illustrates the curing properties of photoinitiators disclosed in this invention.

Testing Protocol: a formulation was prepared by combining all materials listed in Table 1 in the given proportions (in % by weight based on the total weight of the formulation) and then stirring at 30-40°C until the sample was fully homogeneous; the formulation was then allowed to cool to 20°C. All components remained fully dissolved in the resin formulation throughout the experiment.

| | **F8** | **F9** | **F10** | **F11** | **F12** | **F13** | **F14** | **F15** |
|---|---|---|---|---|---|---|---|---|
| Black Flexo Ink | 82 | 82 | 80 | 73.5 | - | - | - | - |
| Magenta Flexo Ink | - | - | - | - | 82 | 82 | 80 | 73.5 |
| LM 7401 | 8 | 8 | 8 | 8 | 8 | 8 | 8 | 8 |
| BDMB | 3 | - | - | - | 3 | - | - | - |
| BMS | - | 3 | - | - | - | 3 | - | - |
| **(III)** of Example 3 | - | - | 5 | - | - | - | 5 | - |
| **(V)** of Example 5 | - | - | - | 11.5 | - | - | - | 11.5 |
| DETX | 3 | 3 | 3 | 3 | 3 | 3 | 3 | 3 |
| 2100 | 4 | 4 | 4 | 4 | 4 | 4 | 4 | 4 |

*Black Flexo Ink = Inxflex UV Euro process black from INX.*
*Magenta Flexo Ink = Inxflex UV Euro process magenta from INX.*
*LM7401 = Acrylated amine synergist from Arkema*
*BDMB = 2-Benzyl-2-(dimethylamino)-4'-morpholinobutyrophenone from Arkema*
*BMS = 4-benzoyl-4'-methyldiphenyl sulphide from Arkema*
*DETX : 2,4-diethylthioxanthone from Arkema*
*2100 : Mixture of SpeedCure® TPO-L and SpeedCure® BPO from Arkema*

A 6 μm film was prepared on a contrast card (Leneta Form 3NT-31 Gloss Finish Coated Book Printing Ink

Drawdown Sheet) with the aid of suitable K-bar coating rollers, then curing of the film was achieved a 395 nm LED lamp. The minimum passage speed necessary (in m/min) to obtain a dry film to the touch was measured.

Surface curing was checked by lightly scratching the surface of the film. Average curing speed was calculated using the belt speed divided by the passing number recorded when full cured resin was achieved. Duplicate or triplicate curing was done for the same formulation, average curing speed was used for final comparisons.

The surface cure results are detailed in Figure 2.

When tested at same active content in flexographic inks, compounds **(III)** and (V) according to the invention performed the same as BDMB and BMS at 395nm LED.

## Claims

1. A photoinitiator according to formula (1):

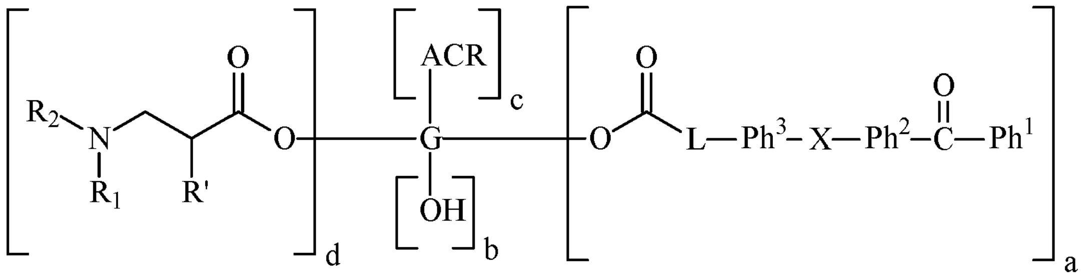
(1)

wherein
each ACR is independently a (meth)acrylate group;
each R' is independently H or methyl;
each $R_1$ and $R_2$ is independently H or an optionally substituted group selected from alkyl, cycloalkyl and aryl; or $R_1$ and $R_2$ may, with the nitrogen atom to which they are attached, form a 5-7-membered ring; or one of $R_1$ and $R_2$ is an optionally substituted group selected from alkyl, cycloalkyl and aryl and the other of $R_1$ and $R_2$ is a moiety bearing a -O-C(=O)-L-$Ph^3$-X-$Ph^2$-C(=O)-$Ph^1$ group;
G is a (a+b+c+d)valent linker;
each L is independently a direct bond or a divalent linker;
each $Ph^1$ is independently an optionally substituted phenyl;
each $Ph^2$ and $Ph^3$ is independently an optionally substituted phenylene;
each X is independently -S-, -S(=O)-, -O- or -NR-;
each R is independently H or an optionally substituted group selected from alkyl and aryl;
a is from 1 to 7;
b is from 0 to 7;
c is from 0 to 7;
d is from 0 to 7;
a+b+c+d is from 1 to 12.

2. The photoinitiator according to claim 1, wherein each L is a direct bond or *-C(=O)-$L_1$-, wherein $L_1$ is an optionally substituted group selected from alkylene, alkenylene and arylene, any of which may be optionally interrupted with one or more of -O-, -S-, and -C(=O)-; the symbol * representing the point of attachment to -$Ph^3$-.

3. The photoinitiator according to claim 1 or 2, wherein $L_1$ is according to one of the following formulae:

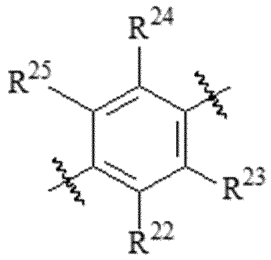 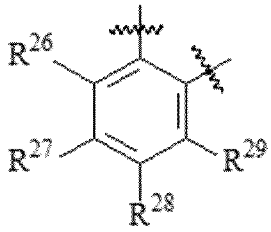 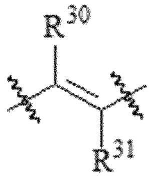 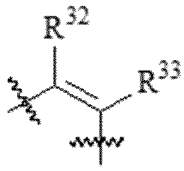

-ALK-

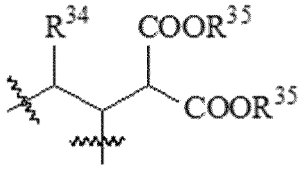 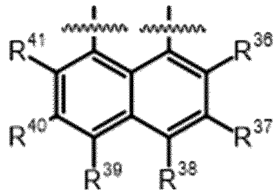 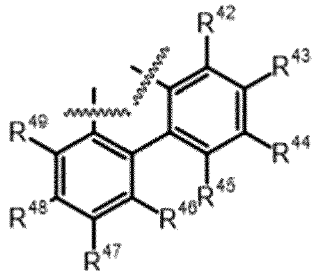

wherein

each $R^{22}$, $R^{23}$, $R^{24}$, $R^{25}$, $R^{26}$, $R^{27}$, $R^{28}$, $R^{29}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$ and $R^{49}$ is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, $-NO_2$, -CN, $-C(=O)OR_a$, $-OC(=O)R_b$, $-C(=O)R_c$, $-NR_dR_e$ and $-C(=O)NR_dR_e$; or any two of to $R^{25}$ and $R^{26}$ to $R^{29}$ and $R^{36}$ to $R^{41}$ and $R^{42}$ to $R^{49}$ may, with the carbon atoms to which they are attached, form a 5 to 6-membered ring;
each $R_a$, $R_b$ and $R_c$ is independently an optionally substituted group selected from alkyl and aryl;
each $R_d$ and $R_e$ is independently H or an optionally substituted group selected from alkyl and aryl;
each $R^{30}$, $R^{31}$, $R^{32}$, $R^{33}$ and $R^{34}$ is independently H or an optionally substituted group selected from alkyl and aryl;
ALK is an optionally substituted alkylene;
each $R^{35}$ is independently an optionally substituted group selected from alkyl and aryl.

**4.** The photoinitiator according to any one of claims 1 to 3, wherein X is -S-.

**5.** The photoinitiator according to any one of claims 1 to 4, wherein G is selected from:

- a divalent moiety according to any one of formulae (9) to (18):

-(CR4R'4)e- (9)

$-CH_2-O-CH_2-$ (10)

$-[(CR_5R'_5)_f-W]_g-(CR_5R'_5)_f-$ (11)

$-[(CR_6R'_6)_h-W]_i-(CR_7R'_7)_j-[W-(CR_6R'_6)_h]_i-$ (12)

$-[(CR_8R'_8)_k-C(=O)O]_l-(CR_9R'_9)_m-$ (13)

$-(CR_9R'_9)_m-[OC(=O)-(CR_8R'_8)_k]_l-$ (14)

$-[(CR_{10}R'_{10})_n-O-C(=O)-(CR_{11}R'_{11})_o-C(=O)-O]_p-(CR_{10}R'_{10})_n-$ (15)

$-(CR_{12}R'_{12})_q-C(=O)-O-(CR_{13}R'_{13})_r-O-C(=O)-(CR_{12}R'_{12})_q-$ (16)

$-(CR_{14}R'_{14})_s-Cy-[L_0-Cy]_t-(CR_{14}R'_{14})_s-$ (17)

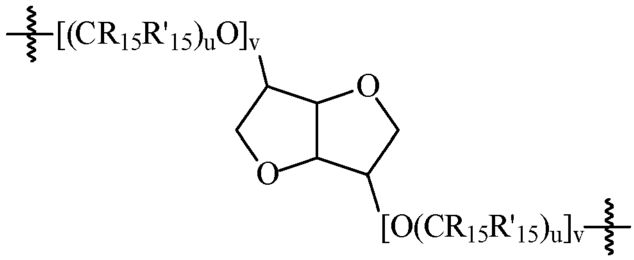

(18)

wherein:

- each $R_4$, $R'_4$, $R_7$, $R'_7$, $R_9$, $R'_9$, $R_{10}$, $R'_{10}$, $R_{11}$, $R'_{11}$, $R_{12}$, $R'_{12}$, $R_{13}$, $R'_{13}$, $R_{14}$ and $R'_{14}$ is independently H or alkyl;
- each $R_5$, $R'_5$, $R_6$, $R'_6$, $R_8$, $R'_8$, $R_{15}$ and $R'_{15}$ is independently H or methyl;
- each W is independently -O- or -S-;
- each Cy is independently an optionally substituted ring, in particular an optionally substituted cyclohexylene or phenylene;
- each $L_0$ is independently a bond or a linker such as Alk, -C(=O)-, -C(=O)-O-Alk-O-C(=O)-, -SO-, $-SO_2-$, $-C(=CCl_2)-$ and -Alk-Ph'-Alk-;
- Alk is an optionally substituted alkylene;
- Ph' is an optionally substituted phenylene;
- each e, j, m, n, o and r is independently an integer from 2 to 20;
- each s is independently an integer from 0 to 20;
- each f, h and u is independently an integer from 2 to 4;
- t is an integer equal to 0 or 1;
- each g, l, p and q is independently an integer from 1 to 20;
- each v is independently an integer from 0 to 10, in particular from 1 to 6;
- each i is independently an integer from 0 to 20 with the proviso that at least one i is not 0;
- k is an integer from 3 to 12;

- a trivalent moiety according to any one of formulae (19) to (24):

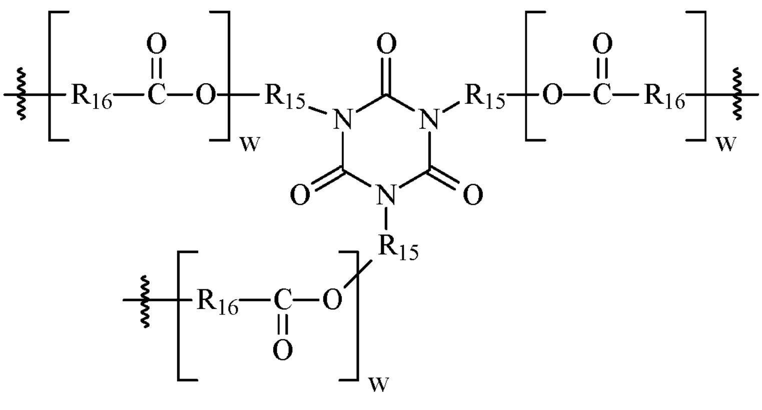

(19)

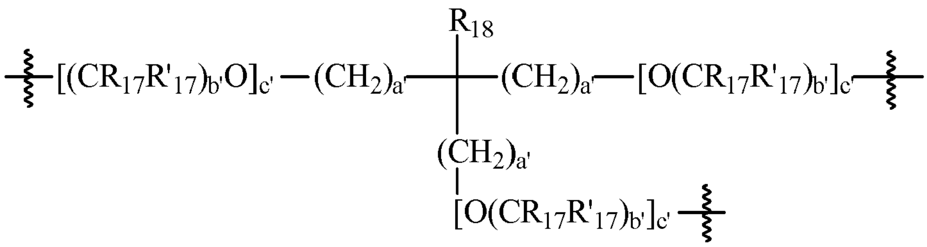

(20)

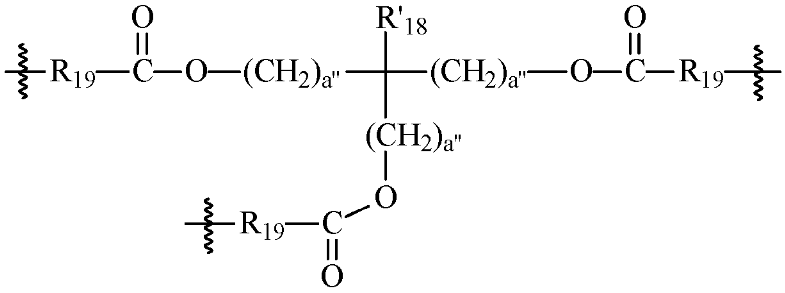

(21)

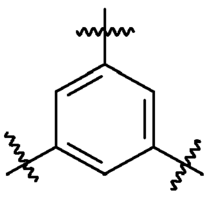

(22)

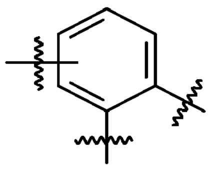

(23)

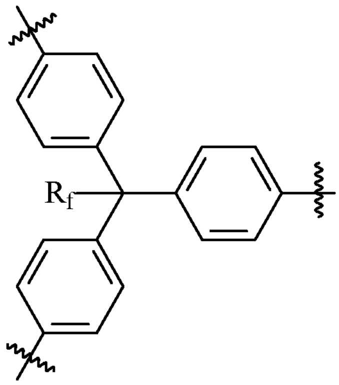

(24)

wherein:

- each $R_{15}$ and $R_{16}$ is independently a linear or branched alkylene;
- each $R_{17}$ and $R'_{17}$ is independently H or methyl;
- each $R_{18}$ and $R'_{18}$ is independently H, alkyl or alkoxy, preferably $R_{18}$ and $R'_{18}$ are alkyl;
- each $R_{19}$ is independently a linear or branched alkylene;
- $R_f$ is H or methyl;
- w is an integer equal to 0 or 1;
- each a' is independently an integer from 0 to 2 with the proviso that not more than one a' is equal to 0, preferably each a' is equal to 1 or one a' is equal to 0 and the two other a' are equal to 1;
- each a" is independently an integer from 0 to 2 with the proviso that not more than one a" is equal to 0, preferably each a" is equal to 1 or one a" is equal to 0 and the two other a" are equal to 1;
- each b' is independently an integer from 2 to 4, in particular 2;
- each c' is independently an integer from 0 to 10, in particular from 1 to 6;

- a tetravalent moiety according to any one of formulae (25) to (28):

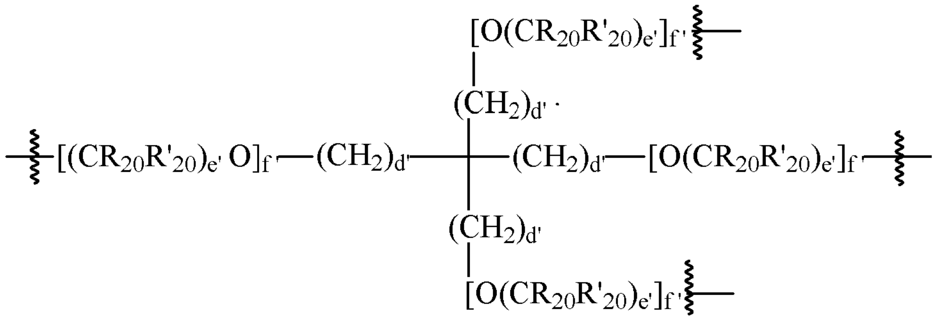

(25)

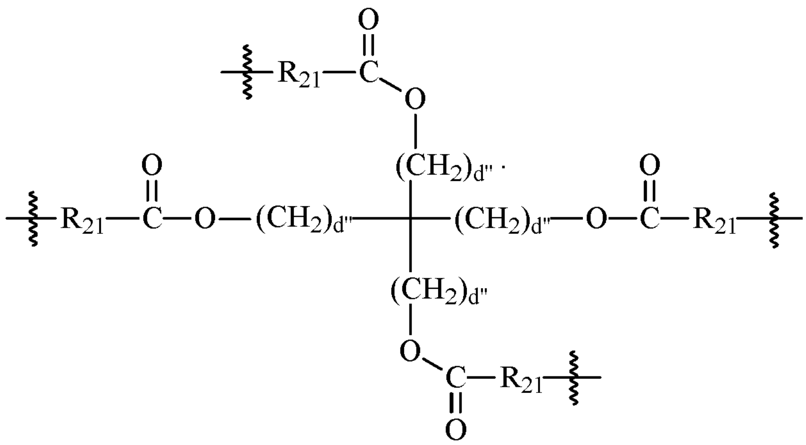

(26)

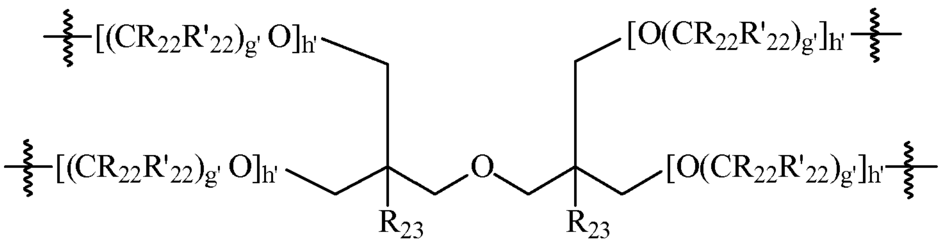

(27)

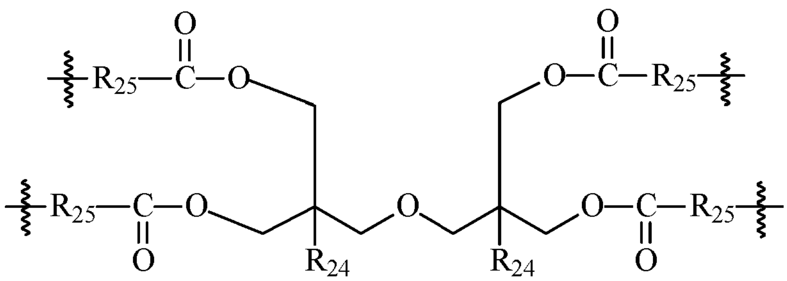

(28)

wherein:

- each $R_{20}$, $R'_{20}$, and $R'_{22}$ is independently H or methyl;
- each $R_{21}$ and $R_{25}$ is independently a linear or branched alkylene;
- each $R_{23}$ and $R_{24}$ is independently H, alkyl or alkoxy, preferably alkyl;
- each d' is independently an integer from 0 to 2 with the proviso that not more than one d' is equal to 0, preferably each d' is equal to 1;

- each d" is independently an integer from 0 to 2 with the proviso that not more than one d" is equal to 0, preferably each d" is equal to 1;
- each e' and g' is independently an integer from 2 to 4, in particular 2;
- each f' and h' is independently an integer from 0 to 10, in particular from 1 to 6;

- a tetra-, penta- or hexavalent moiety according to formula (29):

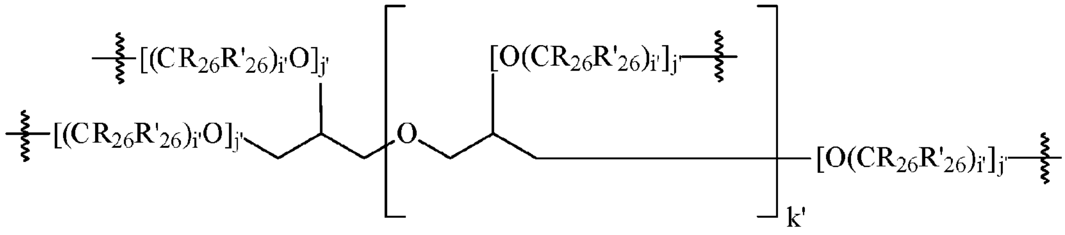

(29)

wherein

- each $R_{26}$ and $R'_{26}$ is independently H or methyl;
- each i' is independently an integer from 2 to 4, in particular 2;
- each j' is independently an integer from 0 to 10, in particular from 1 to 6;
- k' is an integer from 1 to 3;

- a tri-, tetra-, penta- or hexavalent moiety according to formula (30):

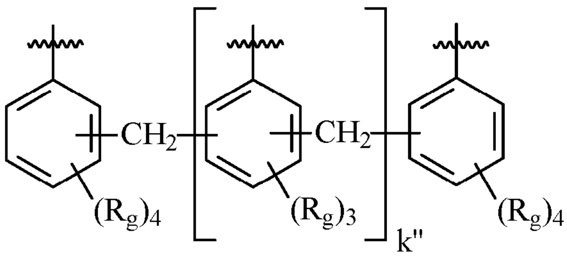

(30)

wherein

- each $R_g$ is independently selected from H, alkyl, cycloalkyl, aryl, alkaryl, aralkyl, alkoxy, -C(=O)O-Alkyl and a halogen atom;
- k" is an integer from 1 to 4.

- a hexavalent moiety according to any one of formulae (31) to (33):

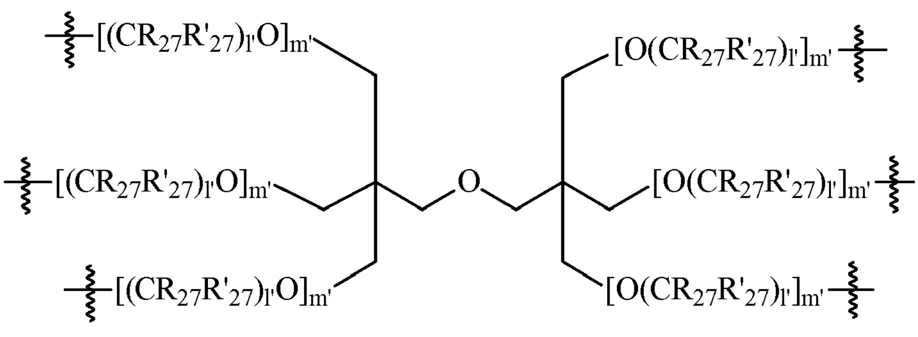

(31)

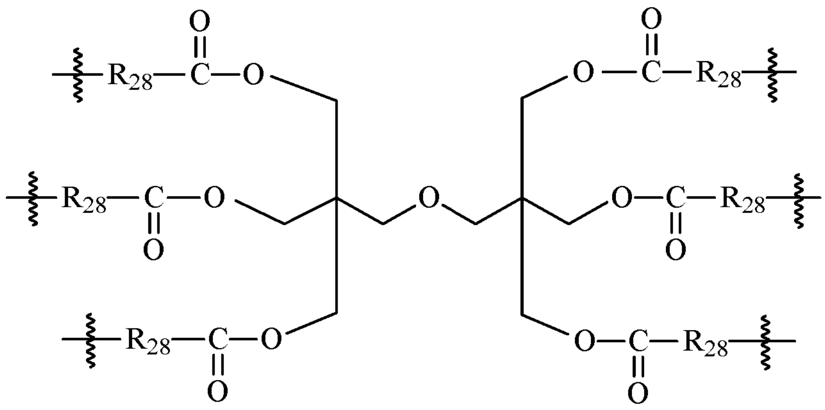

(32)

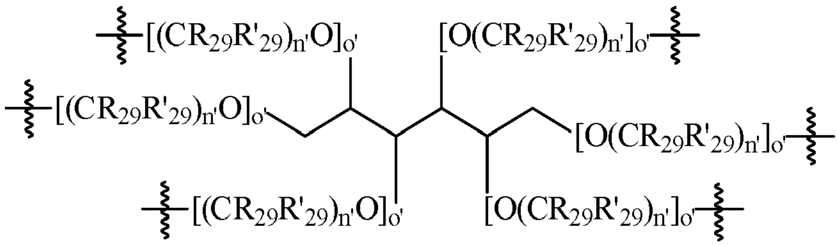

(33)

wherein

- each $R_{27}$, $R'_{27}$, $R_{29}$ and $R'_{29}$ is independently H or methyl;
- each $R_{28}$ is independently a linear or branched alkylene;
- each l' and n' is independently an integer from 2 to 4, in particular 2;
- each m' and o' is independently an integer from 0 to 10, in particular from 1 to 6.

**6.** A process for the preparation of a photoinitiator of formula (1) according to any one of claims 1 to 5, wherein the process comprises:

- reacting at least one precursor of formula (3) with at least one compound of formula (4) to provide a compound of formula (1a) and optionally reacting the compound of formula (1a) with at least one amine of formula (8); or
- reacting at least one precursor of formula (3) with at least one compound of formula (5a) to provide a compound of formula (1b); or
- reacting at least one precursor of formula (3) with at least one compound of formula (5) and at least one compound of formula (6) to provide a compound of formula (1a) and optionally reacting the compound of formula (1a) with at least one amine of formula (8); or
- reacting at least one precursor of formula (3) with at least one compound of formula (7) to provide a compound of formula (1c); or
- reacting at least one precursor of formula (3) with at least one compound of formula (7) and at least one compound of formula (6) to provide a compound of formula (1d) and optionally reacting the compound of formula (1d) with at least one amine of formula (8) to provide a compound of formula (1e);

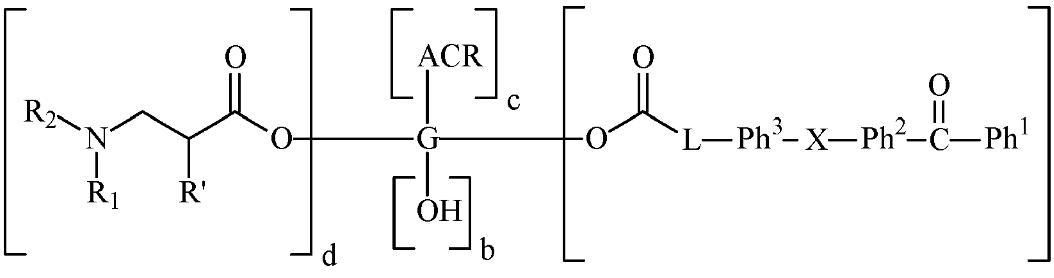

(1)

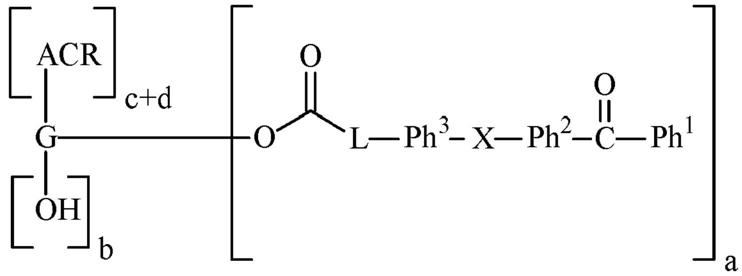

(1a)

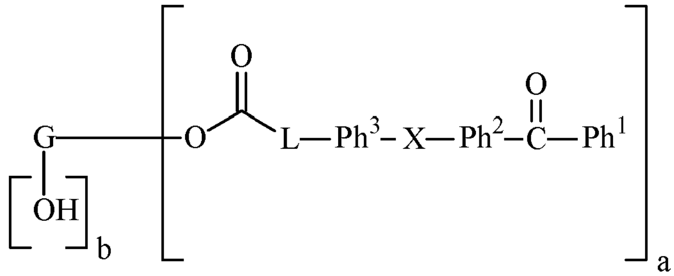

(1b)

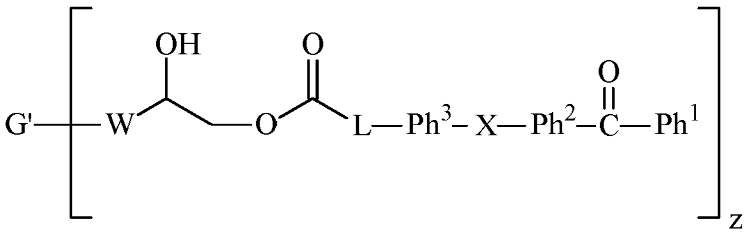

(1c)

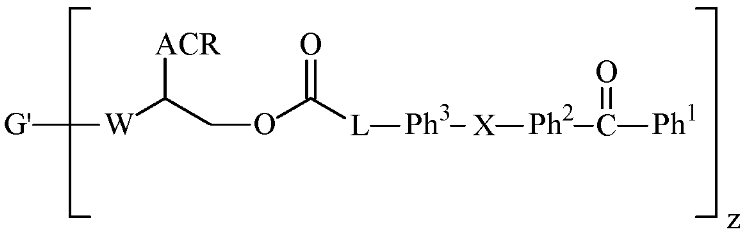

(1d)

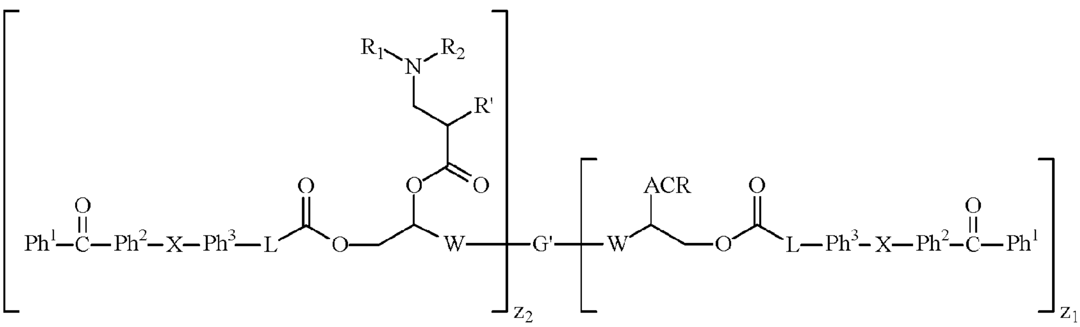

(1e)

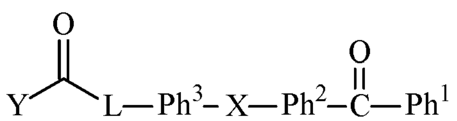

(3)

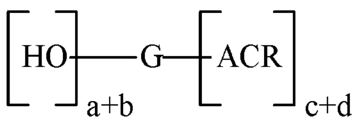

(4)

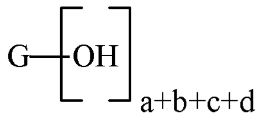 (5) 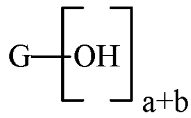 (5a) 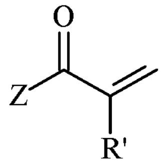 (6)

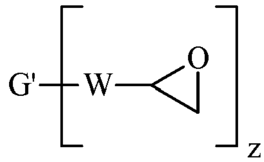 (7) 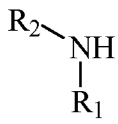 (8)

wherein:

G, G', L, ACR, $Ph^1$, $Ph^2$, $Ph^3$, R', $R_1$, $R_2$, W, X, a, b, c, d and z are as defined in any one of claims 1 to 5;
each Y and Z is independently OH, a halogen atom or -O-C(=O)-J;
J is alkyl or aryl, in particular tert-butyl
$Z_1 + Z_2 = Z$.

**7.** A precursor according to formula (3):

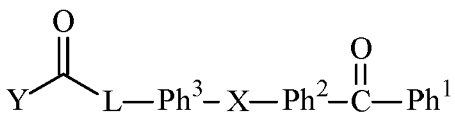 (3)

wherein:

L, $Ph^1$, $Ph^2$, $Ph^3$, X are as defined in any one of claims 1 to 5;
Y is OH, a halogen atom or -O-C(=O)-J;
J is alkyl or aryl, in particular tert-butyl.

**8.** A photoinitiator composition comprising a mixture of at least two photoinitiators of formula (1) according to any one of claims 1 to 5.

**9.** A photoinitiator composition comprising a photoinitiator of formula (1) according to any one of claims 1 to 5, and a photoinitiator other than a photoinitiator of formula (1), in particular a photoinitiator which is a thioxanthone, more particularly a thioxanthone selected from a polymeric thioxanthone, a polymerizable thioxanthone, isopropyl thioxanthone, 2,4-diethylthioxanthone, 1-chloro-4-propoxythioxanthone and mixtures thereof.

**10.** A process for photopolymerizing one or more ethylenically unsaturated compounds, wherein the process comprises contacting one or more ethylenically unsaturated compounds with a photoinitiator of formula (1) according to any one of claims 1 to 5 or a photoinitiator composition according to claim 8 or 9, and irradiating the mixture, in particular with UV, near-UV, visible, infrared and/or near-infrared radiation.

**11.** A curable composition comprising:

a) a photoinitiator of formula (1) according to any one of claims 1 to 5 or a photoinitiator composition according to claims 8 or 9; and
b) an ethylenically unsaturated component.

**12.** The curable composition of claim 11, wherein component b) comprise at least one ethylenically unsaturated compound selected from a (meth)acrylate-functionalized monomer, an amine-modified acrylate and mixtures thereof.

**13.** The curable composition of claim 11 or 12, wherein the composition further comprises an amine synergist, in particular

an amine synergist selected from aminobenzoates, polymerizable aminobenzoates, polymeric aminobenzoates and mixtures thereof.

**14.** A process for the preparation of a cured product, comprising curing the curable composition according to any one of claims 11 to 12, preferably by exposing the curable composition to radiation such as UV, near-UV, visible, infrared and/or near-infrared radiation, more preferably by exposing the polymerizable composition to a LED light source.

**15.** A use of a photoinitiator of formula (1) according to any one of claims 1 to 5 or a photoinitiator composition according to Aspect 8 or 9, to obtain a cured product having a reduced amount of extractables.

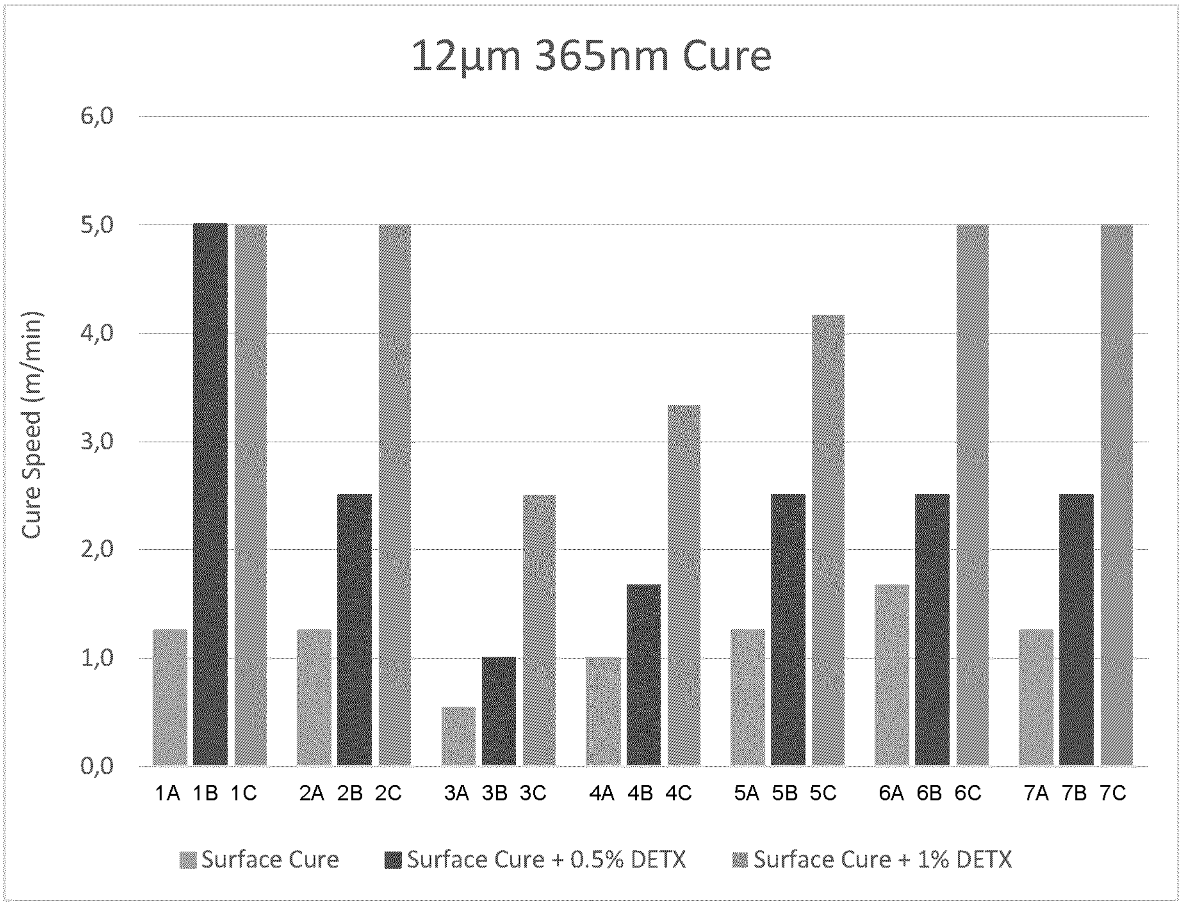
12µm 365nm Cure
Cure Speed (m/min)
6,0
5,0
4,0
3,0
2,0
1,0
0,0
1A 1B 1C
2A 2B 2C
3A 3B 3C
4A 4B 4C
5A 5B 5C
6A 6B 6C
7A 7B 7C
Surface Cure
Surface Cure + 0.5% DETX
Surface Cure + 1% DETX

**Figure 1**

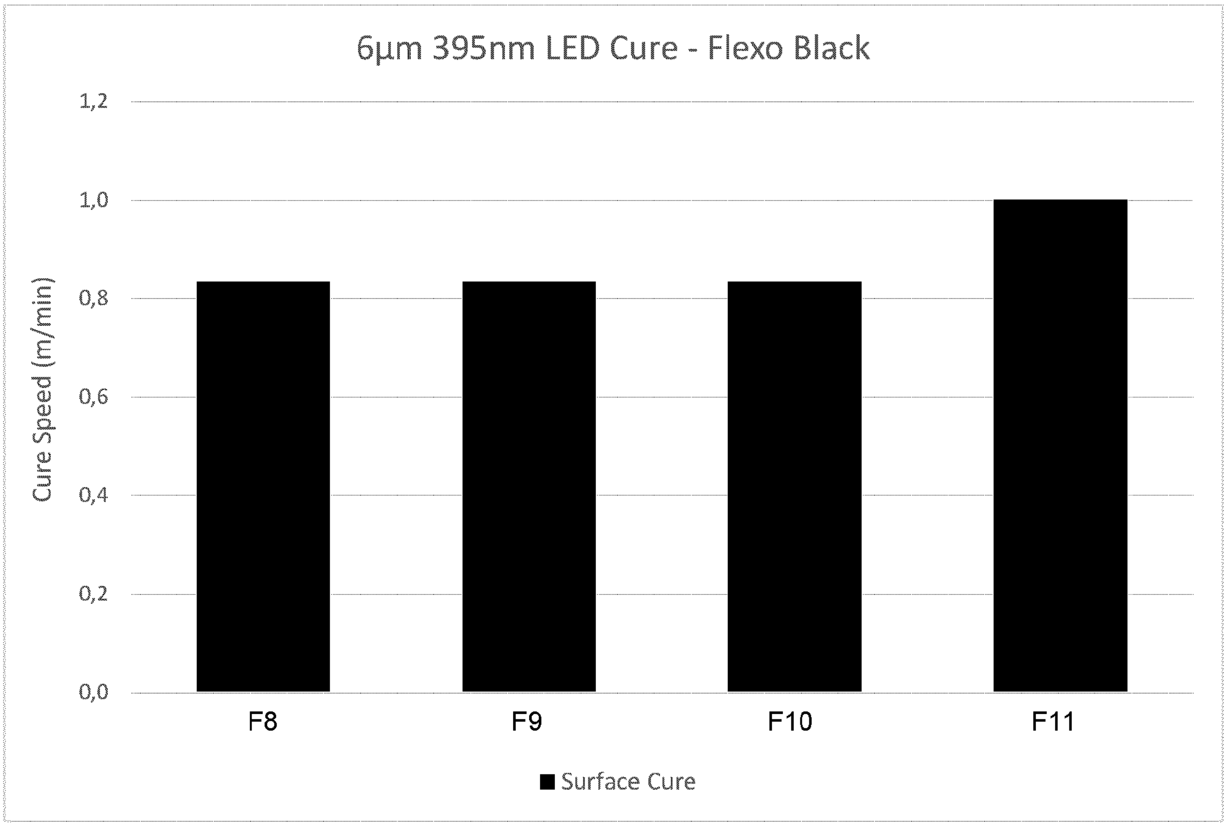
6µm 395nm LED Cure - Flexo Black
Cure Speed (m/min)
1,2
1,0
0,8
0,6
0,4
0,2
0,0
F8
F9
F10
F11
Surface Cure

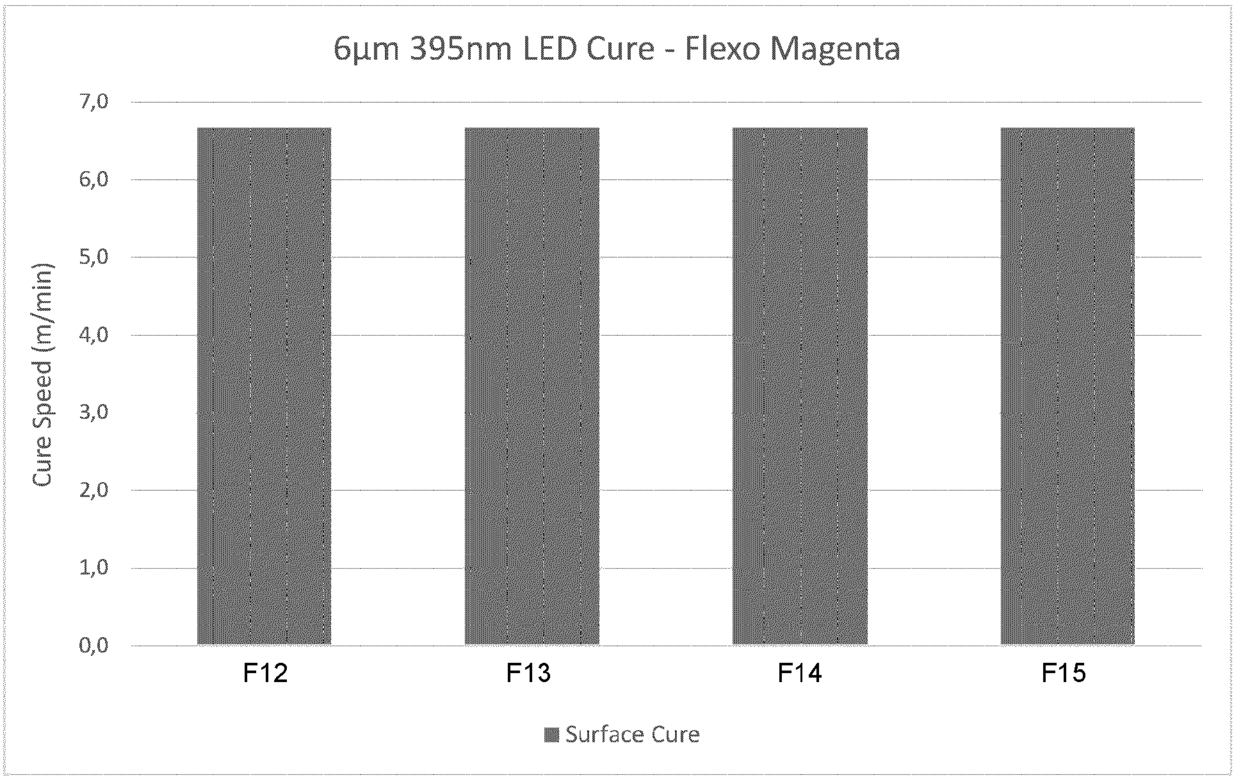
6µm 395nm LED Cure - Flexo Magenta
Cure Speed (m/min)
7,0
6,0
5,0
4,0
3,0
2,0
1,0
0,0
F12
F13
F14
F15
Surface Cure

**Figure 2**

Europäisches Patentamt
European Patent Office
Office européen des brevets

# EUROPEAN SEARCH REPORT

Application Number
EP 24 30 7277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/237837 A1 (LEE KYUNG-HOON [KR] ET AL) 2 December 2004 (2004-12-02)<br>* claim 8; compound 12 *<br>* product compound of Example 1 *<br>* example 6 *<br>* the whole document *<br>----- | 1,2,6-8 | INV.<br>C08F2/50<br>C07C323/22<br>C07C323/62<br>C08F222/10 |
| X | US 2004/237836 A1 (LEE KYUNG-HOON [KR] ET AL) 2 December 2004 (2004-12-02)<br>* claim 9; compound 11 *<br>* the whole document *<br>----- | 1,2,6-8 | |
| X | DENG LIUYANG ET AL: "Rapid construction of acridines via BF3-Et2O promoted cyclization of 2-phenylamino benzophenones",<br>TETRAHEDRON LETTERS, ELSEVIER, AMSTERDAM, NL,<br>vol. 105, 26 July 2022 (2022-07-26), XP087154704,<br>ISSN: 0040-4039, DOI: 10.1016/J.TETLET.2022.154044<br>[retrieved on 2022-07-26]<br>* Scheme 1, precursor of compound 2i *<br>* the whole document *<br>----- | 1,2,6 | TECHNICAL FIELDS SEARCHED (IPC)<br>C08F<br>C07C |
| X | JP 2009 242756 A (FUJIFILM CORP)<br>22 October 2009 (2009-10-22)<br>* compound s-24 in first row, rightmost column of paragraph [0024] *<br>* the whole document *<br>-----<br>-/-- | 7 | |

The present search report has been drawn up for all claims

3

| Place of search | Date of completion of the search | Examiner |
|---|---|---|
| The Hague | 3 June 2025 | Langer, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 30 7277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HASSAN Y ABOUL-ENEIN ET AL: "Prooxidant and antioxidant action of 4-(4-phenoxybenzoyl)benzoic acid derivatives", BIOPOLYMERS, JOHN WILEY, HOBOKEN, USA, vol. 73, no. 5, 17 February 2004 (2004-02-17), pages 631-639, XP071112540, ISSN: 0006-3525, DOI: 10.1002/BIP.10559 * figure 1; compound 2 * * the whole document * ----- | 7 | |
| X | KR 2014 0117301 A (ONO PHARMACEUTICAL CO [JP]) 7 October 2014 (2014-10-07) * example 9(19) * * the whole document * ----- | 7 | |
| X | WO 2022/207945 A2 (IGM RESINS ITALIA SRL [IT]) 6 October 2022 (2022-10-06) * claims 1-17 * * examples 1-15 * * page 18, line 2 - line 25 * * page 18, line 29 - page 19, line 29 * * page 21, line 7 - line 14 * * the whole document * ----- | 1,2,4-6, 8-15 | TECHNICAL FIELDS SEARCHED (IPC) |
| X | WO 2022/238591 A1 (IGM RESINS ITALIA SRL [IT]) 17 November 2022 (2022-11-17) * claims 1-17 * * page 16, line 5 - line 27 * * page 17, line 1 - line 31 * * page 18, line 12 - line 19 * * the whole document * ----- -/-- | 1,2,4-6, 8-15 | |

The present search report has been drawn up for all claims

3

| Place of search | Date of completion of the search | Examiner |
|---|---|---|
| The Hague | 3 June 2025 | Langer, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 30 7277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/238592 A2 (IGM RESINS ITALIA SRL [IT]) 17 November 2022 (2022-11-17)<br>* claim 1 *<br>* examples 1-4 *<br>* page 10, line 14 - line 21 *<br>* page 8, line 12 - page 9, line 3 *<br>* the whole document *<br>----- | 1,2,4,<br>9-15 | |
| X | US 3 592 841 A (BROADHEAD RONALD L ET AL) 13 July 1971 (1971-07-13)<br>* claims 1, 2, 4, 5, 12-14 *<br>* column 1 *<br>* the whole document *<br>----- | 1-4,6-15 | |
| X | YOSHIDA S ET AL: "SYNTHESIS OF ALL-AROMATIC PHTALAZIONE-CONTAINING POLYMERS BY A NOVEL N-C COUPLING REACTION",<br>MACROMOLECULES, AMERICAN CHEMICAL SOCIETY, US,<br>vol. 30, no. 8, 21 April 1997 (1997-04-21), pages 2254-2261, XP000687671,<br>ISSN: 0024-9297, DOI: 10.1021/MA961659L<br>* Compound 7 in Scheme 3 *<br>* the whole document *<br>----- | 1-4 | TECHNICAL FIELDS SEARCHED (IPC) |
| X | PUZIN YU I ET AL: "Quantitative Determination of Phthalides in Molecules of Vinyl Polymers and Their Role in Radical Polymerization",<br>RUSSIAN JOURNAL OF APPLIED CHEMISTRY,<br>vol. 74, no. 2,<br>28 February 2001 (2001-02-28), pages 292-297, XP093020422,<br>* page 293, left-hand column; compound (X) *<br>* the whole document *<br>-----<br>-/-- | 1-4 | |

The present search report has been drawn up for all claims

| Place of search | Date of completion of the search | Examiner |
|---|---|---|
| The Hague | 3 June 2025 | Langer, Oliver |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 4

Europäisches Patentamt
European Patent Office
Office européen des brevets

# EUROPEAN SEARCH REPORT

Application Number
EP 24 30 7277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GILEVA N G ET AL: "4',4''-bis(2-carboxybenzoyl)diphenyl sulfone pseudodichloride as a new monomer for polyarylenephthalides", RUSSIAN JOURNAL OF APPLIED CHEMISTRY, PLEIADES PUBLISHING, MOSCOW, vol. 88, no. 6, 30 September 2015 (2015-09-30), pages 930-934, XP035550877, ISSN: 1070-4272, DOI: 10.1134/S1070427215060063 [retrieved on 2015-09-30] * Compounds 1 and 2 in Scheme 1 * * the whole document * ----- | 1-4 | |
| X | JP H07 41510 A (NIPPON KAYAKU KK) 10 February 1995 (1995-02-10) * table 1; compound 4 * * the whole document * ----- | 1-4, 6-12,14, 15 | |
| X | CN 109 503 735 A (CHANGZHOU TRONLY ADVANCED ELECTRONIC MAT CO LTD ET AL.) 22 March 2019 (2019-03-22) * page 12, paragraph 0094 - paragraph 0095; compound 6 * * paragraph [0097] * * the whole document * ----- | 1-4, 6-12,14, 15 | TECHNICAL FIELDS SEARCHED (IPC) |
| X | US 5 171 506 A (SAKUDA MITSUHIRO [JP] ET AL) 15 December 1992 (1992-12-15) * example 1 * * the whole document * ----- | 1-3,6,7 | |
| X | US 2011/190227 A1 (HUTCHINSON JOHN HOWARD [US] ET AL) 4 August 2011 (2011-08-04) * starting material; paragraph [00652]; example 80 * * the whole document * ----- | 1-3,6 | |

The present search report has been drawn up for all claims

3

| Place of search | Date of completion of the search | Examiner |
|---|---|---|
| The Hague | 3 June 2025 | Langer, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 24 30 7277

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 24 30 7277

The Search Division considers that the present European patent application does not comply with the requirements of unity of invention and relates to several inventions or groups of inventions, namely:

1. claims: 1, 2, 4-15(all partially)

Compound ("photoinitiator") according to formula (1) wherein L is a bond; process for the preparation of said compound; precursor according to formula (3) wherein L is a bond; photoinitiator composition comprising said photoinitiator; process according to claim 10 involving said photoinitiator or photoinitiator composition; curable composition comprising said photoinitiator; process for the preparation of a cured product, comprising curing said curable composition; use of said photoinitiator of formula (1) or said photoinitiator composition as defined in claim 15.

---

2. claims: 3(completely); 1, 2, 4-15(partially)

Compound ("photoinitiator") according to formula (1) wherein L is a divalent linker; process for the preparation of said compound; precursor according to formula (3) wherein L is a divalent linker; photoinitiator composition comprising said photoinitiator; process according to claim 10 involving said photoinitiator or photoinitiator composition; curable composition comprising said photoinitiator; process for the preparation of a cured product, comprising curing said curable composition; use of said photoinitiator of formula (1) or said photoinitiator composition as defined in claim 15.

---

# ANNEX TO THE EUROPEAN SEARCH REPORT ON EUROPEAN PATENT APPLICATION NO. EP 24 30 7277

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2025

| Patent document cited in search report | Publication date | Patent family member(s) | Publication date |
|---|---|---|---|
| US 2004237837 A1 | 02-12-2004 | CN 1590357 A | 09-03-2005 |
| | | JP 2005002111 A | 06-01-2005 |
| | | KR 20040101865 A | 03-12-2004 |
| | | US 2004237837 A1 | 02-12-2004 |
| US 2004237836 A1 | 02-12-2004 | KR 20040101861 A | 03-12-2004 |
| | | US 2004237836 A1 | 02-12-2004 |
| JP 2009242756 A | 22-10-2009 | NONE | |
| KR 20140117301 A | 07-10-2014 | JP 6264134 B2 | 24-01-2018 |
| | | JP 2014208622 A | 06-11-2014 |
| | | KR 20140117301 A | 07-10-2014 |
| WO 2022207945 A2 | 06-10-2022 | CN 118201906 A | 14-06-2024 |
| | | CN 118475556 A | 09-08-2024 |
| | | CN 118696028 A | 24-09-2024 |
| | | EP 4320097 A2 | 14-02-2024 |
| | | EP 4412989 A2 | 14-08-2024 |
| | | EP 4412990 A1 | 14-08-2024 |
| | | ES 3011842 T3 | 08-04-2025 |
| | | JP 2024537185 A | 10-10-2024 |
| | | JP 2024537186 A | 10-10-2024 |
| | | JP 2024537187 A | 10-10-2024 |
| | | KR 20240089414 A | 20-06-2024 |
| | | KR 20240113759 A | 23-07-2024 |
| | | KR 20240115815 A | 26-07-2024 |
| | | US 2024400503 A1 | 05-12-2024 |
| | | US 2024400504 A1 | 05-12-2024 |
| | | US 2024409507 A1 | 12-12-2024 |
| | | WO 2022207945 A2 | 06-10-2022 |
| | | WO 2022238591 A1 | 17-11-2022 |
| | | WO 2022238592 A2 | 17-11-2022 |
| WO 2022238591 A1 | 17-11-2022 | CN 118201906 A | 14-06-2024 |
| | | CN 118475556 A | 09-08-2024 |
| | | CN 118696028 A | 24-09-2024 |
| | | EP 4320097 A2 | 14-02-2024 |
| | | EP 4412989 A2 | 14-08-2024 |
| | | EP 4412990 A1 | 14-08-2024 |
| | | ES 3011842 T3 | 08-04-2025 |
| | | JP 2024537185 A | 10-10-2024 |
| | | JP 2024537186 A | 10-10-2024 |
| | | JP 2024537187 A | 10-10-2024 |
| | | KR 20240089414 A | 20-06-2024 |
| | | KR 20240113759 A | 23-07-2024 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 24 30 7277

This annex lists the patent family members relating to the patent documents cited in the above-mentioned European search report. The members are as contained in the European Patent Office EDP file on
The European Patent Office is in no way liable for these particulars which are merely given for the purpose of information.

03-06-2025

| Patent document cited in search report | Publication date | Patent family member(s) | Publication date |
|---|---|---|---|
| | | KR 20240115815 A | 26-07-2024 |
| | | US 2024400503 A1 | 05-12-2024 |
| | | US 2024400504 A1 | 05-12-2024 |
| | | US 2024409507 A1 | 12-12-2024 |
| | | WO 2022207945 A2 | 06-10-2022 |
| | | WO 2022238591 A1 | 17-11-2022 |
| | | WO 2022238592 A2 | 17-11-2022 |
| WO 2022238592 A2 | 17-11-2022 | CN 118201906 A | 14-06-2024 |
| | | CN 118475556 A | 09-08-2024 |
| | | CN 118696028 A | 24-09-2024 |
| | | EP 4320097 A2 | 14-02-2024 |
| | | EP 4412989 A2 | 14-08-2024 |
| | | EP 4412990 A1 | 14-08-2024 |
| | | ES 3011842 T3 | 08-04-2025 |
| | | JP 2024537185 A | 10-10-2024 |
| | | JP 2024537186 A | 10-10-2024 |
| | | JP 2024537187 A | 10-10-2024 |
| | | KR 20240089414 A | 20-06-2024 |
| | | KR 20240113759 A | 23-07-2024 |
| | | KR 20240115815 A | 26-07-2024 |
| | | US 2024400503 A1 | 05-12-2024 |
| | | US 2024400504 A1 | 05-12-2024 |
| | | US 2024409507 A1 | 12-12-2024 |
| | | WO 2022207945 A2 | 06-10-2022 |
| | | WO 2022238591 A1 | 17-11-2022 |
| | | WO 2022238592 A2 | 17-11-2022 |
| US 3592841 A | 13-07-1971 | NONE | |
| JP H0741510 A | 10-02-1995 | NONE | |
| CN 109503735 A | 22-03-2019 | NONE | |
| US 5171506 A | 15-12-1992 | GB 2243154 A | 23-10-1991 |
| | | JP 3038779 B2 | 08-05-2000 |
| | | JP H042813 A | 07-01-1992 |
| | | US 5171506 A | 15-12-1992 |
| US 2011190227 A1 | 04-08-2011 | AR 074165 A1 | 29-12-2010 |
| | | AU 2009266793 A1 | 07-01-2010 |
| | | BR PI0913930 A2 | 20-10-2015 |
| | | CA 2729292 A1 | 07-01-2010 |
| | | CN 102076661 A | 25-05-2011 |
| | | EA 201100133 A1 | 30-08-2011 |
| | | EP 2307362 A2 | 13-04-2011 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 24 30 7277

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2025

| Patent document cited in search report | Publication date | Patent family member(s) | Publication date |
|---|---|---|---|
| | | GB 2461629 A | 13-01-2010 |
| | | HK 1156301 A1 | 08-06-2012 |
| | | IL 210000 A | 30-09-2014 |
| | | JP 5531370 B2 | 25-06-2014 |
| | | JP 2011526930 A | 20-10-2011 |
| | | KR 20110022688 A | 07-03-2011 |
| | | PE 20100094 A1 | 18-02-2010 |
| | | TW 201014590 A | 16-04-2010 |
| | | US 2010004331 A1 | 07-01-2010 |
| | | US 2011144160 A1 | 16-06-2011 |
| | | US 2011190227 A1 | 04-08-2011 |
| | | US 2012016029 A1 | 19-01-2012 |
| | | UY 31956 A | 29-01-2010 |
| | | WO 2010003120 A2 | 07-01-2010 |
| | | WO 2010003127 A2 | 07-01-2010 |
| | | ZA 201009090 B | 28-09-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3429852 A
- WO 2023161049 A1
- WO 2022157274 A
- WO 2021074363 A1


### Non-patent literature cited in the description


- Radiation Curing in Science and Technology. Elsevier Appl. Sci., 1993, vol. 2, 283-321
- **FOUASSIER, J. P**. Photoinitiation, Photopolymerization and Photocuring, Fundamentals and Applications. Hanser Publishers, 1995, 71-73
- **DAVID, C.** ; **DEMARTEU, W** ; **GEUSKENS, G.** *Polymer*, 1969, vol. 10, 21-27
- **CARLINI, C.** ; **CIARDELLI, F** ; **DONATI, D.** ; **GURZONI, F.** *Polymer*, 1983, vol. 24, 599-606
- **WILLY et al.** Industrial Organic Pigments, Production, Properties, Applications. Wiley - VCH, 2004